# EUROPEAN PATENT APPLICATION

(11) **EP 0 623 596 A1**
(43) Date of publication of application: **09.11.1994**
(21) Application number: 94106853.8
(22) Date of filing: 02.05.1994
(51) Int. Cl.: C07D 207/09, C07D 211/26, C07D 211/60, C07D 401/12, A61K 31/40, A61K 31/445

(54) **N-acylpyrrolidines or N-acylpiperidines having a guanidinyl- or amidinyl-substituted side chain as thrombin inhibitors**

(30) Priority: 03.05.1993 US 56017
(71) Applicant: BRISTOL-MYERS SQUIBB COMPANY, Princeton, NJ 08543-4000 (US)
(72) Inventor: Kimball, Spencer D., East Windsor, NJ (US); Das, Jagabandhu, Mercerville, NJ (US); Lau, Wan Fang, Lawrenceville, NJ (US)
(74) Representative: VOSSIUS & PARTNER

(57) **Abstract**

Guanidinyl- or amidinyl-substituted heterocyclic thrombin inhibitors are provided which have the structure 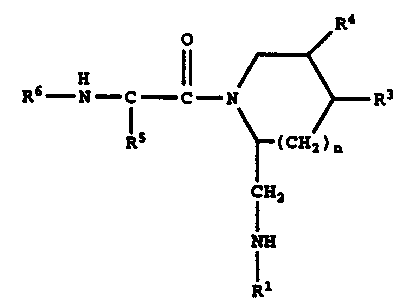
wherein n is 0, 1 or 2;
R¹ is -A-R², -CO-A-R² or -SO₂-A-R²;
wherein R² is guanidine, amidine or amino, and A is an alkyl, alkenyl or alkynyl chain of 2 to 6 carbons; or
R¹ is -(CH₂)ₚ-A'-R^{2'} or -(CH₂)ₚ-CO-A'R^{2'} where p is 0, 1 or 2, R^{2'} is amidine and A' is an azacycloalkyl or azaheteroalkyl ring of 4 to 8 atoms, optionally substituted by alkyl, CO or halo;
R¹ is -(CH₂)ₚ-A''-R^{2''}, -(CH₂)ₚ-CO-A''-R^{2''}, or -(CH₂)ₚ-SO₂-A''-R^{2''},
wherein R^{2''}is guanidine, amidine or aminomethyl, and A'' is aryl or cycloalkyl;
and R³, R⁴, R⁵ and R⁶ are as defined herein.

## Description

The present invention relates to guanidinylor amidinyl-substituted methylamino heterocyclic compounds, which are thrombin inhibitors and thus useful in inhibiting formation of thrombi.

The guanidinyl- or amidinyl-substituted methylamino heterocyclic thrombin inhibitors of the invention have the structure I 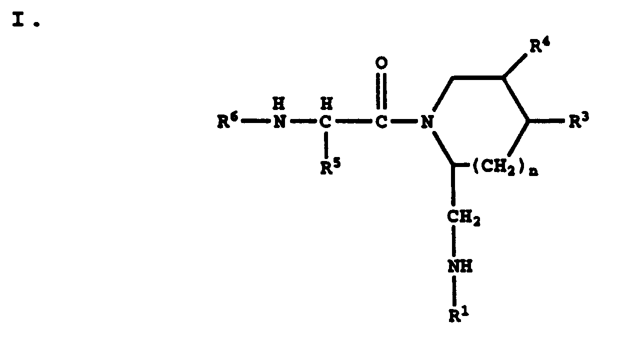
including all stereoisomers thereof
wherein n is 0, 1 or 2;
R¹ is -A-R², -CO-A-R² or -SO²-A-R²; wherein R² is guanidine, amidine or amino, and A is an alkyl, alkenyl or alkynyl chain of 2 to 6 carbons; or
R¹ is -(CH₂)ₚ-A'-R^{2'} or -(CH₂)ₚ-CO-A'R^{2'} where p is 0, 1 or 2, R^{2'} is amidine and A' is an azacycloalkyl, azaheteroalkyl or azaheteroalkenyl ring of 4 to 8 atoms, optionally substituted by alkyl, CO or halo as given by the structure: 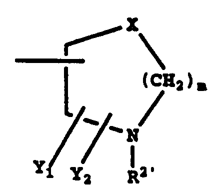
where X is CH₂, O, S, NH or CH=CH;
m = 0, 1, 2, 3 or 4 if X = CH₂ or CH=CH;
m = 2, 3 or 4 if X = O, S, NH; and
Y₁, Y₂ are independently H, alkyl, halo or keto; or
R¹ is -(CH₂)ₚ-A''-R^{2''}, -(CH₂)ₚ-CO-A''-R^{2''}, or -(CH₂)ₚ-SO₂-A''-R^{2''},
wherein R^{2''} is guanidine, amidine or aminomethyl, A'' is aryl or cycloalkyl, and p is as defined above;
R³ and R⁴ are independently hydrogen, lower alkyl, cycloalkyl, aryl, hydroxy, alkoxy, keto, thioketal, thioalkyl, thioaryl, amino or alkylamino; or R³ and R⁴ together with the carbons to which they are attached form a cycloalkyl, aryl, or heteroaryl ring;
R⁵ is hydrogen, hydroxyalkyl, aminoalkyl, amidoalkyl, alkyl, cycloalkyl, aryl, arylalkyl, alkenyl, alkynyl, arylalkoxyalkyl, or an amino acid side chain, either protected or unprotected; and
R⁶ is hydrogen, 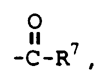
-SO₂R⁷ or -CO₂R⁷ (wherein R⁷ is lower alkyl, aryl or cycloheteroalkyl);
including pharmaceutically acceptable salts thereof.

The term "lower alkyl" or "alkyl" as employed herein by itself or as part of another group includes both straight and branched chain radicals of up to 18 carbons, preferably 1 to 8 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as well as such groups including 1, 2 or 3 halo substituents, an aryl substituent, an alkylaryl substituent, a haloaryl substituent, a cycloalkyl substituent, an alkylcycloalkyl substituent, an alkenyl substituent, an alkynyl substituent, hydroxy or a carbon substituent.

The term "cycloalkyl" by itself or as part of another group includes saturated cyclic hydrocarbon groups containing 3 to 12 carbons, preferably 3 to 8 carbons, which include cyclopropyl, cyclobutyl, cyclopentyl, cyclohexyl, cycloheptyl, cyclooctyl, cyclodecyl and cyclododecyl, any of which groups may be substituted with substituents such as halogen, lower alkyl, alkoxy and/or hydroxy groups.

The term "aryl" or "Ar" as employed herein by itself or as part of another group refers to monocyclic or bicyclic aromatic groups containing from 6 to 10 carbons in the ring portion, such as phenyl, or naphthyl. Aryl (or Ar), phenyl or naphthyl may include substituted aryl, substituted phenyl or substituted naphthyl, which may include 1 or 2 substituents on either the Ar, phenyl or naphthyl such as lower alkyl, cyano, amino, alkylamino, dialkylamino, nitro, carboxy, carboalkoxy, trifluoromethyl, halogen (Cl, Br, I or F), lower alkoxy, arylalkoxy, hydroxy, alkylthio, alkylsulfinyl, alkylsulfonyl, arylthio, arylsulfinyl and/or arylsulfonyl.

The term "aralkyl", "aryl-alkyl" or "aryl-lower alkyl" as used herein by itself or as part of another group refers to lower alkyl groups as discussed above having an aryl substituent, such as benzyl.

The term "lower alkoxy", "alkoxy" or aralkoxy" includes any of the above lower alkyl, alkyl or aralkyl groups linked to an oxygen atom.

The term "halogen" or "halo" as used herein by itself or as part of another group refers to chlorine, bromine, fluorine or iodine with chlorine being preferred.

The term "lower alkenyl" or "alkenyl" as employed herein by itself or as part of another group includes a carbon chain of up to 16 carbons, preferably 3 to 10 carbons, containing one double bond which will be separated from "N" by at least one saturated carbon moiety such as -(CH₂)_{q}- where q can be 1 to 14, such as 2-propenyl, 2-butenyl, 3-butenyl, 2-pentenyl, 4-pentenyl and the like, and may include a halogen substituent such as I, Cl, or F.

The term "lower alkynyl" or "alkynyl" as employed herein by itself or as part of another group includes a carbon chain of up to 16 carbons, preferably 3 to 10 carbons, containing one triple bond which will be separated from "N" by at least one saturated carbon moiety such as -(CH2)_{q'}- where q' can be 1 to 14, such as 2-propynyl, 2-butynyl, 3-butynyl and the like.

The term "heteroaryl" or heteroaromatic by itself or as part of another group refers to a 5- or 6-membered aromatic ring which includes 1 or 2 hetero atoms such as nitrogen, oxygen or sulfur, such as 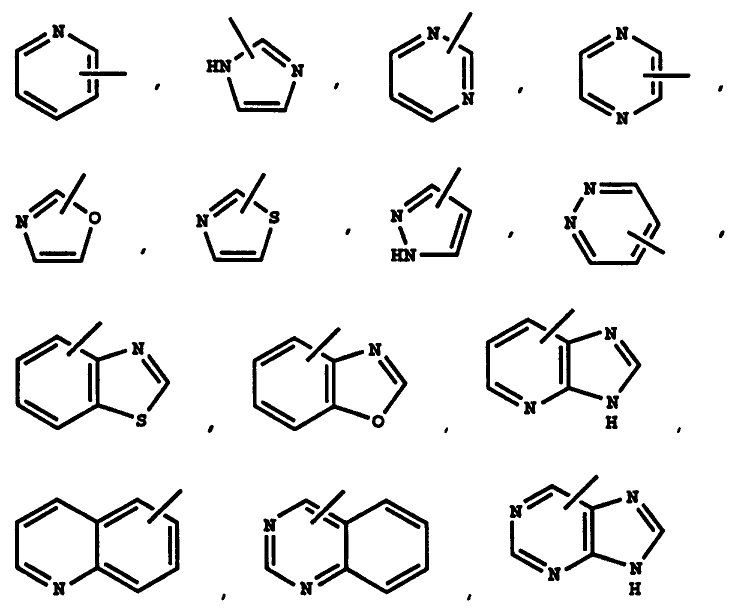
and the like. The heteroaryl rings may optionally be fused to aryl rings defined previously. The hetero-aryl rings may optionally include 1 or 2 substituents such as halogen (Cl, Br, F or CF₃), lower alkyl, lower alkoxy, carboxy, amino, lower alkylamino and/or dilower alkylamino.

The term "cycloheteroalkyl" as used herein refers to a 5-, 6- or 7-membered saturated ring which includes 1 or 2 hetero atoms such as nitrogen, oxygen and/or sulfur, such as 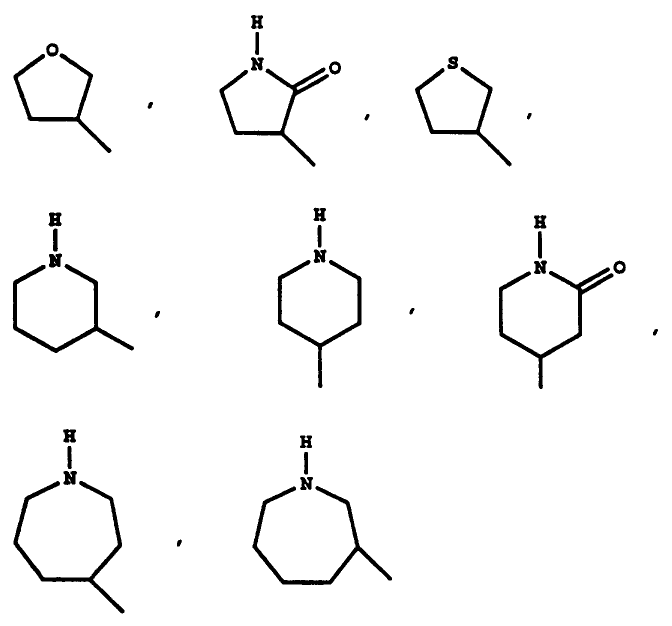
and the like.

The term "azaheteroalkenyl" as used herein refers to a 4- to 8-membered ring which includes 1 or 2 hetero atoms such as nitrogen, oxygen and/or sulfur, such as 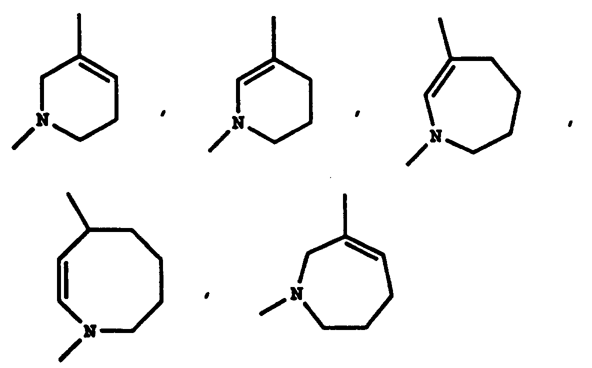
The term "amino acid side chain" refers to any of the known alpha-amino acids such as arginine, histidine, alanine, glycine, lysine, glutamine, leucine, valine, serine, homoserine, allothreonine, naphthylalanine, isoleucine, phenylalanine and the like.

Preferred are compounds of formula I wherein n is 0, R¹ is 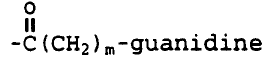
or -CH₂(CH₂)ₘ-guanidine, and m is 3, 4 or 5; and compounds of formula I wherein R¹ is A''-R^{2''}, A'' is phenyl, and R^{2''} is amidine and compounds of formula I wherein R¹ is -(CH₂)ₚ-A'-R^{2'}, or 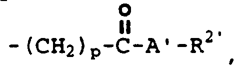
p is 0 or 1, A' is azacylcloalkyl or azacycloalkenyl, and R^{2'} is amidine;
R³ and R⁴ are each H, R⁵ is hydroxymethyl, -CH₂COOalkyl, or benzyl and R⁶ is 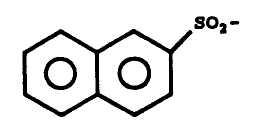
H, BOC or CBZ.

Most preferred are compounds of formula I wherein n is 0, R¹ is -(CH₂)ₚ-A'-R^{2'} or 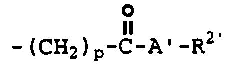
wherein p is 0 or 1, A' is 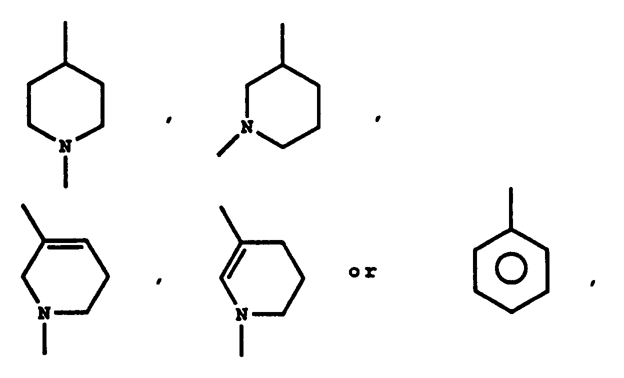
R^{2'} is amidine, R³ and R⁴ are each H, R⁵ is hydroxymethyl, -CH₂COOCH₃, or benzyl, and R⁶ is 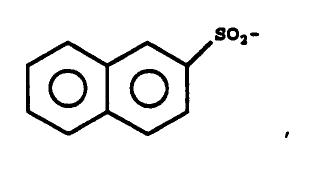
H, BOC or CBZ.

The compounds of formula I of the invention may be prepared according to the following reaction sequences. 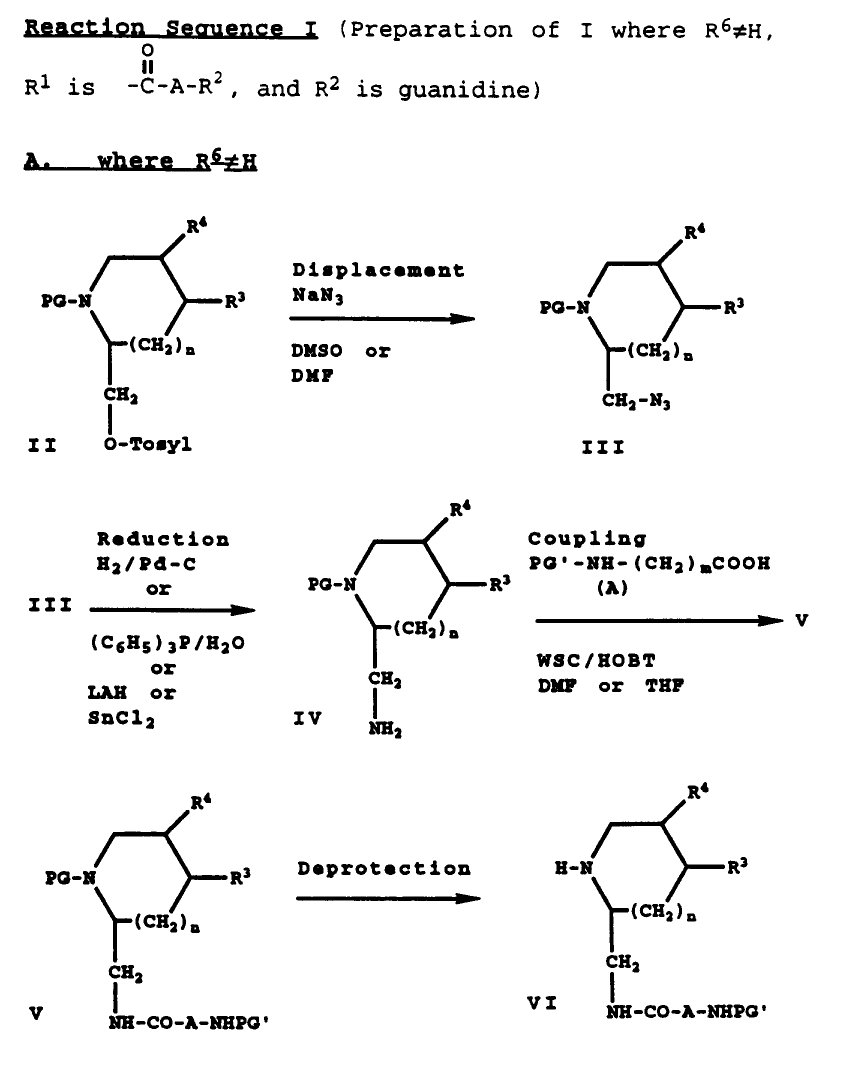 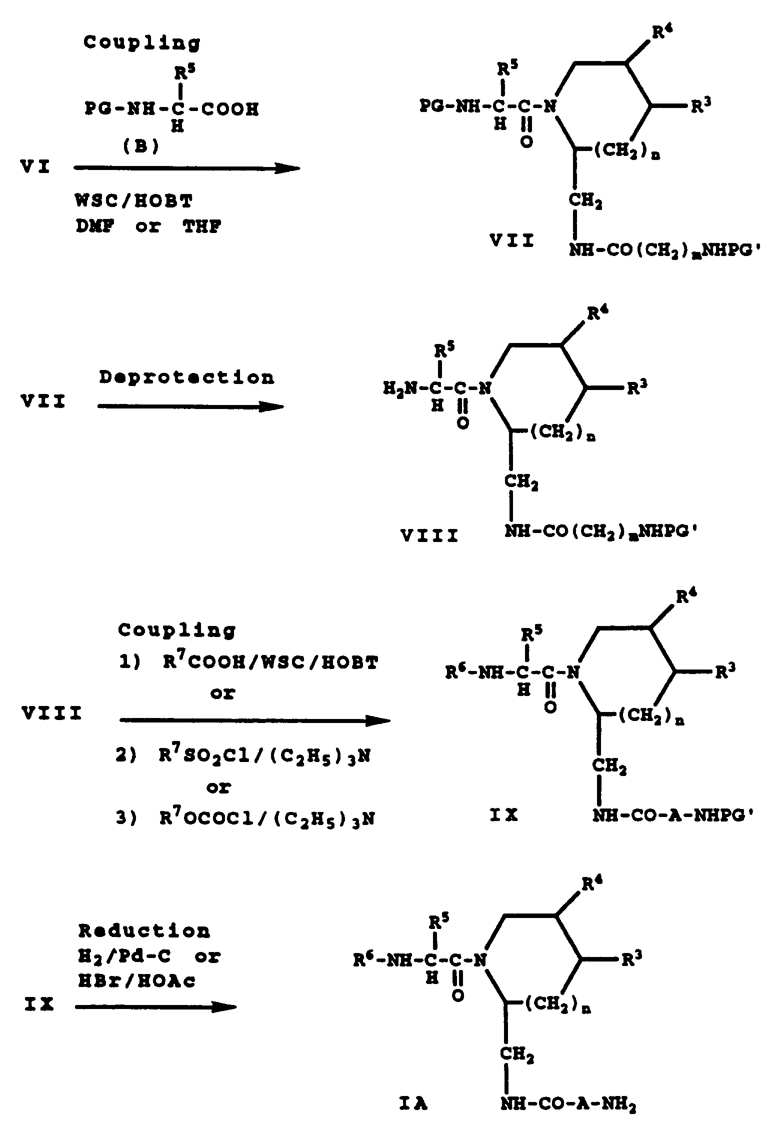 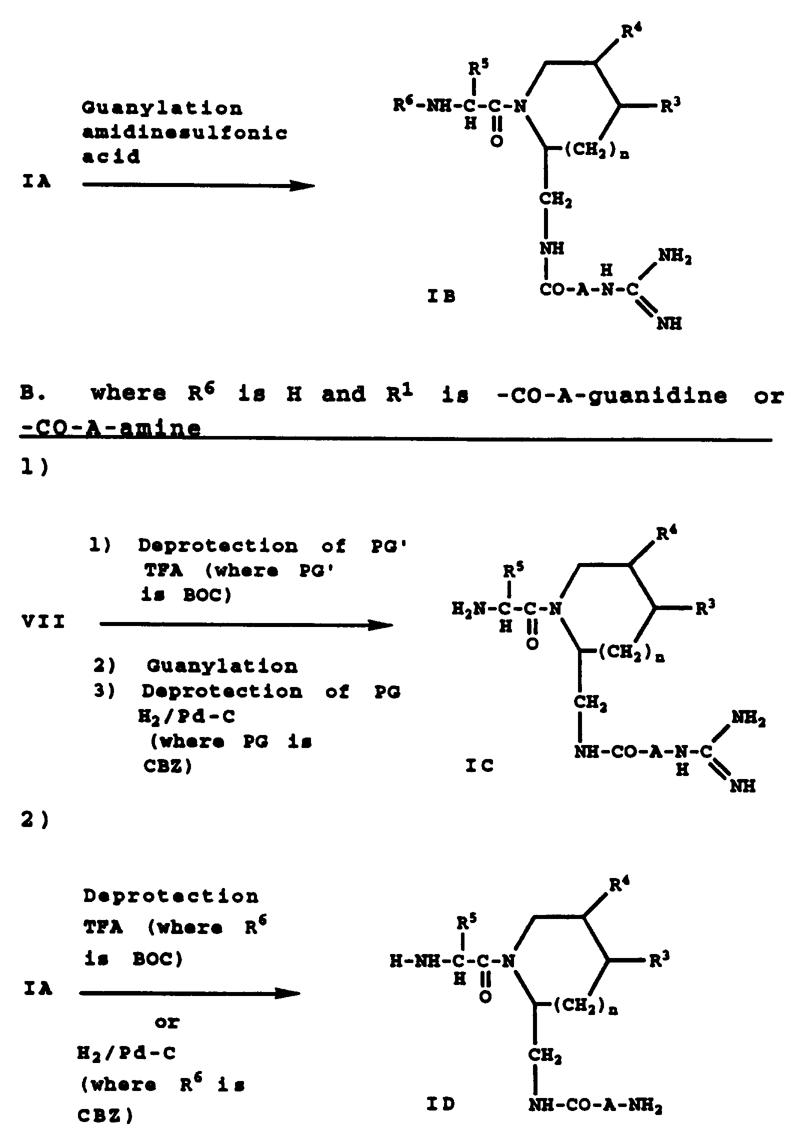 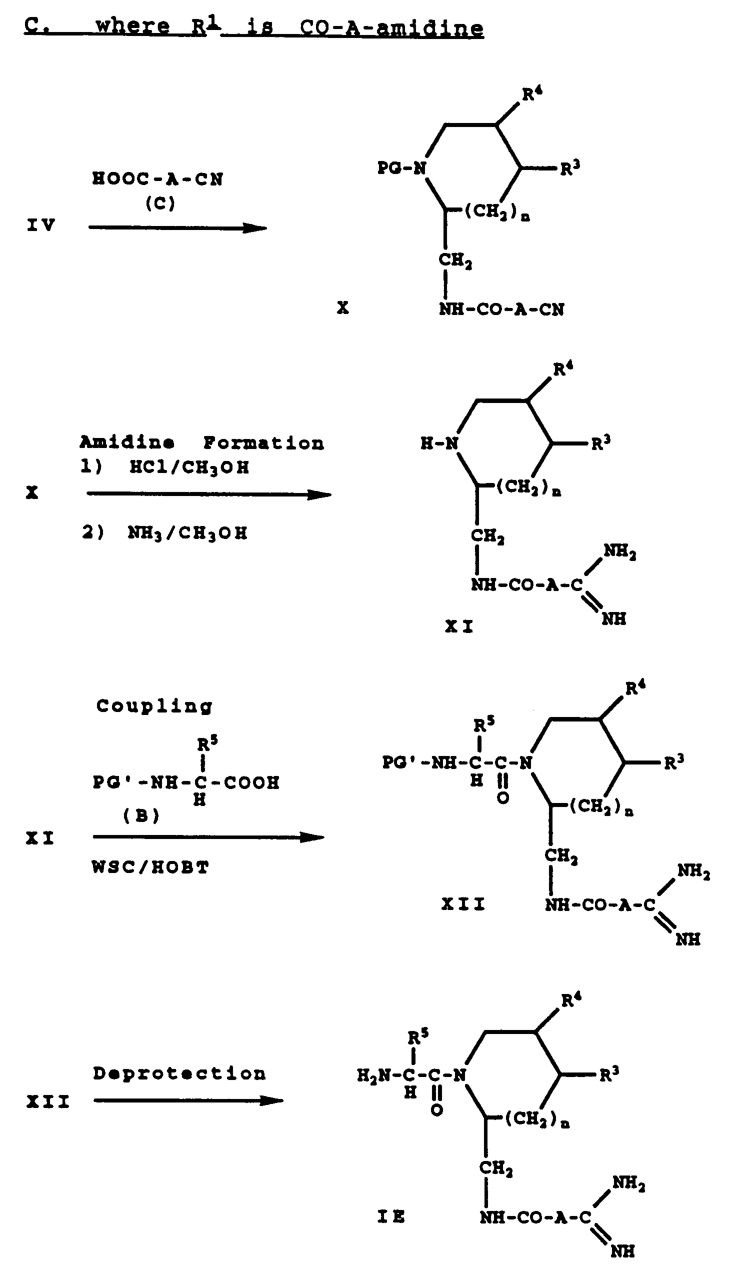 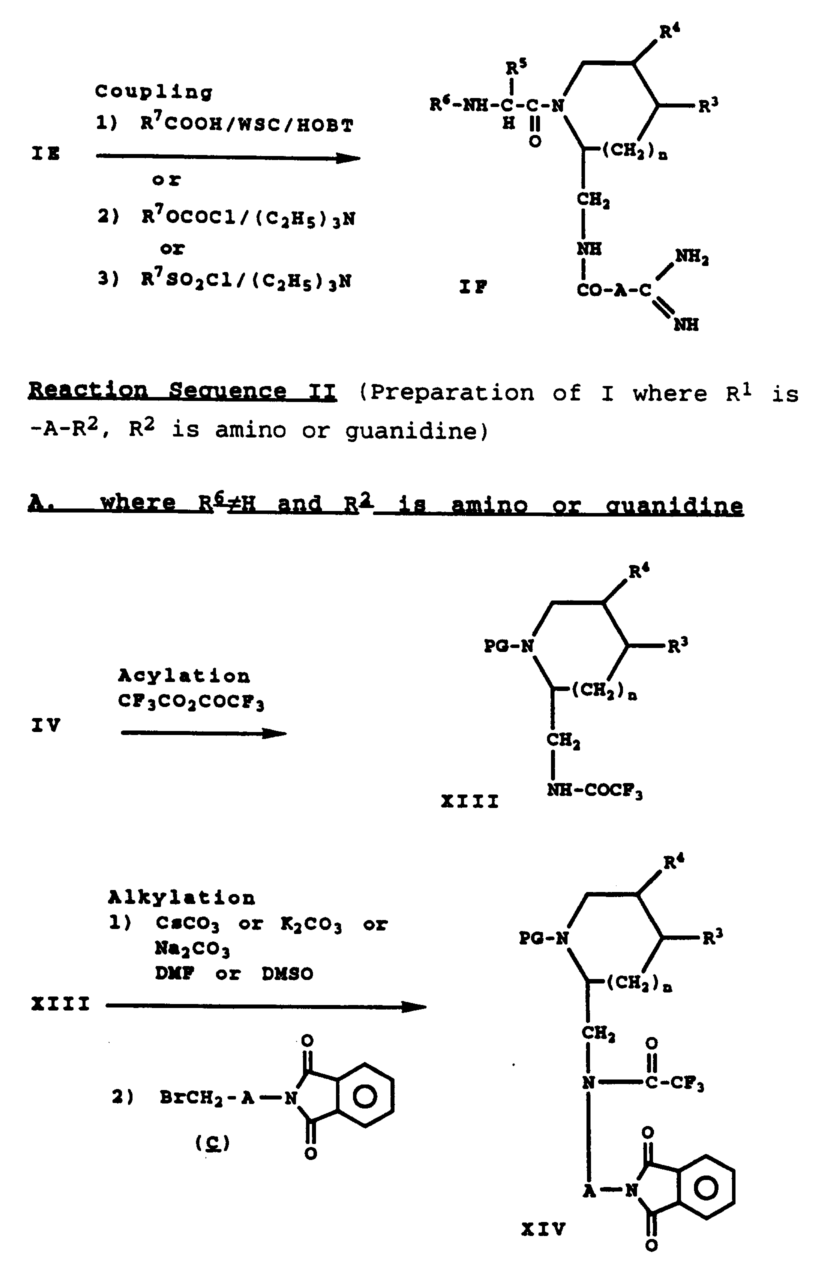 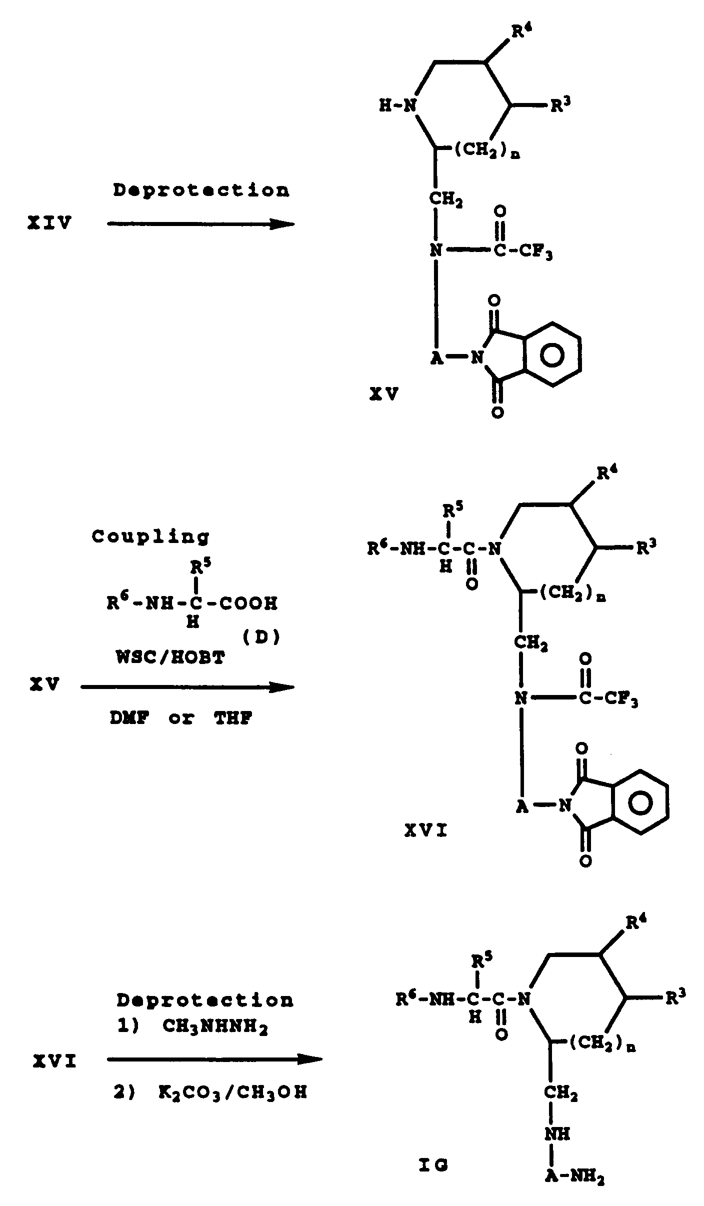 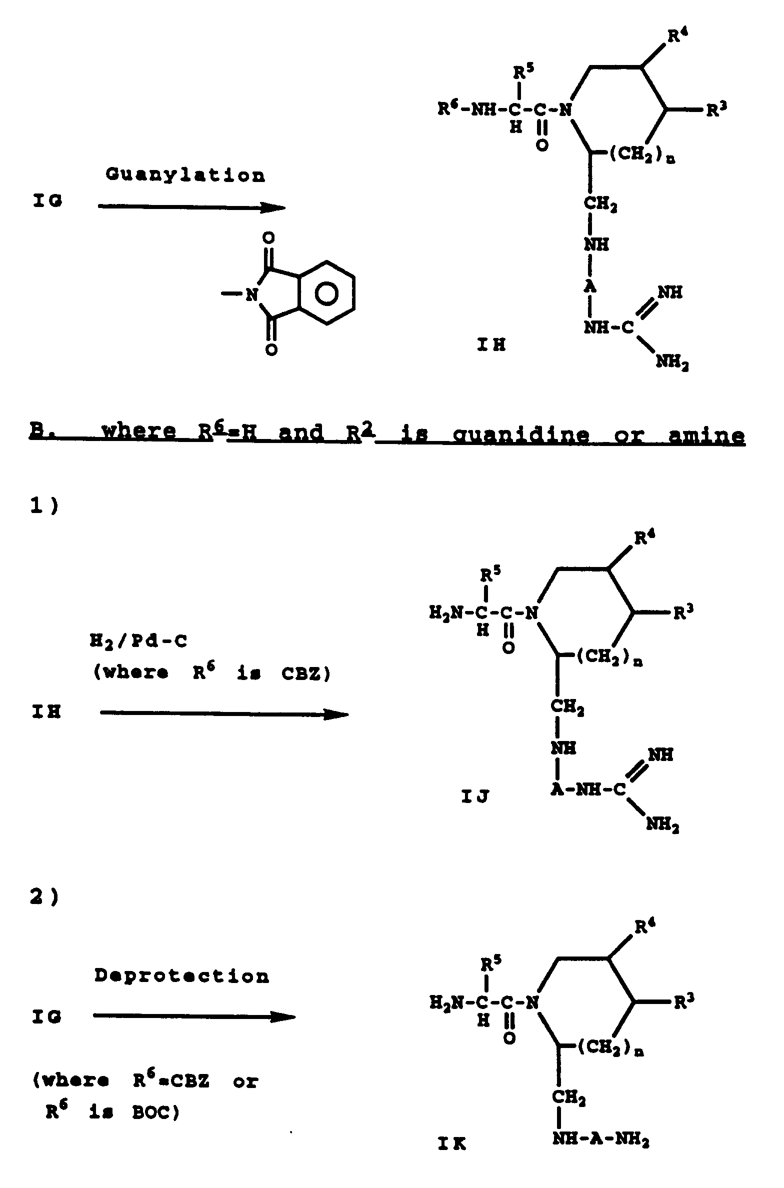 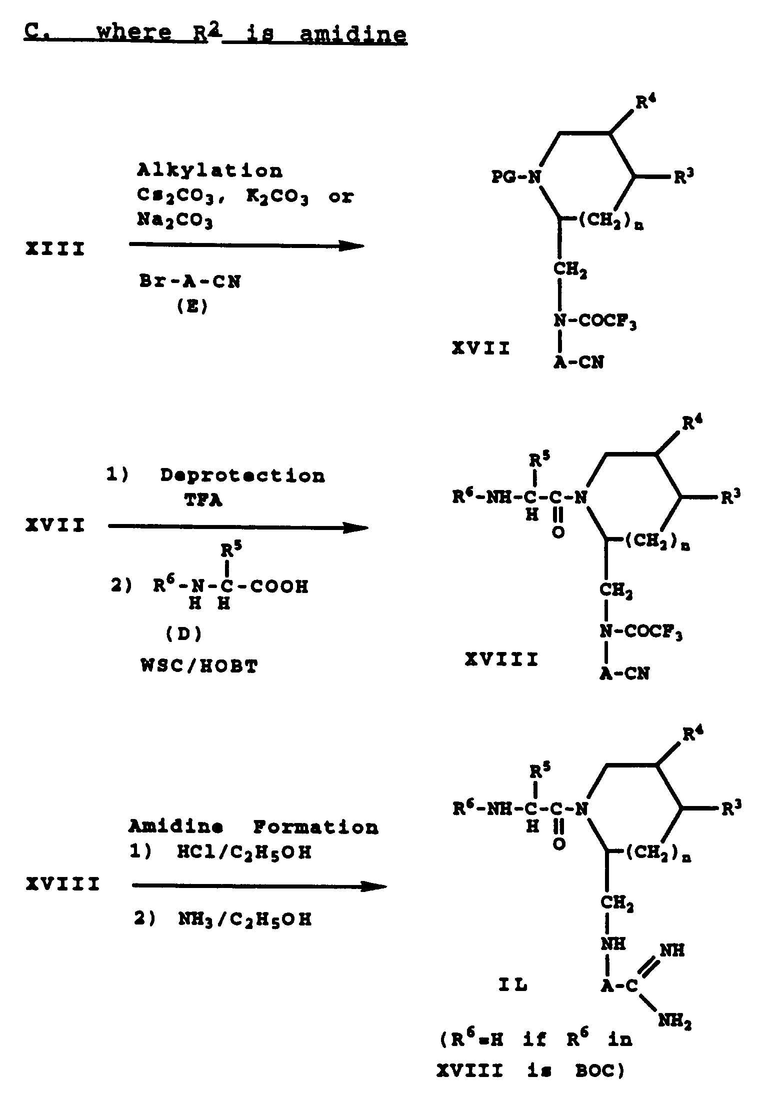 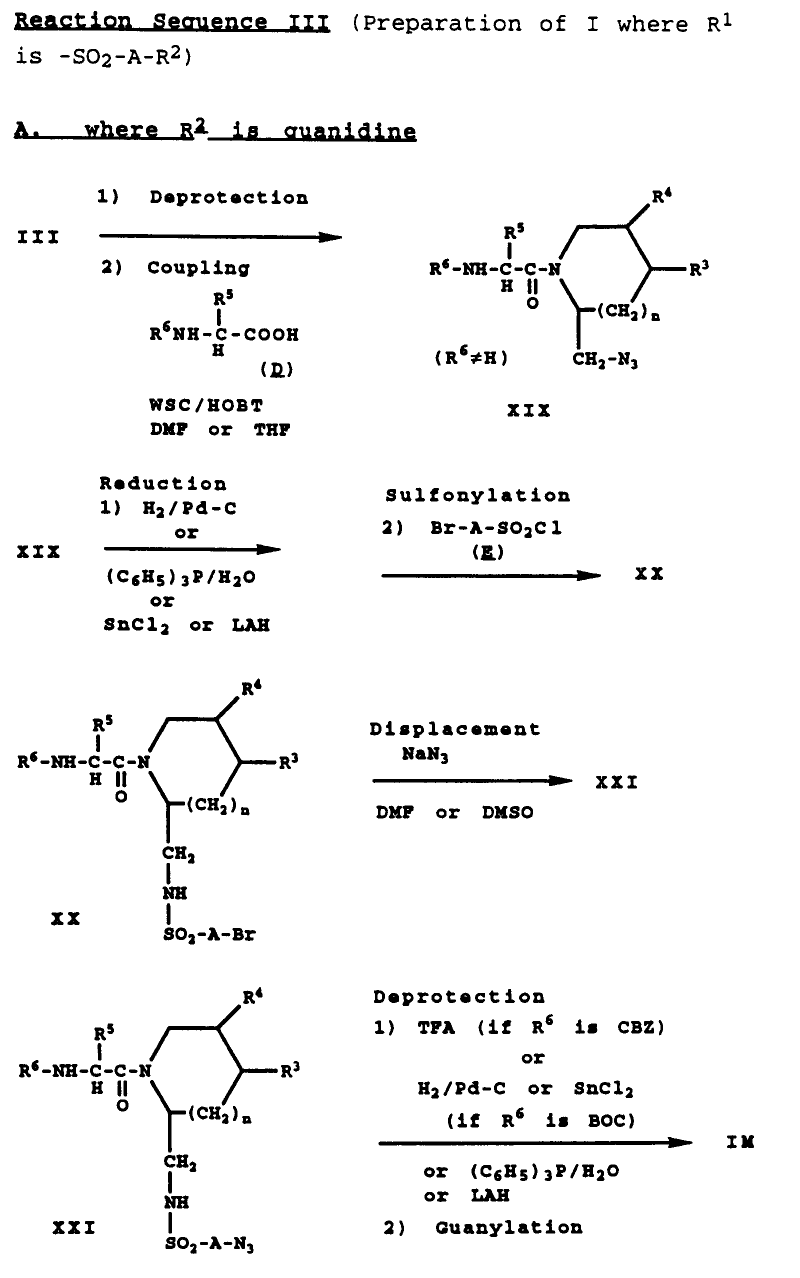 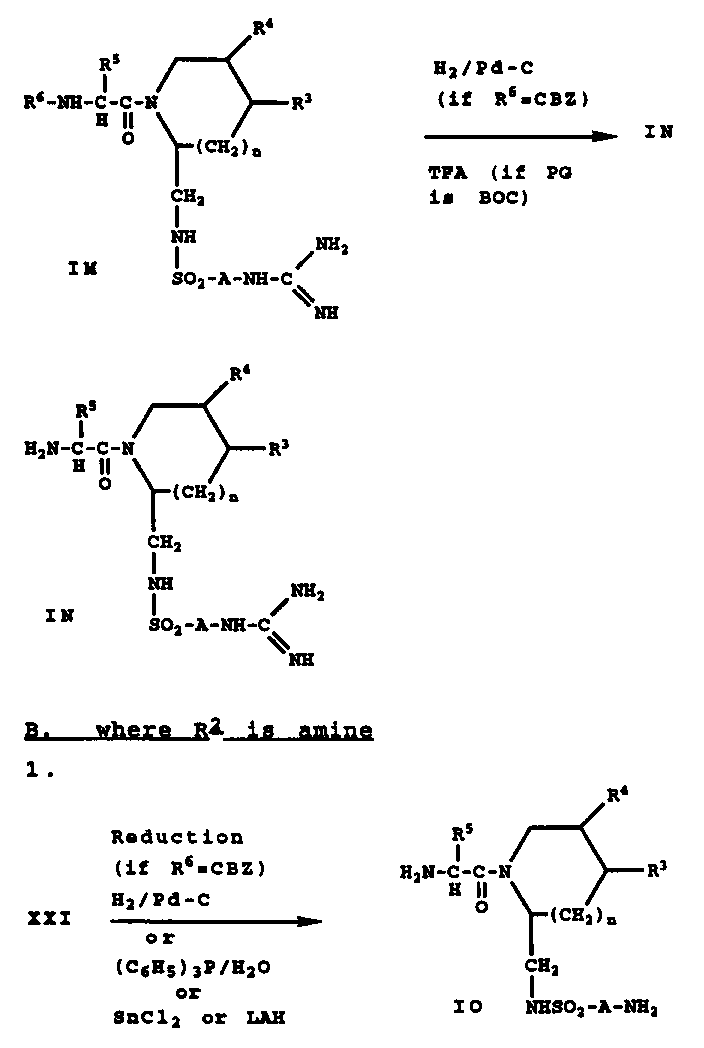 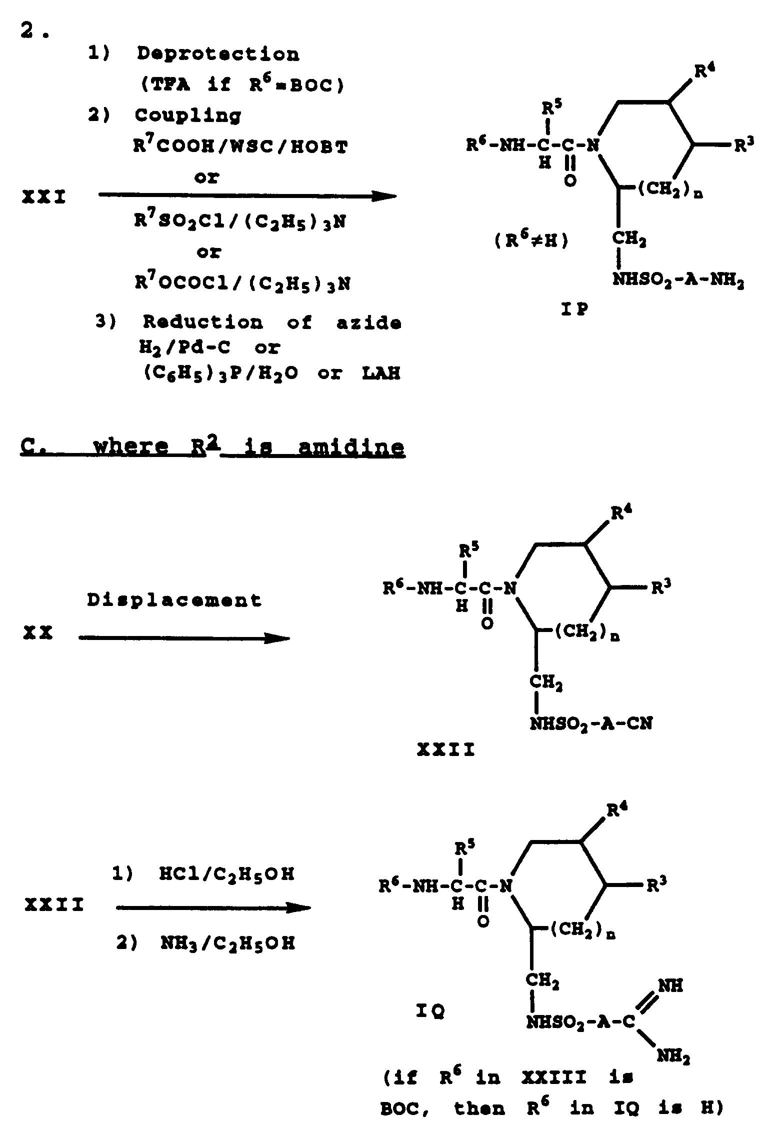 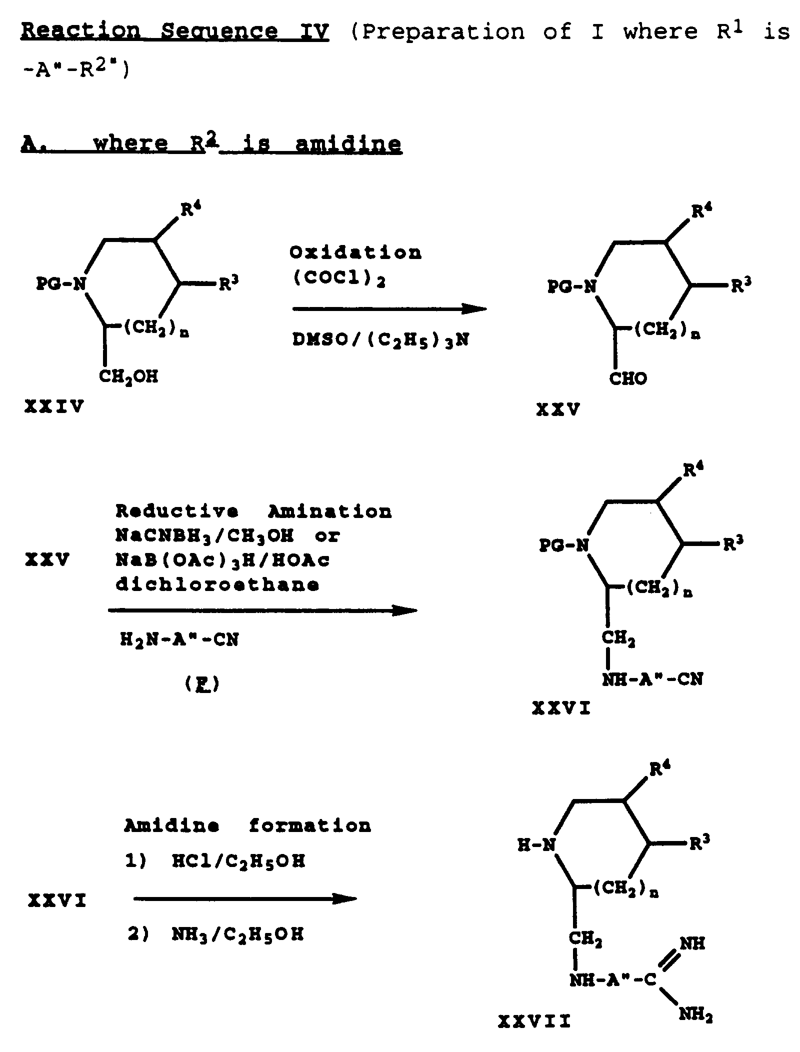 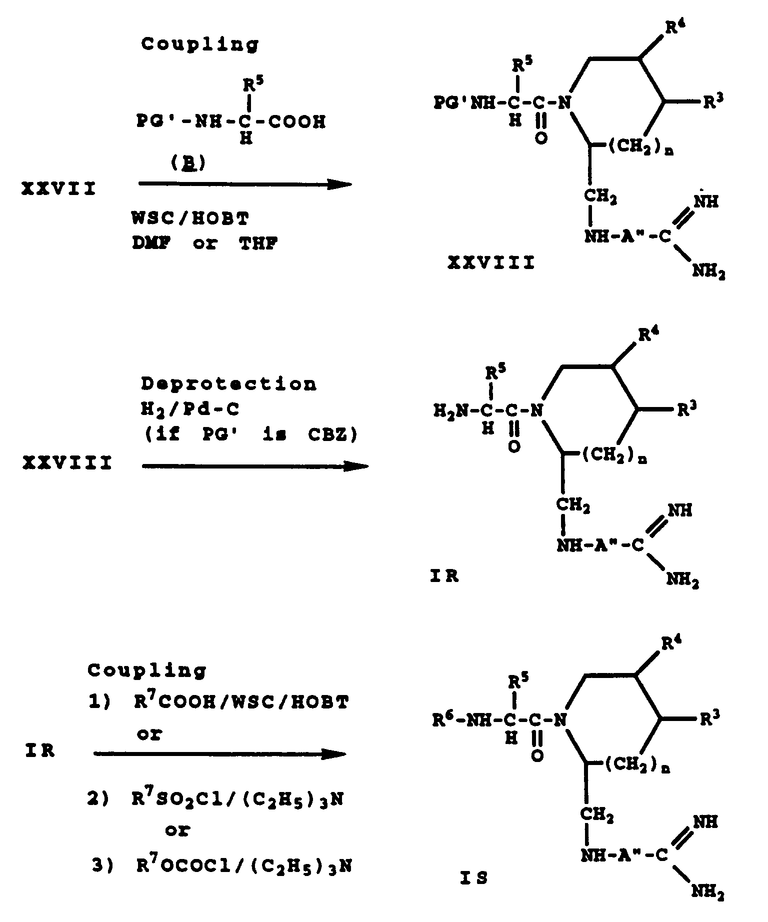 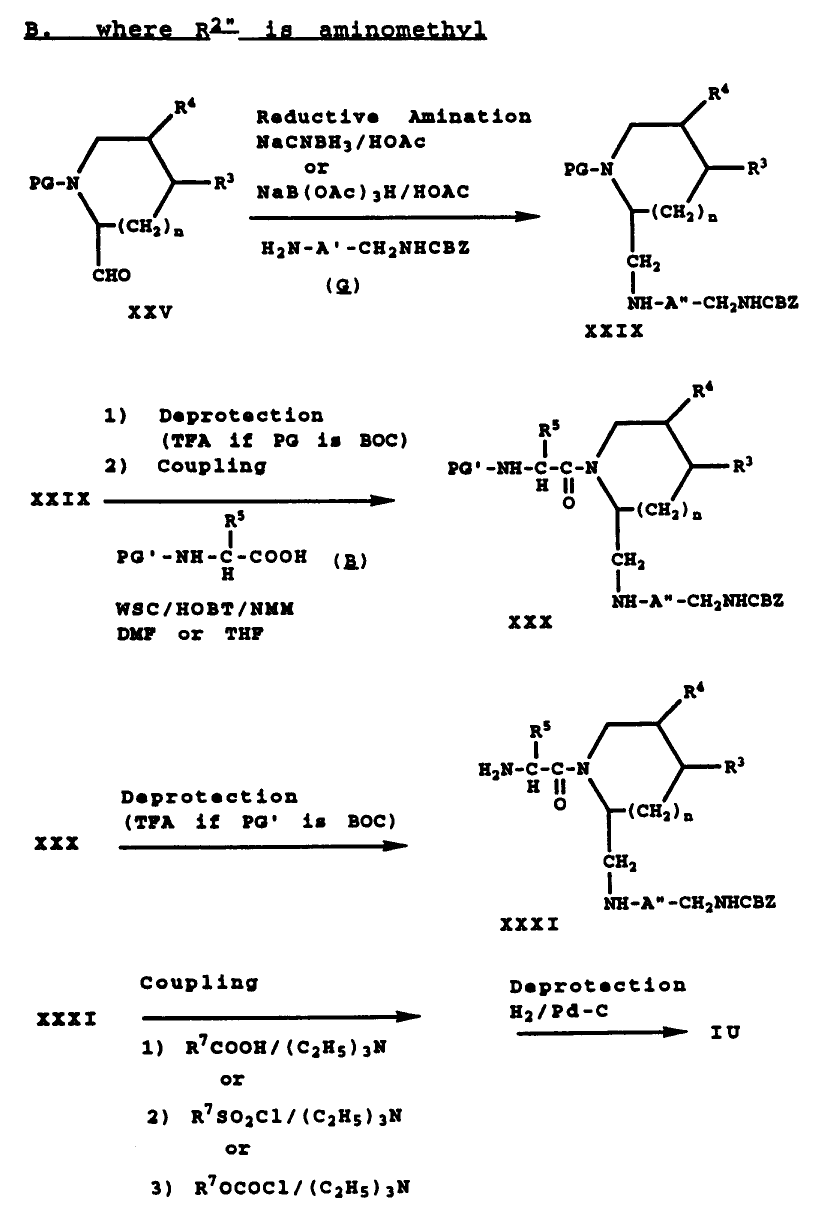 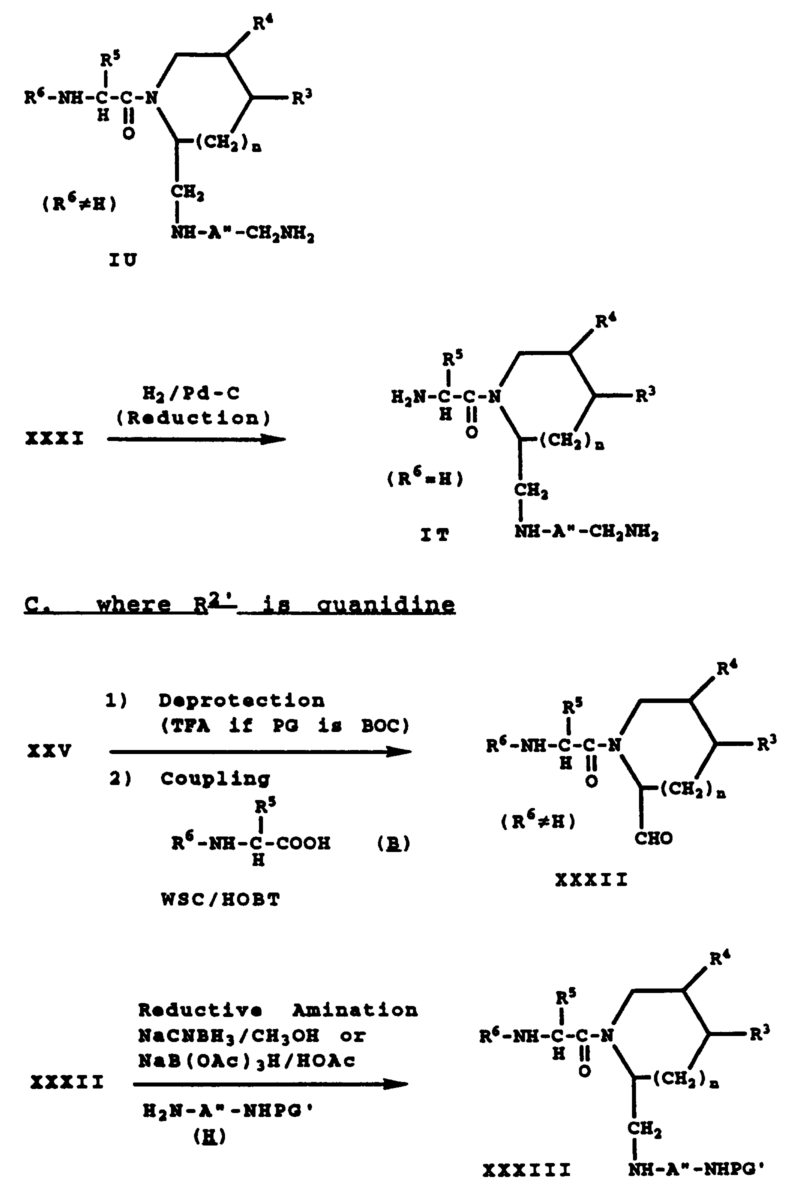 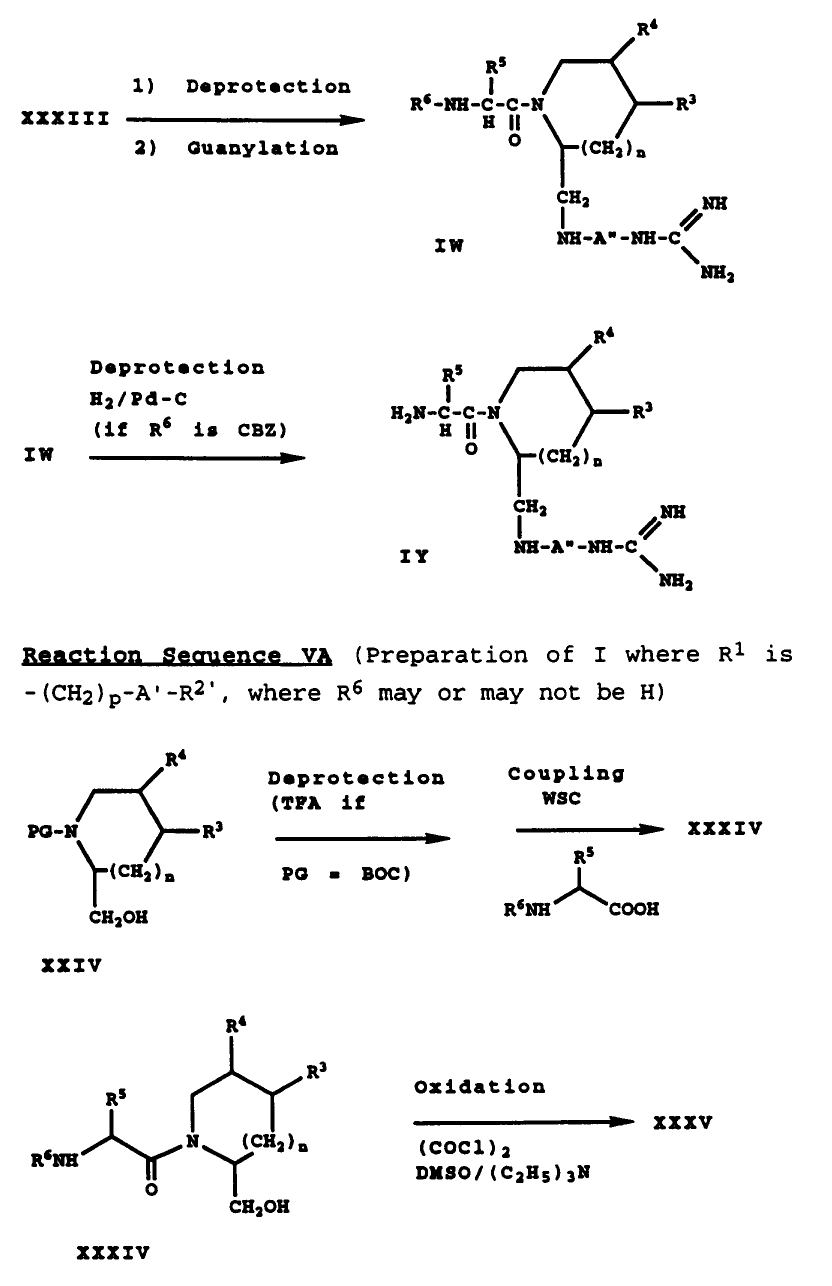 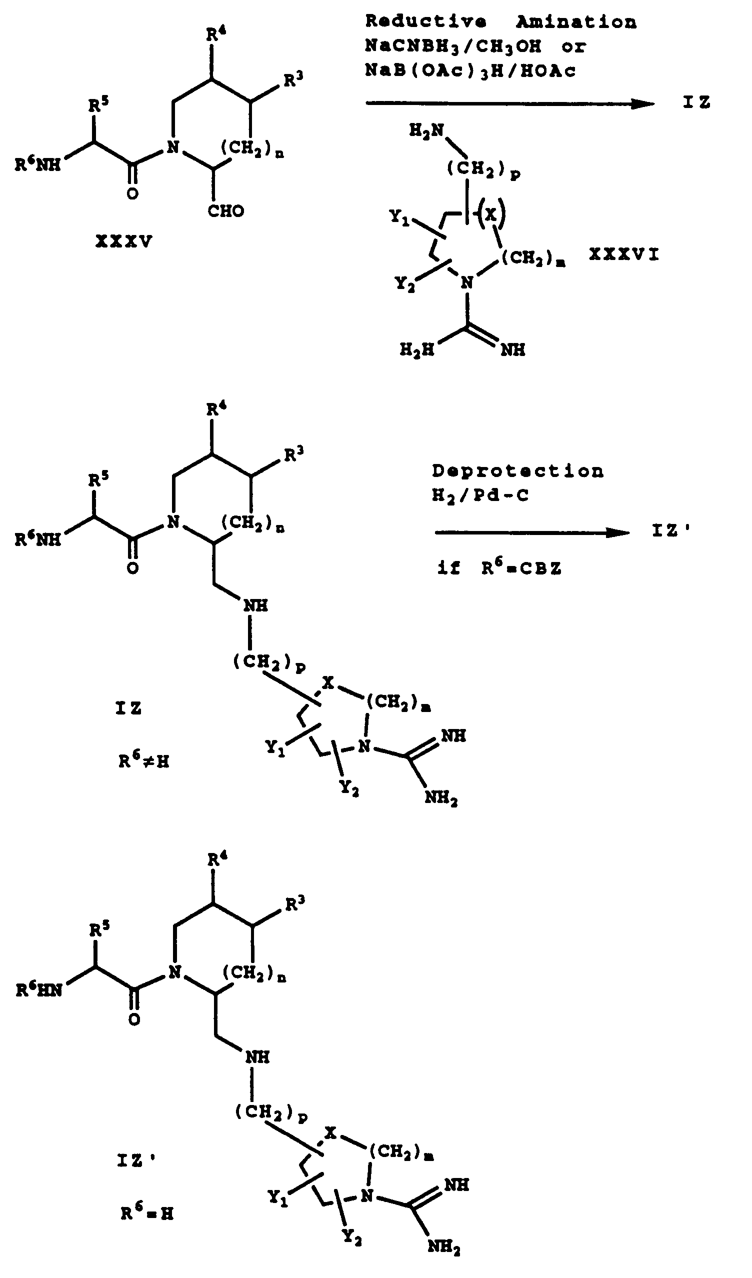 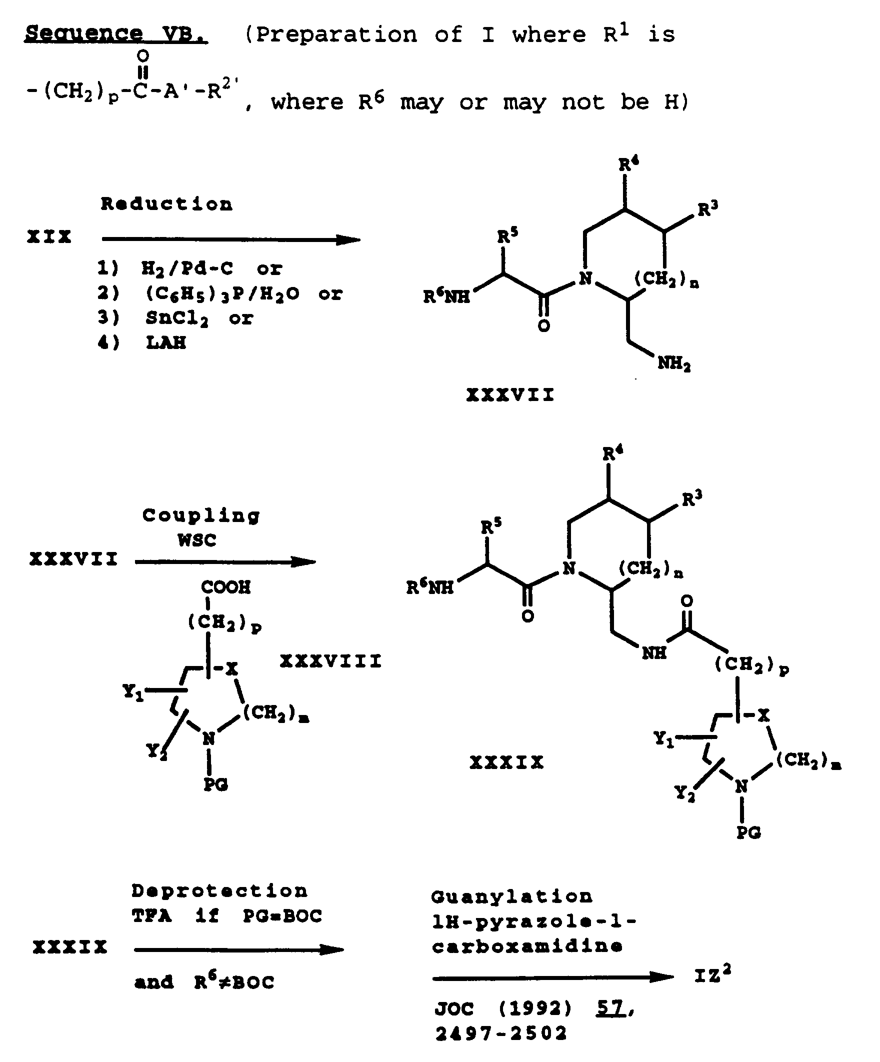 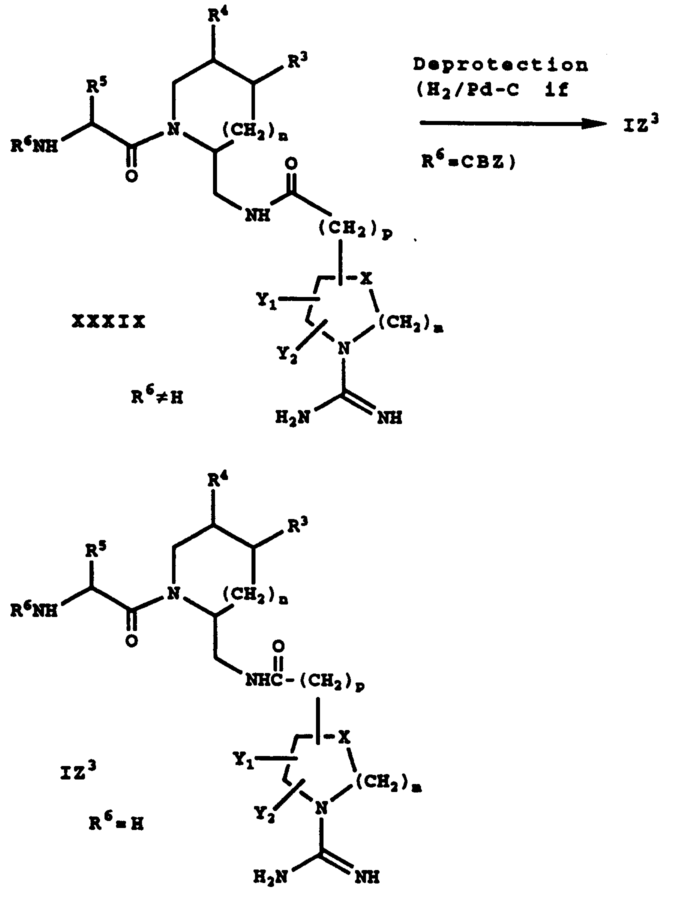
As seen in Reaction Sequence I, Part A, compounds of formula I of the invention wherein R¹ is 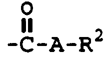
and R² is guanidine are prepared as follows. Tosylate II (prepared as described by J. Das et al, J. Med. Chem., 1992, Vol. 35, 2610) (wherein P.G. represents a protecting group such as t-butyloxy carbonyl (BOC), carbobenzyloxy (CBZ), or fluorenylmethyloxycarbonyl (FMOC) is made to undergo a displacement reaction with sodium azide in the presence of an inert organic solvent such as dimethylsulfoxide (DMSO) or dimethylformamide (DMF), at a temperature within the range of from about 50 to about 90°C, to form azide III which is reduced by reaction with a reducing agent such as H₂/Pd-C or triphenylphospine/H₂O, lithium aluminum hydride (LAH) or stannous chloride, to form the corresponding amine IV.

The amine IV is made to undergo a carbodiimide coupling reaction with protected amino acid A (where PG' is a protecting group such as any of the PG protecting groups set out above) in the presence of ethyl 3-(3-dimethylamino)propyl carbodiimide hydrochloride (WSC) or dicyclohexylcarbodiimide (DCC), and 1-hydroxybenzotriazole monohydrate (HOBT), and N-methylmorpholine (NMM), in the presence of an inert organic solvent such as dimethylformamide (DMF), THF or N-methylpyrrolidone (NMP), to form the amide V. Amide V is deprotected by treatment with trifluoroacetic acid (TFA) (where PG is BOC) or with H₂/Pd-C (where PG is CBZ) with or without the presence of dry inert organic solvent such as dichloromethane, chloroform or THF, at temperatures within the range of from about -15° to about 20°C, to form amide VI. Amide VI is then made to undergo a carbodiimide coupling reaction with protected amino acid B in the presence of WSC or DCC, HOBT and NMM, as described above with respect to amine IV, to form compound VII. The protecting group PG of VII is removed by reacting VII with TFA where PG is BOC and PG' is other than BOC, to form partially deprotected compound VIII. Compound VIII is then subjected to a carbodiimide coupling reaction wherein VIII is treated with an acid R⁷COOH in the presence of WSC or DCC, and HOBT, and NMM, in the presence of an inert organic solvent such as dimethylformamide (DMF), THF or N-methylpyrrolidone, to form compound IX. Alternatively, VIII may be treated with R⁷SO₂Cl and triethylamine or R⁷OCOCl and triethylamine to form IX.

Compound IX is then treated with H₂/Pd-C or HBr/acetic acid (HOAc) (where PG' is CBZ) to form compound of the invention IA. IA is separated by conventional procedures and the desired isomers are guanylated with amidine sulfonic acid, or other guanylation agent such as 1H-pyrazole-1-carboxamidine in the presence of an alcohol solvent such as ethanol to form the compound of the invention IB.

Referring to Reaction Sequence I, Part B1) compounds of formula I of the invention wherein R⁶ is H and R¹ is -CO-A- guanidine may be prepared by deprotecting amide VII with TFA where PG' is BOC and PG is not BOC, guanylating the deprotected amide by treatment with amidinesulfonic acid (as described above with regard to IA) and then removing the remaining protecting group PG by treatment with H₂/Pd-C where PG is CBZ, to form compound IC of the invention.

In Reaction Sequence I, Part B2) compounds of formula I of the invention wherein R⁶ is H and R¹ is -CO-A-NH₂ are prepared by deprotecting IA with TFA (where R⁶ is BOC) or with H₂/Pd-C (where R⁶ is CBZ) to form compound ID of the invention.

In Reaction Sequence I, Part C, compounds of formula I of the invention where R¹ is -CO-A-R² and R² is amidine may be prepared by treating amine IV with acid C in the presence of WSC, HOBT and triethylamine, at a temperature within the range of from about -30° to about 50°C, to form nitrile X which is made to undergo amidine formation by treating X with HCl in the presence of methanol and then reacting the resulting compound with ammonia in the presence of methanol to form amidine XI. The protecting group, PG, is removed in this process if PG=BOC. Amidine XI is then made to undergo a carbodiimide coupling reaction with protected amino acid B in the presence of WSC or DCC, and HOBT and NMM (as described above with respect to amide IV) to form amidine XII which is deprotected with TFA (where PG' is BOC) to form compound IE of the invention where R⁶ is H.

Amidine IE may be coupled with coupling agent R⁷COOH, R⁷OCOCl, or R⁷SO₂Cl (as described above with respect to amide VIII) to form amidine compound IF of the invention where R⁶ is not H.

In Reaction Sequence II, Part A compounds of formula I of the invention where R¹ is -A-R² and R² is amino or guanidine are prepared starting with amine IV which is acylated by treating amine IV with trifluoroacetic anhydride in the presence of an inert organic solvent such as dichloromethane, chloroform or ether and a weak organic base such as pyridine, triethylamine or NMM, to form trifluoroacetamide XIII which is alkylated by treating XIII with bromoalkylphthalimide C in the presence of a base such as an alkali metal carbonate, and an inert organic solvent DMSO or DMF, to form protected phthalimide derivative XIV. The phthalimide derivative XIV is deprotected, for example, by treatment with TFA where PG is BOC, to form phthalimide derivative XV which is coupled with amino acid derivative D in the presence of WSC or DCC, and HOBT and NMM as described hereinbefore in Reaction Sequence I with respect to amine IV, to form phthalimide derivative XVI. Phthalimide derivative XVI is then deprotected by treatment with hydrazine or methyl hydrazine in the presence of an alcohol solvent such as ethanol, and then with base such as potassium carbonate or cesium carbonate, and methanol to form amine compound IG of the invention. IG may then be guanylated as described hereinbefore in Sequence I, Part A with respect to IA, to form guanidine compound IH of the invention.

Compounds of formula I of the invention wherein R⁶ is H and R¹ is -A-R² and R² is guanidine or amine are prepared as outlined in Reaction Sequence II, Part B1) and 2), respectively, by deprotecting IH (where R⁶ is CBZ) or deprotecting IG (where R⁶ is CBZ), with H₂/Pd-C to form guanidine compound IJ and amino compound IK of the invention.

In Reaction Sequence II, Part C, compounds of formula I where R¹ is -A-R² and R² is amidine are prepared by alkylating compound XIII with nitrile E in the presence of a base such as an alkali metal carbonate like cesium carbonate, to form nitrile XVII which is deprotected with TFA (where PG is BOC) and then subjected to a carbodiimide coupling reaction with amino acid D and WSC or DCC, and HOBT and NMM as described hereinbefore with respect to VI in Sequence I, Part A, to form nitrile XVIII. Nitrile XVIII is then treated with HCl and ammonia in the presence of an alcohol solvent such as ethanol to form amidine compound IL of the invention. If in compound XVIII, R⁶ is BOC, then in the final product IL, R⁶ will be H.

Compounds of formula I of the invention wherein R¹ is -SO₂-A-R² and R² is guanidine are prepared as outlined in Reaction Sequence III, Part A starting with azide III which is deprotected with TFA (where PG is BOC) and then subjected to a carbodiimide coupling reaction by treating the so-formed, deprotected compound with amino acid D in the presence of WSC or DCC, and HOBT and NMM (as described in Sequence I for VI) to form azide XIX. Azide XIX is reduced by treating XIX with H₂/Pd-C or other reducing agent as described in Sequence I with respect to the reduction of III) to form the corresponding amine which is sulfonylated by treatment with the sulfonyl chloride F in the presence of dichloromethane or other inert solvent such as chloroform or ether, and a weak organic base such as triethylamine or pyridine, at a temperature of within the range of from about -30° to about 50°C, to form bromosulfonamide XX. Compound XX is then reacted with sodium azide in the presence of an inert organic solvent such as DMF or DMSO to form azide XXI which is deprotected and then guanylated (as described with respect to IX in Sequence I, Part A) to form guanidine compound IM of the invention. IM may then be deprotected where R⁶ is CBZ or BOC as shown to form compound IN of the invention.

In Reaction Sequence III, Part B1) the preparation of compounds of formula I wherein R¹ is -SO₂-A-NH₂ and R⁶ is H is shown starting with azide XXI which is reduced to form the amine IO where R⁶ is H.

In Part B2), compounds of formula I wherein R¹ is -SO₂-A-NH₂ and R⁶ is other than H is formed starting with azide XXI which is deprotected and then coupled with R⁷COOH, R⁷SO₂Cl or R⁷OCOCl (as described above in Sequence I with respect to VIII) to form amine IP.

Compounds of formula I of the invention wherein R¹ is -SO₂-A-amidine are prepared as shown in Reaction Sequence IIIC by subjecting compound XX to a displacement reaction by reacting XX with cyanide to form nitrile XXII which is then amidinated by treatment with HCl and NH₃ in the presence of ethanol (as described hereinbefore in Sequence I, Part C with respect to IV) to form amidine IQ of the invention wherein if R⁶ in XXIII is BOC, then R⁶ in IQ is H.

Preparation of compounds of formula I of the invention wherein R¹ is -A''-R^{2''} and A^{2''} is amidine is outlined in Reaction Sequence IV, Part A starting with alcohol XXIV which is oxidized by treatment with, for example, oxalyl chloride or other oxidizing agent such as pyridine·SO₃ or pyridinum dichromate, in the presence of an inert organic solvent such as DMSO, ether or methylene chloride, and a weak organic base such as triethylamine to form aldehyde XXV which is made to undergo reductive amination by treating XXV with NaCNBH₃ in the presence of methanol, or NaB(OAc)₃H in the presence of acetic acid and dichloroethylene, and nitrile F to form nitrile XXVI. Nitrile XXVI is reacted with HCl and then ammonia in the presence of ethanol to form amidine XXVII wherein the protecting group PG is removed if PG=BOC. So-formed amidine XXVII is made to undergo a carbodiimide coupling reaction by treating XXVII with amino acid coupling agent B in the presence of WSC or DCC, and HOBT (as described hereinbefore in Sequence I, Part A with respect to VI) to form amidine XXVIII which is deprotected to form amidine compound IR of the invention where R⁶ is H.

Amidine IR may be subjected to a coupling reaction with R⁷COOH, R⁷SO₂Cl or R⁷OCOCl (as described hereinbefore in Sequence I, Part A with respect to VIII) to form amidine compound IS of the invention wherein R⁶ is other than H.

Compounds of formula I of the invention wherein R¹ is -A''-CH₂NH₂ are prepared as shown in Reaction Sequence IV, Part B wherein aldehyde XXV is subjected to a reductive amination employing protected amine G and the procedure as described in Sequence IV, Part A with respect to aldehyde XXV to form protected amine XXIX which is deprotected and coupled with coupling agent B (as described in Sequence I, Part A with respect to VI) to form protected amine XXX which is deprotected to form compound XXXI. Compound XXXI may then be deprotected to form amine compound IT of the invention where R⁶ is H or XXXI may be made to undergo a coupling reaction (as described in Sequence I, Part A with respect to VIII) to form amine compound IU of the invention.

In Reaction Sequence IV, Part C compounds of formula I wherein R¹ is -A''-R^{2''} and R^{2''} is guanidine are prepared starting with XXV which is deprotected and subjected to a carbodiimide coupling with amino acid D (as described hereinbefore in Sequence II, Part C) to form aldehyde XXXII. Aldehyde XXXII is then made to undergo reductive amination with amine H (using procedures as described in Sequence IV, Part A with respect to XXI) to form compound XXXIII which is deprotected and guanylated (as described in Sequence I, Part A with respect to IA) to form guanidine compound IW of the invention. Where R⁶ in IW is CBZ, IW may be reduced with H₂/Pd-C to form guanidine compound IY of the invention.

As seen in reaction sequence V, part A, compounds of formula IZ and IZ' may be prepared by deprotecting previously described alcohol XXIV and coupling the free amine with a protected amino acid to give compound XXXIV. The alcohol XXXIV is oxidized to aldehyde XXXV as described previously in the preparation of compound XXV. The aldehyde is coupled with a compound of formula XXXVI using the reductive amination methodology described in the preparation of compound XXIX to give compound IZ. In the case where R⁶ = H, IZ can be deprotected (Pd-C/H₂ if R⁶ = CBZ) to provide compound IZ'. Compounds of formula IZ² and IZ³ can be prepared by reduction of azide XIX as described in reaction sequence III A to give the amine XXXVII. This amine is coupled to an acid of formula XXXVIII using standard carbodiimide coupling methodology described in the preparation of V to give a compound of formula XXXIX. Compound XXXIX is deprotected (TFA if PG = BOC) and guanylated with a reagent such as 1H-pyrazole-1-carboxamidine to provide a compound of formula IZ². In the case where R⁶ = CBZ, the compounds of formula IZ² can be deprotected to give a compound of formula IZ³ where R⁶ = H.

The compounds of formula I of the invention can be obtained as pharmaceutically acceptable acid addition salts by reacting a free base with an acid, such as hydrochloric, hydrobromic, hydroiodic, nitric, sulfuric, phosphoric, acetic, citric, maleic, succinic, lactic, tartaric, gluconic, benzoic, methanesulfonic, ethanesulfonic, benzenesulfonic, p-toluenesulfonic acid or the like.

The compounds of the present invention are serine protease inhibitors, and in particular may inhibit thrombin, Factor Xa, and/or trypsin. The compounds of the present invention are useful for the treatment or prophylaxis of those processes which involve the production and/or action of thrombin. This includes a number of thrombotic and prothrombotic states in which the coagulation cascade is activated which include, but are not limited to, deep vein thrombosis (DVT), disseminated intravascular coagulopathy (DIC), Kasabach-Merritt syndrome, pulmonary embolism, myocardial infarction, stroke, thromboembolic complications of surgery (such as hip replacement and endarterectomy) and peripheral arterial occlusion. In addition to its effects on the coagulation process, thrombin has been shown to activate a large number of cells (such as neutrophils, fibroblasts, endothelial cells, smooth muscle cells). Therefore, the compounds of the present invention may also be useful for the treatment or prophylaxis of adult respiratory distress syndrome, septic shock, septicemia, inflammatory responses which include, but are not limited tot edema, acute or chronic atherosclerosis, and reperfusion damage.

The compounds of the invention may also be useful in treating neoplasia/metastasis (in particular those which utilize fibrin) and neurodegenerative diseases such as Alzheimer's disease and Parkinson's disease. In addition, the compounds of the present invention may be useful to prevent restenosis following arterial injury induced by endogenous (rupture of an atherosclerotic plaque) or exogenous (invasive cardiological procedure) events.

The compounds of the present invention may also be used as an anticoagulant in extracorpeal blood circuits, such as those necessary in dialysis and surgery (such as coronary artery bypass surgery).

The compounds of the present invention may also be used in combination with thrombolytic agents, such as tissue plasminogen activator (natural or recombinant), streptokinse, urokinase, prourokinase, anisolated streptokinase plasminogen activator complex (ASPAC), animal salivary gland plasminogen activators, and the like. The compounds of the present invention may act in a synergistic fashion to prevent reocclusion following a successful thrombolytic therapy and/or reduce the time to reperfusion. The compounds of the present invention may also allow for reduced doses of the thrombolytic agent to be used and therefore minimize potential hemorrhagic side-effects.

The compounds of the present invention may also be used in combination with other antithrombotic or anticoagulant drugs such as thromboxane receptor antagonists, prostacyclin mimetics, phosphodiesterase inhibitors, fibrinogen antagonists, and the like.

Compounds of the present invention that inhibit trypsin may also be useful for the treatment of pancreatitis.

The compounds of the invention can be administered orally or parenterally to various mammalian species known to be subject to such maladies, e.g., humans, cats, dogs and the like in an effective amount within the dosage range of about 0.1 to about 100 mg/kg, preferably about 0.2 to about 50 mg/kg and more preferably about 0.5 to about 25 mg/kg (or from about 1 to about 2500 mg, preferably from about 5 to about 2000 mg) on a regimen in single or 2 to 4 divided daily doses.

The active substance can be utilized in a composition such as tablet, capsule, solution or suspension containing about 5 to about 500 mg per unit of dosage of a compound or mixture of compounds of formula I. They may be compounded in conventional matter with a physiologically acceptable vehicle or carrier, excipient, binder, preservative, stabilizer, flavor, etc., as called for by accepted pharmaceutical practice.

The following Examples represent preferred embodiments of the present invention. Unless otherwise indicated, all temperatures are expressed in degrees Centigrade.

### Example 1

### [S-(R*,R*)]-4-Amino-N-[[1-[3-hydroxy-2-[(2-naphthalenylsulfonyl)amino]-1-oxopropyl]-2-pyrrolidinyl]methyl]butanamide, hydrochloride

### A. (S)-2-(Azidomethyl)-1-pyrrolidinecarboxylic acid, 1,1-dimethylethyl ester

(S)-2-[[[(4-Methylphenyl)sulfonyl]oxy]methyl]-1-pyrrolidinecarboxylic acid, 1,1-dimethylethyl ester (3.55g, 10mmol, see J. Das et al, J. Med Chem, 1992, Vol.35,2610 for preparation) was dissolved in dimethyl sulfoxide (DMSO) (40 mL) and treated with sodium azide (980 mg, 15 mmol). The mixture was heated at 70°C for 5 hours, then cooled and diluted with ether. After washing with water (3x50 mL), the ether layer was dried (MgSO₄ ) and freed of solvent in vacuo to give title compound as a colorless oil (2.05 g, 91%) which was used without further purification.

### B. N-BOC-L-Proline Methylamine

The Part A azide (1.45g, 6.4 mmol) was dissolved in absolute ethanol (50mL), and treated with 10% palladium on carbon (290 mg). The flask was equipped with a hydrogen filled balloon via a three way stopcock. Air inside the flask was evacuated under reduced pressure and the flask was then filled with hydrogen from the balloon. This process was repeated three times. The mixture was stirred overnight in the hydrogen atmosphere. The catalyst was then removed by filtration through a pad of MgSO₄ and the pad was washed with more ethanol. The solvent was removed in vacuo to give title compound as a colorless oil (1.19 g, 93%).

### C. (S)-2-[[[1-Oxo-4-[[(phenylmethoxy)carbonyl]amino]butyl]amino]methyl]-1-pyrrolidinecarboxylic acid, 1,1-dimethylethyl ester

Part B N-BOC-L-proline methylamine (1.04 g, 5.2 mmol) and N-CBZ-4-aminobutyric acid (1.24g, 5.2 mmol) were dissolved in dimethyl formamide (DMF) (20 mL) at room temperature (RT). HOBT (702mg, 5.2 mmol), WSC (995mg, 5.2mmol) and NMM (570µL, 5.2 mmol) were added. The reaction was stirred for 16 hours (h), partitioned between ethyl acetate(50 mL) and 10% KHSO₄ (100 mL). The aqueous layer was extracted with ethyl acetate (2x50mL), and the organic layers were combined. The combined organic layers were washed with saturated NaHCO₃ (50 mL) and saturated NaCl (50mL), dried over magnesium sulfate and concentrated in vacuo to provide an oil. The crude material was purified by chromatography on silica gel. Elution with ethyl acetate:hexanes(2:1) followed by ethyl acetate gave title compound, (1.809 g, 83%) as a viscous oil which was characterized by NMR and used in the next step.

### D. (S)-4-[[(Phenylmethoxy)carbonyl]amino]-N-(2-pyrrolidinylmethyl)butanamide, trifluoroacetate salt

Part C BOC derivative (915 mg, 2.19 mmol) was dissolved in trifluoroacetic acid (TFA) (15 mL) and stirred at RT 80 min. The TFA was removed by distillation under reduced pressure and by coevaporation with toluene to give title amine.

### E. [S-(R*,R*)]-[2-Oxo-2-[2-[[[1-oxo-4-[[(phenylmethoxy)carbonyl]amino]butyl]amino]methyl]-1-pyrrolidinyl]-1-[(phenylmethoxy)methyl]ethyl]carbamic acid, 1,1-dimethylethyl ester

The Part D crude TFA salt (2.19 mmol) and BOC-Ser(OBn)-OH (710mg, 2.41 mmol) were dissolved in DMF (10 mL) at RT. HOBT (326 mg, 2.41 mmol), WSC (463mg, 2.41 mmol) and NMM (570 µL) were added. The reaction was stirred for 18 h, partitioned between ethyl acetate (50 mL) and saturated KHSO₄ solution (50 mL). The aqueous layer was reextracted with ethyl acetate (2x50 mL) and the organic layers were combined. The combined organic layers were washed with saturated NaHCO₃ (30 mL) and saturated NaCl, dried over magnesium sulfate and concentrated in vacuo. The remaining yellow oil was purified by chromatography on silica gel. Elution with ethyl acetate gave title compound, (611mg, 47%) which was characterized by NMR.

### F. [S-(R*,R*)]-N-[[1-[2-[(2-Naphthalenylsulfonyl)amino]-1-oxo-3-(phenylmethoxy)propyl]-2-pyrrolidinyl]methyl]-4-[[(phenylmethoxy)carbonyl]amino]butanamide

Part E BOC derivative (610 mg, 1.02 mmol) was dissolved in TFA (15 mL) and stirred at RT 100 min. The TFA was removed by distillation under reduced pressure and by coevaporation with toluene. The crude TFA salt was dissolved in dichloromethane (15 mL), cooled in an ice bath, and 2-naphthylsulfonyl chloride (Aldrich, 254mg, 1.12 mmol) was added, followed by triethylamine (855 µL, 6.12 mmol). The solution was warmed to RT and stirred 1.75 h before diluting with dichloromethane (50 mL). This solution was washed with potassium hydrogen sulfate solution (2x30mL) and saturated NaHCO₃ (2x30mL), dried over magnesium sulfate, and evaporated to provide crude title compound. The crude product was chromatographed on silica gel and eluted with EtOAc:hexanes (2:1) followed by EtOAc followed by 5%MeOH in CH₂Cl₂ to provide title sulfonamide as a foam (384mg, 72% overall in two steps).

### G. [S-(R*,R*)]-4-Amino-N-[[1-[3-hydroxy-2-[(2-naphthalenylsulfonyl)amino]-1-oxopropyl]-2-pyrrolidinyl]methyl]butanamide, hydrochloride

Part F compound (480 mg, 0.71 mmol) was dissolved in ethanol (60 mL) to which acetyl chloride (1.4 mL) had been added and the mixture was hydrogenated over 10%Pd-C (150 mg) at 55 psi. After 17 h, additional catalyst (100 mg) and acetyl chloride (0.5 mL) were added. After an additional 18 h, the reaction mixture was filtered and the residual catalyst was washed with EtOH. The filtrate was concentrated in vacuo to obtain title des-benzyl, des-CBZ product.

### Example 2

### [S-(R*,R*)]-4-[(Aminoiminomethyl)amino]-N-[[1-[3-hydroxy-2-[(2-naphthalenylsulfonyl)amino]-1-oxopropyl]-2-pyrrolidinyl]methyl]butanamide, trifluoroacetate (1:1) salt

Example 1 compound (0.71 mmol) was dissolved in ethanol (20 mL), to which amidinesulfonic acid (124 mg, 1.0 mmol) and triethylamine (270 µL, 1.9 mmol) were added. After 2.5 h the mixture was filtered through a pad of celite, washed with ethanol and the filtrate was concentrated to dryness. The crude material was purified by preparative HPLC to provide a white solid (218 mg, 47% overall yield from Example 1, Part F), Purity ≧ 98%.
[α]_{D} = -37.2^{o} (c = 0.9, MeOH)

| Analysis calcd for 1.25 TFA + 0.60 H₂O: | | | | | |
|---|---|---|---|---|---|
| | C, 46.55; | H, 5.28; | N, 12.77; | F, 10.83; | S, 4.87. |
| Found: | C, 46.54; | H, 5.56; | N, 12.69; | F, 10.80; | S, 4.93. |

### Example 3

### [R-(R*,S*)]-[2-[2-[[(4-Aminobutyl)amino]methyl]-1-pyrrolidinyl]-2-oxo-1-(phenylmethyl)ethyl]carbamic acid, phenylmethyl ester

### A. (S)-2-[[(Trifluoroacetyl)amino]methyl]-1-pyrrolidinecarboxylic acid, 1,1-dimethylethyl ester

A solution of Example 1, Part B N-BOC-L-prolinemethylamine compound (2.64g, 13.27mmol) in dichloromethane (50 mL) and pyridine (10 mL) was cooled to 0° C and treated dropwise with trifluoroacetic anhydride (3.78 mL, 26.55 mmol). The reaction was allowed to warm slowly to room temperature and left stirring overnight. The mixture was diluted with ethyl acetate (150 mL) and washed with satd. copper sulfate solution (5 x 50 mL) and brine (3 x 30 mL) dried (MgSO₄ ) and freed of solvent in vacuo to give title compound as a yellow oil (3.516 g, 84%) which was used without further purification.

### B. (S)-2-[[[4-(1,3-Dihydro-1,3-dioxo-1H-isoindol-2-yl)butyl](trifluoroacetyl)amino]methyl]-1-pyrrolidinecarboxylic acid, 1,1-dimethylethyl ester

The Part A trifluoroacetamide (3.514g, 11.12 mmol) and N-(4-bromobutyl)-phthalimide (3.136 g, 11.12 mmol) were dissolved in DMF (60mL), and treated with cesium carbonate (7.25g, 22.24 mmol). The reaction was heated in an oil bath maintained at 55°C for 22 hours, cooled to room temperature and partioned between ethyl acetate (150 mL) and water (100 mL). The layers were separated, and the aqueous layer was reextracted with ethyl acetate (50 mL). The combined organic layers were dried over magnesium sulfate and concentrated in vacuo. The crude material was purified by chromatography on silica gel (Merck, 400 mL), eluting with 20-40% ethyl acetate in hexane to give title compound (2.773 g, 50%) as a viscous foam.

### C. [R-(R*,S*)]-[2-[2-[[[4-(1,3-Dihydro-1,3-dioxo-2H-isoindol-2-yl)butyl](trifluoroacetyl)amino]methyl]-1-pyrrolidinyl]-2-oxo-1-(phenylmethyl)ethyl]carbamic acid, phenylmethyl ester

Part A BOC derivative (2.27g, 4.567 mmol) was dissolved in dichloromethane (10 mL) and TFA (30mL) and stirred at RT 2.5 hours. The TFA was removed by distillation under reduced pressure and by coevaporation with toluene to give the TFA salt of the amine. This and Z-D-phenylalanine (1.50 g, 5.02 mmol)were dissolved in DMF (20 mL) at RT. HOBT (680mg, 5.02 mmol), WSC (965mg, 5.02mmol) and NMM (1.1mL, 10 mmol) were added. The reaction was stirred for 16 h, partitioned between ethyl acetate (70 mL) and 10% KHSO₄ (70 mL). The aqueous layer was extracted with ethyl acetate (2x70mL), and the organic layers were combined. The combined organic layers were washed with saturated NaHCO₃ (50 mL) and saturated NaCl (50mL), dried over magnesium sulfate and concentrated in vacuo to provide a viscous oil. The crude material was purified by chromatography on silica gel. Elution with ethyl acetate:hexanes(1:1) followed by ethyl acetate gave title phthalimide, (2.43 g, 78%) as a viscous oil which was characterized by NMR.

### D. [R-(R*,S*)]-[2-[2-[[(4-Aminobutyl)amino]methyl]-1-pyrrolidinyl]-2-oxo-1-(phenylmethyl)-ethyl]carbamic acid, phenylmethyl ester

Part C phthalimide (1.79g, 2.63 mmol) was dissolved in ethanol (12 mL) and treated with methyl hydrazine (2mL). The mixture was heated under reflux for 2 hours at which time the HPLC indicated that no starting material remained, but showed a mixture of products. The solvent was removed in vacuo and by co-evaporation with toluene. The residue was dissolved in methanol (50 mL) and water (15 mL) and treated with potassium carbonate (2 g). The mixture was left stirring overnight at room temperature to complete the removal of the trifluoroacetamide group. 1N HCl solution was added to acidify the mixture and non-basic organics were removed by extracting with dichloromethane (70 mL). The dichloromethane layer was extracted with 1N HCl (50 mL). The combined aqueous layers were basified with NaOH solution and the desired amine was extracted into dichloromethane (3 x 70 mL), dried over magnesium sulfate and concentrated in vacuo to provide title compound as an oil (1.065 g, 90%).

### Example 4

### [R-(R*,S*)]-[2-[2-[[[4-[(Aminoiminomethyl)amino]butyl]amino]methyl]-1-pyrrolidinyl]-2-oxo-1-(phenylmethyl)ethyl]carbamic acid, phenylmethyl ester, trifluoroacetate (1:2) salt

Example 3 compound (1.069g, 2.356 mmol) was dissolved in ethanol (60 mL), to which amidinesulfonic acid (352 mg, 2.84 mmol) and triethylamine (550 µL, 3.94 mmol) were added. After 3.5 h the mixture was filtered through a pad of celite, washed with ethanol and the filtrate was concentrated to dryness. The crude material was purified by preparative HPLC to provide a white solid (1.225g, 69%), Purity ≧ 98%.
[α]_{D} = -2.9^{o} (c = 0.5 MeOH)

| Analysis calcd for 2.20TFA + 0.70 H₂O: | | | | |
|---|---|---|---|---|
| | C, 49.75; | H, 5.53; | N, 11.09; | F, 16.54. |
| Found: | C, 49.71; | H, 5.48; | N, 11.09; | F, 16.57. |

### Example 5

### (2S)-N-[4-[[[1-(D-Phenylalanyl)-2-pyrrolidinyl]methyllamino]butyl]guanidine, trifluoroacetate (1:2) salt

Example 4 compound (540 mg, 0.712 mmol) was dissolved in methanol (30 mL)and treated with Perlman's catalyst (Pd(OH)₂) (125 mg). The flask was equipped with a hydrogen filled balloon via a three-way stopcock. Air inside the flask was removed under reduced pressure and replaced with hydrogen from the balloon. This process was repeated three times. The mixture was stirred under the hydrogen atmosphere for 2.5 hours. The catalyst was removed by filtration and the filter pad was washed with additional methanol. The filtrate was freed of solvent in vacuo to give title compound which was dissolved in water (30 mL), passed through a millipore membrane and lyophilized to provide title compound as a white solid (429mg, 93%), Purity ≧ 98%.

| Analysis calcd for 2.50 TFA + 0.20 H₂O: | | | | |
|---|---|---|---|---|
| | C, 44.41; | H, 5.72; | N, 12.95; | F, 21.95. |
| Found: | C, 44.49; | H, 5.70; | N, 12.86; | F, 21.91. |

### Example 6

### [R-(R*,S*)]-[2-[2-[[[(4-Aminobutyl)sulfonyl]amino]methyl]-1-pyrrolidinyl]-2-oxo-1-(phenylmethyl)ethyl]carbamic acid 1,1-dimethylethyl ester

### A. 4-Bromobutylsulfonylchloride

A stirred suspension of anhydrous sodium bromide (1.13g, 11mmol) and 1,4-butane sultone (1.36g, 10 mmol) in DMF (10 mL) was heated in a bath maintained at 80° C for 2 hours. The clear solution was cooled to room temperature and a white solid precipitated. The mixture was diluted with ethyl acetate and filtered. The white solid collected was washed five times with ether and dried in vacuo to give the sodium salt of 4-bromobutyl sulfonic acid (2.18 g). Solid phosphorous pentachloride (3.12 g, 15 mmol) was added to the sodium salt in several portions with mixing. After addition, the solid mixture was triturated with a spatula. An exothermic reaction ensued, the mixture became colored and became fluid. After stirring at room temperature for 30 min., the mixture was diluted with dichloromethane and treated slowly with ice water. The layers were separated and the dichloromethane solution was dried (MgSO₄), filtered and the solvent was removed in vacuo . The crude colorless oil was purified on silica gel (Merck) eluting with 50% ethyl acetate in hexane to give title compound as a colorless oil (1.69 g, 72%).

### B. [R-(R*,S*)]-[2-[2-(Aminomethyl)-1-pyrrolidinyl]-2-oxo-1-(phenylmethyl)ethyl]carbamic acid, 1,1-dimethylethyl ester

### (1) [R-(R*,S*)]-[2[2-(Azidomethyl)-1-pyrrolidinyl]-2-oxo-1-(phenylmethyl)ethyl]carbamic acid, 1,1-dimethylethyl ester

Example 1, Part A azide (4.52g, 20mmol) was dissolved in dichloromethane (5mL) and trifluoroacetic acid (10mL) and stirred at RT 2 hours. The TFA was removed by distillation under reduced pressure and by coevaporation with toluene to give the amine as a TFA salt. This and t-BOC-D-phenylalanine (5.3g, 20 mmol) were dissolved in DMF (30 mL). HOBT (2.7g, 20mmol), WSC (4g, 20mmol) and NMM (8 mL) were added. The reaction was stirred for 24 h, diluted with ethyl acetate(75 mL) and washed with 10% KHSO₄ (2x), saturated sodium bicarbonate solution (2x) and 10% lithium chloride solution (2x). The ethyl acetate layer was dried over magnesium sulfate and concentrated in vacuo to provide title compound (7.4g, 99%) as an oil which was used without further purification.

### (2) [R-(R*,S*)]-[2-[2-(Aminomethyl)-1-pyrrolidinyl]-2-oxo-1-(phenylmethyl)ethyl]carbamic acid, 1,1-dimethylethyl ester

The Part (1) azide (2.25g, 6.0 mmol) was dissolved in absolute ethanol (40mL), and treated with 10% palladium on carbon (200 mg). The flask was equipped with a hydrogen filled balloon via a three way stopcock. Air inside the flask was evacuated under reduced pressure and the flask was then filled with hydrogen from the balloon. This process was repeated three times. The mixture was stirred overnight in the hydrogen atmosphere. The catalyst was then removed by filtration through a pad of MgSO₄ and the pad was washed with more ethanol. The solvent was removed in vacuo to give title amine as a white foam (2.15 g, Quant).

### C. [R-(R*,S*)]-[2-[2-[[[(4-Bromobutyl)sulfonyl]amino]methyl]-1-pyrrolidinyl]-2-oxo-1-(phenylmethyl)ethyl]carbamic acid, 1,1-dimethylethyl ester

A solution of Part A 4-bromobutyl sulfonyl chloride (942 mg, 4 mmol) in dichloromethane (3 mL) was added dropwise to a cooled (-10°C) stirred solution of Part B amine (868 mg, 2.5 mmol) and triethyl amine (1.2 mL, 8 mmol) in dichloromethane (10 mL). After a few minutes, a precipitate formed. The suspension was stirred at -10°C for 2 hours, diluted with dichloromethane (25 mL) and washed with 1 N HCl solution (2x15 mL). The dichloromethane solution was dried (MgSO₄), filtered and the solvent was removed in vacuo. The crude oil was purified on silica gel (Merck) eluting with 30%, 50%, and 70% ethyl acetate in hexane to give title compound as a white foam (910 mg, 67%).

### D. [R-(R*,S*)]-[2-[2-[[[(4-Azidobutyl)sulfonyl]amino]methyl]-1-pyrrolidinyl]-2-oxo-1-(phenylmethyl)ethyl]carbamic acid, 1,1-dimethylethyl ester

A solution of Part C bromo sulfonamide (800 mg, 1.46 mmol) in DMSO (5 mL) was treated with sodium azide (143 mg, 2.2 mmol) and the mixture was heated in an oil bath maintained at 60°C for 3.5 hours. After cooling, the mixture was partitioned between ether (50 mL) and water (25 mL). The ether layer was washed with water (2x10 mL), dried (MgSO₄), filtered and the solvent was removed in vacuo. The crude oil was purified on silica gel (Merck) eluting with 50% ethyl acetate in hexane to give title compound as an oil (733 mg, 98%).

### E. [R-(R*,S*)]-[2-[2-[[[(4-Aminobutyl)sulfonyl]amino]methyl]-1-pyrrolidinyl]-2-oxo-1-(phenylmethyl)ethyl]carbamic acid, 1,1-dimethylethyl ester

Part D compound (730 mg, 1.437 mmol) was dissolved in ethanol (20 mL)and treated with 10% palladium on carbon (180 mg). The flask was equipped with a hydrogen filled balloon via a three-way stopcock. Air inside the flask was removed under reduced pressure and replaced with hydrogen from the balloon. This process was repeated three times. The mixture was stirred under the hydrogen atmosphere for 18 hours. The catalyst was removed by filtration and the filter pad was washed with additional ethanol. The filtrate was taken to dryness in vacuo leaving title compound as a colorless oil (758 mg, Quant.).

### Example 7

### [R-(R*,S*)]-4-[(Aminoiminomethyl)amino]-N-[[1-(2-amino-1-oxo-3-phenylpropyl)-2-pyrrolidinyl]methyl]butanesulfonamide, trifluoroacetate (1:2) salt

Example 6 amine (1.437 mmol) was dissolved in ethanol (30 mL), to which amidinesulfonic acid (250 mg, 2.0 mmol) and triethyl amine (280µL, 2.0 mmol) were added. After 3 h, the mixture was filtered through a pad of celite, washed with ethanol and the filtrate was concentrated to dryness. The residue was treated with TFA (5 mL) for 3 hours and then freed of excess TFA by coevaporation with toluene. The crude material was purified by preparative HPLC to provide a white solid (523 mg, 55% from Example 6, Part D compound), Purity ≧ 98%.
[α]_{D} = -63.3^{o} (c = 0.6, MeOH).

| Analysis calcd for 2.2 TFA + 0.2 H₂O: | | | | | |
|---|---|---|---|---|---|
| | C, 41.39; | H, 5.14; | N, 12.38; | F, 18.47; | S, 4.72. |
| Found: | C, 41.44; | H, 5.16; | N, 11.89; | F, 18.41; | S, 4.56. |

### Example 8

### [R-(R*,S*)]-[2-[2-[[[(3-Aminopropyl)sulfonyl]amino]methyl]-1-pyrrolidinyl]-2-oxo-1-(phenylmethyl)ethyl]carbamic acid, 1,1-dimethylethyl ester

### A. 3-Bromopropyl sulfonyl chloride

A stirred suspension of anhydrous sodium bromide (1.60g, 15.5 mmol) and 1,3-propane sultone (1.83g, 15 mmol) in DMF (15 mL) was heated in a bath maintained at 80° C for 2 hours, during this time material precipated. The mixture was cooled to room temperature, dilited with ethyl acetate and filtered. The white solid collected was washed five times with ether and dried in vacuo to give the sodium salt of 3-bromopropyl sulfonic acid (3.11g). Solid phosphorous pentachloride (4.68 g, 22.5 mmol) was added to the sodium salt, in several portions, with mixing. After addition, the solid mixture was triturated with a spatula. An exothermic reaction ensued, the mixture became colored and became fluid. After stirring at room temperature for 30 min., the mixture was diluted with dichloromethane and treated slowly with ice water. The layers were separated and the dichloromethane solution was dried (MgSO₄), filtered and the solvent was removed in vacuo. The crude colorless oil was purified on silica gel (Merck) eluting with 30% ethyl acetate in hexane to give title compound as a colorless oil (2.544g, 76%).

### B. [R-(R*,S*)]-[2-[2-[[[(3-Bromopropyl)sulfonyl]amino]methyl]-1-pyrrolidinyl]-2-oxo-1-(phenylmethyl)ethyl]carbamic acid, 1,1-dimethylethyl ester

A solution of Part A 3-bromopropyl sulfonyl chloride (1.06g, 4.8 mmol) in dichloromethane (5 mL) was added dropwise to a cooled (-10°C) stirred solution of Example 6, Part B amine (1.059, 3.02 mmol, see BMS 186,685 for preparation) and triethyl amine (1.44 mL, 9.6 mmol) in dichloromethane (15 mL). After a few minutes, a precipitate formed. The suspension was stirred at -10°C for 2.5 hours, diluted with dichloromethane (35 mL) and washed with 1 N HCl solution (2x15 mL). The dichloromethane solution was dried (MgSO₄), filtered and the solvent was removed in vacuo. The crude oil was purified on silica gel (Merck, 150 mL) eluting with 50% ethyl acetate in hexane to give title compound as a white foam (1.160g, 72%).

### C. [R-(R*,S*)]-[2-[2-[[[(3-Azidopropyl)sulfonyl]amino]methyl]-1-pyrrolidinyl]-2-oxo-1-(phenylmethyl)ethyl]carbamic acid, 1,1-dimethylethyl ester

A solution of Part B bromo sulfonamide (1.10g, 2.06 mmol) in DMSO (8 mL) was treated with sodium azide (202 mg, 3.1 mmol) and the mixture was heated in an oil bath maintained at 60°C for 3.75 hours. After cooling, the mixture was partitioned between ether (60 mL) and water (30 mL). The ether layer was washed with water (2x15 mL), dried (MgSO₄), filtered and the solvent was removed in vacuo. The crude oil was purified on silica gel (Merck, 150mL) eluting with 50% ethyl acetate in hexane to give title compound as a white foam (872 mg, 86%).

### D. [R-(R*,S*)]-[2-[2-[[[(3-Aminopropyl)sulfonyl]amino]methyl]-1-pyrrolidinyl]-2-oxo-1-(phenylmethyl)ethyl]carbamic acid, 1,1-dimethylethyl ester

Part C compound (870 mg, 1.76 mmol) was dissolved in ethanol (25 mL)and treated with 10% palladium on carbon (200 mg). The flask was equipped with a hydrogen filled balloon via a three-way stopcock. Air inside the flask was removed under reduced pressure and replaced with hydrogen from the balloon. This process was repeated three times. The mixture was stirred under the hydrogen atmosphere for 18 hours. The catalyst was removed by filtration and the filter pad was washed with additional ethanol. The filtrate was taken to dryness in vacuo leaving title compound as a colorless oil.

### Example 9

### [R-(R*,S*)]-[2-[2-[[[[3-[(Aminoiminomethyl)amino]propyl]sulfonyl]amino]methyl]-1-pyrrolidinyl]-2-oxo-1-(phenylmethyl)ethyl]carbamic acid, 1,1-dimethylethyl ester, trifluoroacetate (1:1) salt

Example 8 amine (1.76 mmol) was dissolved in ethanol (35 mL) and amidinesulfonic acid (306 mg, 2.46 mmol) and triethylamine (345µL, 2.5mmol) were added. After 3 hours, the mixture was filtered through a pad of celite, washed with ethanol and the filtrate was concentrated to dryness. The crude material was purified by preparative HPLC to provide a white solid (820 mg, 71% from Example 8, Part C compound), Purity ≧ 98%.
[α]_{D} = -38.4^{o} (c = 0.9, MeOH).

| Analysis calcd for 1.25 TFA + 0.2 H₂O: | | | | | |
|---|---|---|---|---|---|
| | C, 46.63; | H, 6.09; | N, 12.80; | F, 10.85; | S, 4.88. |
| Found: | C, 46.87; | H, 6.12; | N, 12.60; | F, 10.83; | S, 4.92. |

### Example 10

### [R-(R*,S*)]-3-[(Aminoiminomethyl)amino]-N-[[1-(2-amino-1-oxo-3-phenylpropyl]-2-pyrrolidinyl]methyl]propanesulfonamide, trifluoroacetate (1:2) salt

Example 9 compound (500 mg, 0,76 mmol) was treated with TFA (5 mL) for 1.5 hours and then freed of excess TFA in vacuo. The residue was dissolved in water (20 mL) and passed through a Millipore filtration membrane. The aqueous solution was frozen and lyophillized to give a dense solid. This was again dissolved in water (20 mL) and relyophillized to give title compound as a fluffy white solid (479mg, 93%), Purity ≧ 98%.
[α]_{D} = -60.2^{o} (c = 0.6, MeOH).

| Analysis calcd for 2.2 TFA + 0.4 H₂O: | | | | | |
|---|---|---|---|---|---|
| | C, 40.24; | H, 4.98; | N, 12.57; | F, 18.75; | S, 4.80. |
| Found: | C, 40.21; | H, 5.10; | N, 12.35; | F, 18.94; | S, 4.81. |

### Example 11

### [R-(R*,S*)]-2-Amino-1-[2-[[[4-(aminomethyl)phenyl]amino]methyl]-1-pyrrolidinyl]-3-phenyl-1-propanone, trifluoroacetate (1:3) salt

### A. [(4-Aminophenyl)methyl]carbamic acid, phenylmethyl ester

To a solution of 4-aminobenzenemethanamine (1.5 g, 12.3 mmol) in 20 mL CH₂Cl₂ was added benzylchloroformate (1.57 mL, 11 mmol), followed by triethylamine (Et₃N) (1.5 mL, 11 mmol) at room temperature. White percipitates was observed immediately. The reaction was stirred at room temperature for 10 minutes and removed Et₃N salt by filtration. The reaction mixture was concentrated in vacuo and partitioned between EtOAc and H₂O. The EtOAc layer was washed with a sat. solution of KHSO₄, a sat. solution of NaHCO₃, concentrated in vacuo and subjected to a silica-gel column using CHCl₃ / CH₃OH = 25 / 1 ratio as an eluting solvent to give title compound (43% yield).

### B. (S)-[[4-[[[1-[(1,1-Dimethylethoxy)carbonyl]-2-pyrrolidinyl]methyl]amino]phenyl]methyl]carbamic acid, phenylmethyl ester

To a solution of Example 16, Part A aldehyde (0.36 g, 1.79 mmol) and Part A compound (0.46 g, 1.79 mmol) in 3 mL CH₂Cl₂CH₂Cl₂ was slowly added acetic acid (AcOH) (140 µl, 2.33 mmol) at 0°C, followed by NaB(AcO)₃H. The reaction stirred at room temperature for 15 minutes and partitioned between EtOAc/hexane (1:1 ratio) and H₂O. The organic layer was washed with H₂O, brine, dried over Na₂SO₄ and concentrated in vacuo to give title compound (98% yield).

### C. (S)-[[4-[(2-Pyrrolidinylmethyl)amino]phenyl]methyl]carbamic acid, phenylmethyl ester, trifluoroacetate (1:1) salt

To a solution of Part B compound (0.8 g, 1.82 mmol) in 5 mL CH₂Cl₂ was added trifluoroacetic acid (TFA) (5.5 mL, 5.46 mmol) at 0°C. The ice bath was removed and stirred at room temperature for 1 hr. The reaction mixture was concentrated in vacuo and treated with a sat. HCl / Et₂O to give title comound (quant. yield).

### D. [R-(R*,S*)]-2-[[(1,1-Dimethylethoxy)carbonyl]amino]-3-phenyl-1-[2-[[[4-[[[(phenylmethoxy)amino]methyl]phenyl]amino]methyl]-1-pyrrolidinyl]-1-propanone

To a solution of Boc-D-Phenylalanine (0.55 g, 2.05 mmol) and HOBT (0.28 g, 2.05 mmol) in 4 mL DMF was added WSC (0.39 g, 2.05 mmol), followed by Part C compound (0.7 g, 1.87 mmol). NMM (226 ml, 2.05 mmol) was used to adjust the pH to 7.5-8.0. The reaction stirred at room temperature for 16 hrs and partitioned between EtOAc and H₂O. The EtOAc layer was further washed with a sat. solution of NaHCO₃, a sat. solution of KHSO₄, brine, dried over Na₂SO₄ and concentrated in vacuo to give title compound (41% yield).

### E. [R-(R*,S*)]-2-Amino-1-[2-[[[4-(aminomethyl)phenyl]amino]methyl]-1-pyrrolidinyl]-3-phenyl-1-propanone, trifluoroacetate (1:3) salt

To a solution of Part D compound (0.2 g, 0.34 mmol) in 1mL HOAc was added slowly 30% HBr in HOAc (1 mL, 1.12 mmol) at 0°C. The ice bath was removed and the reaction was stirred at room temperature for 1 hr. The reaction mixture was concentrated in vacuo and added Et₂O with stirred to formed white percipitates which was filtered to give title compound (59% yield).

| Elemental Analysis: C₂₁H₂₈N₄O · 3.10 TFA · 0.60 H₂O | | | | |
|---|---|---|---|---|
| Calc. : | C, 45.58; | H, 4.54; | N, 7.82; | F, 24.65 |
| Found: | C, 45.59; | H, 4.29; | N, 8.09; | F, 24.80. |

### Example 12

### [R-(R*,S*)]-N-[2-[2-[[[4-(Aminomethyl)phenyl]amino]methyl]-1-pyrrolidinyl]-2-oxo-1-(phenylmethyl)ethyl]-2-naphthalenesulfonamide, trifluoroacetate (2:3) salt

### A. [S(R*,S*)]-2-Amino-3-phenyl-1-[2-[[[4-[[[(phenylmethoxy)amino]methyl]phenyl]amino]methyl]-1-pyrrolidinyl]-1-propanone

To a solution of Example 1, Part D compound (0.2 g, 0.34 mmol) in 2 mL CH₂Cl₂ was added TFA (1 mL, 1.12 mmol) at 0°C. The ice bath was removed and the reaction was stirred at room temperature for 1.5 hrs. The reaction mixture was concentrated in vacuo to give title compound (97% yield).

### B. [R-(R*,S*)]-N-[2-[2-[[[4-[[[(Phenylmethyl)carbonyl]amino]methyl]phenyl]amino]methyl]-1-pyrrolidinyl]-2-oxo-1-(phenylmethyl)ethyl]-2-naphthalenesulfonamide

To a solution of Part C compound (0.17 g, 0.28 mmol) in 3 mL CH₂Cl₂ was added 2-naphthalene sulfonyl chloride (0.07 g, 0.31 mmol), followed by Et₃N (86 ml, 0.62 mmol). Additional Et₃N (20 ml, 0.14 mmol) was added after 1 hr. The reaction was stirred at room temperature for 1 more hr and partitioned between CH₂Cl₂ and H₂O. The CH₂Cl₂ layer was washed with a sat. solution of KHSO₄, a sat. solution of NaHCO₃, brine, dried over Na₂SO₄ and concentrated in vacuo to give title compound (83% yield).

### C. [R-(R*,S*)]-N-[2-[2-[[[4-(Aminomethyl)phenyl]amino]methyl]-1-pyrrolidinyl]-2-oxo-1-(phenylmethyl)ethyl]-2-naphthalenesulfonamide, trifluoroacetate (2:3) salt

To a solution of Part B compound (0.16 g, 0.24 mmol) in 1 mL HOAc was added 30% HBr in HOAc (0.8 mL, 0.71 mmol) at 0°C. The ice bath was removed and the reaction was stirred at room temperature for 4 hrs. The reaction mixture was concentrated in vacuo and subjected to a prep. HPLC using 35 to 90% B over 40 minutes gradient to give title compound (49% yield).

| Elemental Analysis Calcd for C₃₁H₃₄N₄O₃S · 1.60 TFA: | | | | | |
|---|---|---|---|---|---|
| | C, 56.65; | H, 4.95; | N, 7.73; | F, 12.58; | S, 4.42 |
| Found: | C, 56.66; | H, 4.93; | N, 7.79; | F, 12.56; | S, 4.20. |

[α]_{D}= -35.1 (c=0.51, MeOH).

### Example 13

### [R,(R*,S*)]-2-Amino-1-[2-[3-[(aminoiminomethyl)amino]propyl]-1-pyrrolidinyl]-2-phenyl-1-propanone, trifluoroacetate (1:3) salt

### A. (S)-2-[[[3-(1,3-Dihydro-1,3-dioxo-1H-isoindol-2-yl)propyl](trifluoroacetyl)amino]methyl]-1-pyrrolidinecarboxylic acid, 1,1-dimethylethyl ester

A solution of Example 3, Part A trifluoroacetamide (1.26g, 4 mmol) and N-(3-bromopropyl)phthalimide (1.7g, 6 mmol) in DMF (40mL) was treated with cesium carbonate (2.61 g, 8 mmol). The reaction was heated in an oil bath maintained at 55°C for 24 hours, cooled to room temperature and partitioned between ethyl acetate (75 mL) and water. The layers were separated. The organic layer was dried over magnesium sulfate and concentrated in vacuo. The crude material was purified by chromatography on silica gel (Merck, 400 mL), eluting with 10-30% ethyl acetate in hexane to give title compound (1.07 g, 53%) as a colorless oil.

### B. [R-(R*,S*)]-[2-[2-[[[3-(1,3-Dihydro-1,3-dioxo-2H-isoindol-2-yl)propyl](trifluoroacetyl)amino]methyl]-1-pyrrolidinyl]-2-oxo-1-(phenylmethyl)ethyl]carbamic acid, phenylmethyl ester

Part A BOC derivative (974 mg, 2.0 mmol) was dissolved in dichloromethane (2 mL) and TFA (4mL) and stirred at RT 2 hours. The TFA was removed by distillation under reduced pressure and by coevaporation with toluene to give the TFA salt of the amine as an oil. This and Z-D-phenylalanine (600 mg, 2 mmol) were dissolved in DMF (15 mL) at RT. HOBT (270 mg, 2 mmol), WSC (382 mg, 2 mmol) and NMM (0.6 mL, 6 mmol) were added. The reaction was stirred for 16 h, diluted with ethyl acetate (75 mL) and washed with 10% KHSO₄ (20 mL, 2 x) and saturated NaHCO₃ (20 mL, 2 x). The ethyl acetate extract was dried over magnesium sulfate and concentrated in vacuo to provide an oil. The crude material was purified by chromatography on silica gel. Elution with 20, 30, and 50% ethyl acetate in hexanes gave title compound, (1:06 g, 80%) as a white foam.

### C. [R-(R*,S*)]-[2-[2-[[(3-Aminopropyl)amino]methyl]-1-pyrrolidinyl]-2-oxo-1-(phenylmethyl)ethyl]carbamic acid, phenylmethyl ester

Part B phthalimide (1 g, 1.51 mmol) was dissolved in absolute ethanol (15 mL) and treated with methyl hydrazine (1.2 mL). The mixture was heated under reflux for 2 hours. The solvent was removed in vacuo and by co-evaporation with toluene (10 mL) and dichloromethane (10 mL x 2). The residue was dissolved in methanol (30 mL) and water (10 mL) and treated with potassium carbonate (1.5 g), The mixture was left stirring overnight at room temperature to complete the removal of the trifluoroacetamide group. 1N HCl solution (25 mL) was added to acidify the mixture and non-basic organics were removed by extracting with dichloromethane (25mL, x 3). The dichloromethane layers were extracted with 1NHCl (50 mL). The combined aqueous layers were basified with NaOH solution and the desired amine was extracted into dichloromethane (30 mL, x 3), dried over magnesium sulfate and concentrated in vacuo to provide title compound as an oil (615 mg, 93%).

### D. [R-(R*,S*)]-2-[2-[[[3-[(Aminoiminomethyl)amino]propyl]amino]methyl]-1-pyrrolidinyl]-2-oxo-1-(phenylmethyl)ethyl]carbamic acid, phenylmethyl ester, trifluoroacetate (1:3) salt

Part C compound (615 mg, 1.40 mmol) was dissolved in absolute ethanol (25 mL), to which amidinesulfonic acid (445 mg, 3.5 mmol) and triethylamine (560 µL, ∼4 mmol) were added and the mixture left stirring 16 hours at room temperature. The mixture was filtered through a pad of celite. The pad was washed with ethanol and the filtrate was concentrated to dryness. The crude material was purified by preparative HPLC (YMC S-10 ODS 50 x 500 mm column, eluting with 49 - 58% methanol in water, containing 0.1% TFA). Fractions containing title compound were combined and lyophilized to provide a white solid (620mg).

### E. [R-(R*,S*)]-2-Amino-1-[2-[3-[(aminoiminomethyl)amino]propyl]-1-pyrrolidinyl]-3-phenyl-1-propanone, trifluoroacetate (1:3) salt

The Part D TFA salt (∼620 mg, ) was dissolved in methanol (30 mL)and treated with Pearlman's catalyst (150 mg). The flask was equipped with a hydrogen filled balloon via a three-way stopcock. Air inside the flask was removed under reduced pressure and replaced with hydrogen from the balloon. This process was repeated three times. The mixture was stirred under the hydrogen atmosphere for 3 hours. The catalyst was removed by filtration through Celite and the filter pad was washed with additional methanol. The filtrate was freed of solvent in vacuo to give title compound which was dissolved in water (20 mL), passed through a millipore membrane and lyophilized to provide title compound as a white solid (507mg, 53% from Part C compound), Purity ≧ 98%.

| Analysis calcd for 2.90 TFA + 0.10 H₂O: | | | | |
|---|---|---|---|---|
| | C, 42.10; | H, 4.91; | N, 12.38; | F, 24.34. |
| Found: | C, 42.10 | H, 4.97;, | N, 12.28; | F, 24.41. |

### Example 14

### [S-(R*,S*)]-4-[(Aminoiminomethyl)amino]-N-[[1-(2-amino-1-oxo-3-phenylpropyl)-2-pyrrolidinyl]methyl]butanamide, trifluoroacetate (1:1) salt

### A. [R-(R*,S*)]-N-[[1-[2-[[(1,1-Dimethylethoxy)carbonyl]amino]-1-oxo-3-phenylpropyl]-2-pyrrolidinyl]methyl]-4-[[(phenylmethoxy)carbonyl]amino]butanamide

Example 6, Part B compound (694mg, 2.0 mmol) and Z-4-aminobutyric acid (478mg, 2.0 mmol) were dissolved in DMF (10 mL) at RT. HOBT (270mg, 2.0 mmol), WSC (382mg, 2.0 mmol) and NMM (500µL) were added. The reaction was stirred for 16 h, diluted with ethyl acetate (50 mL) and washed with 10% KHSO₄ (25 mL), saturated NaHCO₃ (25 mL) and saturated NaCl (30mL), dried over magnesium sulfate and concentrated in vacuo to provide title compound (1.04g, 92%) as a viscous oil which was used without further purification.

### B. [R-(R*,S*)]-4-Amino-N-[[1-[2-[[(1,1-dimethylethoxy)carbonyl]amino]-1-oxo-3-phenylpropyl]-2-pyrrolidinyl]methyl]butanamide

Part A compound (1.04g, 1.83 mmol) was dissolved in ethanol (100 mL) to which acetyl chloride (150µL, 2 mmol) had been added and the mixture was treated with 10% palladium on carbon (400 mg). The flask was equipped with a hydrogen filled balloon via a three way stopcock. Air inside the flask was evacuated under reduced pressure and the flask was then filled with hydrogen from the balloon. This process was repeated three times. The mixture was stirred in the hydrogen atmosphere for 20 hours. The catalyst was then removed by filtration and the pad was washed with more ethanol. The solvent was removed in vacuo to give title compound as a viscous oil (932mg, > 100%). The material was characterized by NMR and MS. The NMR indicated an appreciable amount of ethanol was trapped in the product.

### C. [R-(R*,S*)]-4-[(Aminoiminomethyl)amino]-N-[[1-[2-[[(1,1-dimethylethoxy)carbonyl]amino]-1-oxo-3-phenyl-propyl]-2-pyrrolidinyl]methyl]butanamide

Part B BOC derivative (1.83 mmol) was dissolved in ethanol (50 mL), to which amidinesulfonic acid (318 mg, 2.56 mmol) and triethylamine (690 µL, 4.94 mmol) were added. After 5 h the mixture was filtered through a pad of celite. The pad was washed with ethanol and the filtrate was concentrated to dryness to give the title BOC compound which was carried on without purification.

### D. [S-(R*,S*)]-4-[(Aminoiminomethyl)amino]-N-[[1-(2-amino-1-oxo-3-phenylpropyl)-2-pyrrolidinyl]methyl]butanamide, trifluoroacetate (1:1) salt

Part C BOC derivative ( 1.83 mmol) was dissolved in TFA (10 mL) and stirred at RT 90 min. The TFA was removed by distillation under reduced pressure and by coevaporation with toluene. The crude material was purified by preparative HPLC (YMC S-10 ODS 30 x 500 mm column, eluting with 38% methanol in water, containing 0.1% TFA). Fractions containing title compound were combined and lyophilized to provide a white solid (712mg, 61% from Part A compound), Purity ≧ 98%.
[α]_{D} = -55.7^{o} (c = 0.7, MeOH)

| Analysis calcd for 2.25 TFA + 0.10 H₂O: | | | | |
|---|---|---|---|---|
| | C, 44.60; | H, 5.17; | N, 13.28; | F, 20.26. |
| Found: | C, 44.59; | H, 5.43; | N, 13.17; | F, 20.40. |

### Example 15

### [S-(R*,S*)]-4-[(Aminoiminomethyl)amino]-N-[[1-(2-[(methylsulfonyl)amino]-1-oxo-3-phenylpropyl]-2-pyrrolidinyl]methyl]butanamide, trifluoroacetate (1:1) salt

Part D compound (158 mg, 0.25 mmol) was dissolved in a mixture of dichloromethane (2 mL)and dist. THF (4 mL). The solution was treated with triethyl amine (140 µL) followed by dropwise addition of methanesulfonyl chloride (24 µL, 0.3 mmol). The mixture was stirred at room temperature for 4 hours and then treated with water (∼ 0.35 mL). The mixture was taken to dryness in vacuo. The crude material was purified by preparative HPLC to provide a white solid which was used to take NMR spectra. The material was recovered, dissolved in water (15mL), passed through a millipore membrane and lyophilized to provide title compound as a white solid (126mg, 85%).

| Analysis calcd for 1.15 TFA + 0.60 H₂O: | | | | | |
|---|---|---|---|---|---|
| | C, 45.05; | H, 5.82; | N, 14.14; | F, 11.02; | S, 5.39. |
| Found: | C, 45.34 | H, 5.71;, | N, 13.75; | F, 11.03; | S, 5.50. |

### Example 16

### [S-(R*,S*)]-4-[[[1-(2-Amino-1-oxo-3-phenylpropyl)-2-pyrrolidinyl]methyl]amino]benzenecarboximidamide, trifluoroacetate (1:2) salt

### A. (S)-2-Formyl-1-pyrrolidinecarboxylic acid, 1,1-dimethylethyl ester

To a solution of oxalyl chloride (28mL, 56.2 mmol) in 14 mL dry CH₂Cl₂ cooled at -78°C (a dry ice acetone bath) was added 12 mL dry DMSO over 40 minutes. The reaction was stirred at -74°C for additional 40 minutes and (S)-2-(hydroxymethyl)-1-pyrrolidinecarboxylic acid, 1,1-dimethylethyl ester, (5.40 g, 26.86 mmol) in 14 mL CH₂Cl₂ was added dropwise at -65°C over 30 minutes. The reaction stirred additional 20 minutes at room temperature and partitioned between Et₂O and H₂O. The Et₂O layer was washed with more H₂O (50 mL x 4) and the H₂O layers was extracted with Et₂O (40 mL x 3). All the Et₂O layers was combined, washed with H₂O, brine, dried over Na₂SO₄ and concentrated in vacuo. The crude product was isolated by a silica-gel column using EtOAc and hexane (1:5 ratio) as an eluting solvent to give title compound (84% yield).

### B. (S)-2-[[(4-Cyanophenyl)amino]methyl]-1-pyrrolidinecarboxylic acid, 1,1-dimethylethyl ester

To a solution of Part A aldehyde, (2.0 g, 10.05 mmol) and 4-aminobenzonitrile, (1.3 g, 11.05 mmol) in dichloroethane (10 mL) was added acetic acid (844 µl, 14.07 mmol) dropwise at 0°C, followed by NaB(AcO)₃H (2.34 g, 11.05 mmol). The ice bath was removed and the reaction stirred at room temperature for 1 hr. The reaction mixture was diluted with ethylacetate and hexane (1:1 ratio) and washed with H₂O (20 mL), brine, dried over Na₂SO₄. The organic layer was concentrated in vacuo and isolated on a silica-gel column using CHCl₃ / EtOAc (50:1 ratio) as an eluting solvent to give title compound (83% yield).

### C. (S)-4-[(2-Pyrrolidinylmethyl)amino]benzenecarboximidamide

To a solution of Part B nitrile (1.36 g, 4.52 mmol) in dry EtOH was bubbled HCl gas at 0°C for 10 minutes. Sealed and stirred at room temperature for 72 hrs. The reaction was concentrated in vacuo and was redissolved in dry EtOH. NH₃ gas was bubbled in the reaction until it is basic and the reaction was stirred at room temperature for 24 hrs and concentrated in vacuo to give title compound (quant. yield).

### D. [S-(R*,S*)]-4-[[[1-[2-[[(1,1-Dimethylethoxy)carbonyl]amino]-1-oxo-3-phenylpropyl]-2-pyrrolidinyl]methyl]amino]benzenecarboximidamide

To a solution of L-Boc-phenylalanine (1.45 g, 5.50 mmol) and HOBT (743 mg, 5.50 mmol) in 26 mL DMF was added WSC (1.05 g, 5.50 mmol) and Part C compound (1.20 g, 5.50 mmol) . After 5 minutes, NMM (605 µl, 5.50 mmol) was added to adjust the pH to 7.5-8.0. The reaction was stirred at room temperature for 16 hrs and partitioned between EtOAc and H₂O. The EtOAc layer was further washed with a sat. solution of NaHCO₃, a sat. solution of KHSO₄, brine, dried over Na₂SO₄ and concentrated in vacuo. The crude product was subjected to a Prep. HPLC to obtain title compound (42% yield).

### E. [S-(R*,S*)]-4-[[[1-(2-Amino-1-oxo-3-phenylpropyl)-2-pyrrolidinyl]methyl]amino]benzenecarboximidamide, trifluoroacetate (1:2) salt

To a solution of Part D compound (390 mg, 0.84 mmol) in 5 mL CH₂Cl₂ was added TFA at 0°C. Removed the ice bath and the reaction stirred at room temperature for 2 hrs. The reaction mixture was concentrated in vacuo and subjected to a Prep. HPLC to give title compound (68% yield).

| Elemental analysis C₂₁H₂₇H₅O · 2.10 TFA · 0.80 H₂O | | | | |
|---|---|---|---|---|
| Calc: | C, 48.87; | H, 5.00; | N, 11.31; | F, 19.33 |
| Found: | C, 49.13; | H, 4.68; | N, 10.89; | F, 19.40. |

### Example 17

### [S-(R*,R*)]-4-[(Aminoiminomethyl)amino]-N-[[1-[3-hydroxy-2-[[(7-methoxy-2-napthalenyl)sulfonyl]amino]-1-oxopropyl]-2-pyrrolidinyl]methyl]butanamide, trifluoroacetamide (1:1) salt

### A. [S-(R*,R*]-1-[2-(Azidomethyl)-1-pyrrolidinyl]-2-[[(1,1-dimethylethoxy)carbonyl]amino]-3-(phenylmethoxy)-1-propanone

Example 1 Part A compound (2.13g, 9.4mmol) was dissolved in dichloromethane (5mL) and trifluoroacetic acid (7mL) and stirred at RT 3 hours. The TFA and dichloromethane were removed by distillation under reduced pressure and by coevaporation with toluene to give the amine as a TFA salt. This and N-BOC-O-benzyl-L-serine (2.95g, 10 mmol) were dissolved in DMF (50 mL). HOBT (1.35g, 10 mmol), WSC (1.91g, 10mmol) and NMM (3.3mL, 30 mmol) were added. The reaction was stirred for 8 h at room temperature, diluted with ethyl acetate (50 mL) and satd. KHSO₄ solution (30 mL). The layers were separated and the aqueous layer was reextracted with ethyl acetate (50 mL). The combined organic layers were washed with saturated sodium bicarbonate solution (30 mL)) and 10% lithium chloride solution (30 mL). The ethyl acetate layer was dried over magnesium sulfate and concentrated in vacuo to provide title compound (3.87g, 100%) as an oil which was used without further purification.

### B. [S-(R*,R*)]-2-Amino-1-[2-(azidomethyl)-1-pyrrolidinyl]-3-(phenylmethoxy)-1-propanone

Part A BOC-azide ( 604 mg, 1.5 mmol) was dissolved in dichloromethane (1 mL) and TFA (2 mL) and stirred at RT 3 hours. Solvents were removed by distillation under reduced pressure and by coevaporation with toluene to give title amine as the TFA salt which was used in the next step.

### C. [S-(R*,R*)]-N-[2-[2-(Azidomethyl)-1-pyrrolidinyl]-2-oxo-1-[(phenylmethoxy)methyl]ethyl]-7-methoxy-2-naphthalenesulfonamide

The crude Part B TFA salt (1.5 mmol) was dissolved in dichloromethane (5 mL), cooled in an ice bath, and 7-methoxynapthene-2-sulfonyl chloride (384mg, 1.5 mmol) was added, followed by dropwise addition of triethylamine (2 mL). The solution was stirred cold for 2 h before diluting with ethyl acetate (30 mL). This solution was washed with 1N HCl solution (10mL), dried over magnesium sulfate and evaporated to provide crude title sulfonamide. The crude product was chromatographed on silica gel and eluted with 20 and 30% EtOAc in hexane to provide title sulfonamide as a foam (536mg, 68% overall in two steps).

### D. [S-(R*,R*)-N-[2-[2-(Aminomethyl)-1-pyrrolidinyl]-2-oxo-1-[(phenylmethoxy)methyl]ethyl]-7-methoxy-2-naphthalenesulfonamide

The Part C sulfonamide (536 mg, 1 mmol) was dissolved in absolute ethanol (15mL), and treated with 10% palladium on carbon (105 mg). The flask was equipped with a hydrogen filled balloon via a three way stopcock. Air inside the flask was evacuated under reduced pressure and the flask was then filled with hydrogen from the balloon. This process was repeated three times. The mixture was stirred eight hours in the hydrogen atmosphere. The catalyst was then removed by filtration through a pad of MgSO₄ and the pad was washed with ethyl acetate. The solvent was removed in vacuo to give title compound as a light yellow oil (477 mg, 94%).

### E. [S-(R*,R*)]-N-[[1-[2-[[(7-Methoxy-2-naphthalenyl)sulfonyl]amino]-1-oxo-3-(phenylmethoxy)propyl]-2-pyrrolidinyl]methyl]-4-[[(phenylmethoxy)carbonyl]amino]butanamide

Part D compound (477mg, 0.96 mmol) and Z-4-aminobutyric acid (237mg, 1.0 mmol) were dissolved in DMF (10 mL) at RT. HOBT (135mg, 1.0 mmol), WSC (191mg, 1.0 mmol) and NMM (330µL) were added. The reaction was stirred for 5 h, diluted with satd KHSO₄ solution (15 mL) and water (15 mL). The product was extracted into ethyl acetate (2 x 20 mL) and washed with saturated NaHCO₃ (15 mL) and 10% LiCl solution (15mL), dried over magnesium sulfate and concentrated in vacuo. The crude yellow oil was chromatographed on silica gel, eluting with 50% EtOAc in hexane followed by 1-3% MeOH in EtOAc to provide title compound as a foam (500mg, 73%).

### F. [S-(R*,R*)]-4-Amino-N-[[1-[3-hydroxy-2-[[(7-methoxy-2-naphthalenyl)sulfonyl]amino]-1-oxopropyl]-2-pyrrolidinyl]methyl]butanamide

Part E compound (500 mg, 0.71 mmol) was dissolved in ethanol (60 mL) to which acetyl chloride (2.5mL) had been added and the mixture was treated with 10% palladium on carbon (200 mg) and hydrogenated at 55 psi for 24 h. The catalyst was removed by filtration and the residual catalyst was washed with EtOH. The filtrate was concentrated in vacuo to obtain title des-benzyl, des-CBZ product. This was used without purification.

### G. [S-(R*,R*)]-4-[(Aminoiminomethyl)amino]-N-[[1-[3-hydroxy-2-[[(7-methoxy-2-naphthalenyl)sulfonyl]amino]-1-oxopropyl]-2-pyrrolidinyl)methyl]butanamide, trifluoroacetamide (1:1) salt

The Part F material (∼0.71 mmol) was dissolved in ethanol (40 mL), to which amidinesulfonic acid (124 mg, 1.0 mmol) and triethylamine (300 µL, 2.1 mmol) were added. After 16 h the mixture was concentrated to dryness. The crude material was purified by preparative HPLC (YMC S-10 ODS 50 x 500 mm column, eluting with 50% methanol in water, containing 0.1% TFA). Fractions containing clean title compound were combined and lyophilized to provide a white solid (167 mg, 35%), Purity ≧ 98%.
[α]_{D} = -62.8^{o} (c = 0.5, MeOH)

| Analysis: Calcd for 1.15 TFA + 0.80 H₂O: | | | | | |
|---|---|---|---|---|---|
| | C, 46.44; | H, 5.45; | N, 12.36; | F, 9.64; | S, 4.71. |
| Found: | C, 46.50; | H, 5.34;, | N, 12.23; | F, 9.52; | S, 4.85. |

### Example 18

### [R-(R*,S*)]-N-[2-[2-[[[4-(Aminoiminomethyl)phenyl]amino]methyl]pyrrolidinyl]-2-oxo-1-(phenylmethyl)ethyl]methanesulfonamide, trifluoroacetate (1:1) salt

To a solution of Example 16 compound (155 mg, 0.25 mmol) in 7 mL dry CH₂Cl₂ and 3 mL THF was added Et₃N (139 ml, 1 mmol), followed by methanesulfonyl chloride (46 ml, 0.58 mmol). The reaction was stirred at room temperature for 2 hrs and concentrated in vacuo. The crude product was subjected to a Prep. HPLC using 40 to 90% B for 45 minutes gradient, A= 90% H₂O/0.1% TFA; B=90% CH₃OH/0.1% TFA; column: YMC S-10 ODS (c=18) to give title compound (69% yield).

| Elemental analysis C₂₂H₂₉N₅O₃S · 1.25 TFA · 0.60 H₂O | | | | |
|---|---|---|---|---|
| Calc: | C, 49.30; | H, 5.31; | N, 11.73; | F, 11.94 |
| Found: | C, 49.10; | H, 5.18; | N, 11.53; | F, 12.10. |

### Example 19

### [1(S),2S]-1-(Aminoiminomethyl)-N-[[1-[3-hydroxy-2-[(2-naphthalenylsulfonyl)amino]-1-oxopropyl]-2-pyrrolidinyl]methyl]-3-piperidinecarboxamide, trifluoroacetate (1:1) salt

### A. [S-(R*,R*)]-N-[2-[2-(Azidomethyl)-1-pyrrolidinyl]-2-oxo-1-[(phenylmethoxy)methyl]ethyl]-2-naphthalenesulfonamide

Example 17 Part A BOC-azide (3.85 mg, 9.55 mmol) was dissolved in trifluoroacetic acid (TFA) (20 ml) and stirred at RT 1.5 hours. The TFA was removed by distillation under reduced pressure and by coevaporation with toluene. The residue was dissolved in dichloromethane (100 mL), cooled in an ice bath, and triethylamine (4.0 mL, 28.7 mmol) and 2-naphthalene sulfonyl chloride (2.38g, 10.5 mmol) were added. The cooling bath was removed and the solution was stirred for 2 h. This solution was washed with KHSO₄ solution (25 mL x 2) and NaHCO₃ solution (25 mL x 2), dried over magnesium sulfate and evaporated to provide crude title compound. The crude product was chromatographed on silica gel to provide title sulfonamide as an oil (2.597 g, 55%).

### B. [S-(R*,R*)]-N-[2-[2-(Aminomethyl)-1-pyrrolidinyl]-2-oxo-1-[(phenylmethoxy)methyl]ethyl]-2-naphthalenesulfonamide

The Part A azide (2.5g, 5 mmol) was dissolved in absolute ethanol (100 mL) and treated with 10% palladium on carbon (300 mg). The flask was equipped with a hydrogen filled balloon via a three way stopcock. Air inside the flask was evacuated under reduced pressure and the flask was then filled with hydrogen from the balloon. This process was repeated three times. The mixture was stirred overnight in the hydrogen atmosphere. The catalyst was then removed by filtration through Celite and the pad was washed with ethanol. The solvent was removed from the filtrate in vacuo to give title amine as a foam (2.236 g, 96%).

### C. 1,3-Piperidinedicarboxylic acid, 1-(phenylmethyl)ester

Benzyl chloroformate (4 mL) was added dropwise to a cooled (0-5°C), stirred solution of piperidine-3-carboxylic acid (1.03 g, 8 mmol) in 1 N aqueous NaOH solution (25 mL). During the twenty minute addition, the pH was maintained between 8 and 9 by addition of 1 N NaOH solution. After addition was complete, the mixture was stirred cold for an additional hour, maintaining the pH thoughout this period. The reaction mixture was then extracted with ether (2 x 40 mL). The ether extracts were discarded and the aqueous layer was acidified with 1 N HCl solution. The acidified aqueous layer was then extracted with dichloromethane (2 x 40 mL). The combined extracts were dried over magnesium sulfate and concentrated in vacuo to give title acid as a viscous oil (1.73 g, 82%).

### D. 3-[[[[(S)-1-[(S)-2-[(Naphthalenylsulfonyl)amino]-1-oxo-3-(phenylmethoxy)propyl]-2-pyrrolidinyl]methyl]amino]carbonyl]-1-piperidinecarboxylic acid, phenylmethyl ester

Part B amine (820mg, 1.75 mmol, and Part C acid (526mg, 2.0 mmol) were dissolved in DMF (20 mL) at RT. HOBT (270mg, 2 mmol), 4-methyl morpholine (1.5 mL) and WSC (400mg, 2 mmol) were added. The reaction was stirred overnight at room temperature. The mixture was then diluted with ethyl acetate (50 mL), satd KHSO₄ solution (10 mL) and water (20 mL). The layers were separated and the aqueous layer was extracted with ethyl acetate (50 mL). The combined organic layers were washed with satd NaHCO₃ solution (20 mL) and 10% lithium chloride solution (2 x 20 mL), dried over magnesium sulfate and concentrated in vacuo The crude yellow foam was chromatographed on silica gel, eluting with 50% EtOAc in hexane followed by 75% EtOAc in hexane and finally with EtOAc to provide title sulfonamide as a foam (1.03 g, 82%).

### E. N-[[(S)-1-[(S)-3-Hydroxy-2-[(naphthalenylsulfonyl)amino]-1-oxopropyl]-2-pyrrolidinyl]methyl]-3-piperidinecarbamide

Part D compound (1.0 g, 1.4 mmol) was dissolved in ethanol (100 mL) to which acetyl chloride (2.0 mL) had been added and the mixture was treated with 10% palladium on carbon (300 mg) and hydrogenated at 55 psi for 44 h. The catalyst was removed by filtration and the pad was washed with EtOH. The filtrate was concentrated in vacuo to obtain crude amine hydrochloride salt.

### F. [1(S),2S]-1-(Aminoiminomethyl)-N-[[1-[3-hydroxy-2-[(2-naphthalenylsulfonyl)amino]-1-oxopropyl]-2-pyrrolidinyl]methyl]-3-piperidenecarboxamide, trifluoroacetate (1:1) salt

The crude Part E amine was dissolved in dimethylformamide (3 mL). H-pyrazole-1-carboxamidine (226 mg, 1.54 mmol) and diisopropylethyl amine (540 µL, 3.1 mmol) were added. The mixture was stirred over a weekend at room temperature. Ether (15 mL) was then added. Gummy material precipitated. The ether was decanted and the precipitate was washed with more ether. The gummy material was dissolved in methanol and taken to dryness in vacuo. The remaining material was purified by preparative HPLC (YMC S-10 ODS 50 x 500 mm column, eluting with 50% methanol in water, containing 0.1% TFA). Fractions containing clean title compound were combined and lyophilized to provide a white solid which was used to obtain NMR spectra, recovered, dissolved in water (20 mL) and relyophilized to give title compound (399 mg, 42%), Purity ≧ 98%.
[α]_{D} = -36.3° (c = 0.6, MeOH)

| Analysis: Calcd for 1.25 TFA + 0.1 H₂O: | | | | | |
|---|---|---|---|---|---|
| | C, 48.94; | H, 5.29; | N, 12.45; | F, 10.56; | S, 4.75. |
| Found: | C, 49.03; | H, 5.30;, | N, 12.27; | F, 10.47; | S, 4.66. |

### Example 20

### [S-(R*,R*)]-1-(Aminoiminomethyl)-N-[[1-[3-hydroxy-2-[(2-naphthalenylsulfonyl)amino]-1-oxopropyl]-2-pyrrolidinyl]methyl]-4-piperidinecarboxamide, trifluoroacetate (1:1) salt

### A. 1,4-Piperidinedicarboxylic acid, 1-(phenylmethyl) ester

Benzyl chloroformate (4 mL) was added dropwise to a cooled (0-5°C), stirred solution of piperidine-4-carboxylic acid (1.03 g, 8 mmol) in 1 N aqueous NaOH solution (25 mL). During the twenty minute addition, the pH was maintained between 8 and 9 by addition of 1 N NaOH solution. After addition was complete, the mixture was stirred cold for an additional hour, maintaining the pH thoughout this period. The reaction mixture was then extracted with ether (2 x 40 mL). The ether extracts were discarded and the aqueous layer was acidified with 1 N HCl solution. The acidified aqueous layer was then extracted with dichloromethane (2 x 40 mL). The combined extracts were dried over magnesium sulfate and concentrated in vacuo to give title acid as a viscous oil (2.0 g, 95%).

### B. [S-(R*,R*)-4-[[[[1-[2-[(Naphthalenylsulfonyl)amino]-1-oxo-3-(phenylmethoxy)propyl]-2-pyrrolidinyl]methyl]amino]carbonyl]-1-piperidinecarboxylic acid, phenylmethyl ester

Example 19, Part B amine (820mg, 1.75 mmol) and Part A acid (526mg, 2.0 mmol) were dissolved in DMF (20 mL) at RT. HOBT (270mg, 2 mmol), 4-methyl morpholine (1.5 mL) and WSC (400mg, 2 mmol) were added. The reaction was stirred overnight at room temperature. The mixture was then diluted with ethyl acetate (50 mL), satd KHSO₄ solution (10 mL) and water (20 mL). The layers were separated and the aqueous layer was extracted with ethyl acetate (50 mL). The combined organic layers were washed with satd NaHCO₃ solution (20 mL) and 10% lithium chloride solution (2 x 20 mL), dried over magnesium sulfate and concentrated in vacuo. The crude yellow foam was chromatographed on silica gel, eluting with 50% EtOAc in hexane followed by 75% EtOAc in hexane and finally with EtOAc to provide title sulfonamide as a foam (980 mg, 78%).

### C. [S-(R*,R*)]-N-[[1-[3-Hydroxy-2-[(naphthalenylsulfonyl)amino]-1-oxopropyl]-2-pyrrolidinyl]methyl]-4-piperidinecarbamide

Part B compound (970 mg, 1.36 mmol) was dissolved in ethanol (100 mL) to which acetyl chloride (2.0 mL) had been added and the mixture was treated with 10% palladium on carbon (400 mg) and hydrogenated at 55 psi for 30 h. The catalyst was removed by filtration and the pad was washed with EtOH. The filtrate was concentrated in vacuo to obtain crude title compound.

### D. [S-(R*,R*)]-1-(Aminoiminomethyl)-N-[[1-[3-hydroxy-2-[(2-naphthalenylsulfonyl)amino]-1-oxopropyl]-2-pyrrolidinyl]methyl]-4-piperidenecarboxamide, trifluoroacetate (1:1) salt

The crude Part C amine was dissolved in dimethylformamide (3 mL). H-pyrazole-1-carboxamidine (226 mg, 1.54 mmol) and diisopropylethyl amine (540 µL 3.1 mmol) were added. The mixture was stirred over a weekend at room temperature. Ether (15 mL) was then added. Gummy material precipitated. The ether was decanted and the precipitate was washed with more ether. The gummy material was dissolved in methanol and taken to dryness in vacuo. The remaining material was purified by preparative HPLC (YMC S-10 ODS 50 x 500 mm column, eluting with 50% methanol in water, containing 0.1% TFA). Fractions containing clean title compound were combined and lyophilized to provide a white solid (33269-139-26) which was used to obtain NMR spectra, recovered, dissolved in water (20 mL) and relyophilized to give title compound (264 mg, 29%), Purity ≧ 98%.
[α]_{D} = -45.4^{o} (c = 0.6, MeOH)

| Analysis: Calcd for 1.25 TFA + 0.1 H₂O: | | | | | |
|---|---|---|---|---|---|
| | C, 48.94; | H, 5.29; | N, 12.45; | F, 10.56; | S, 4.75. |
| Found: | C, 49.12; | H, 5.42;, | N, 12.45; | F, 10.62; | S, 4.82. |

### Example 21

### N-[(S)-2-[(S)-2-[[[[1-(Aminoiminomethyl)-3-piperidinyl]acetyl]amino]methyl]-1-pyrrolidinyl]-1-(hydroxymethyl)-2-oxoethyl]-2-naphthalenesulfonamide, trifluoroacetate (1:1) salt

### A. [S-(R*,R*)]-N-[2-[2-(Azidomethyl)-1-pyrrolidinyl]-2-oxo-1-[(phenylmethoxy)methyl]ethyl]-2-naphthalenesulfonamide

Example 17, Part A BOC-Azide (3.85 mg, 9.55 mmol) was dissolved in trifluoroacetic acid (TFA) (20 mL) and stirred at RT 1.5 hours. The TFA was removed by distillation under reduced pressure and by coevaporation with toluene. The residue was dissolved in dichloromethane (100 mL), cooled in an ice bath, and triethylamine (4.0 mL, 28.7 mmol) and 2-naphthalene sulfonyl chloride (2.38g, 10.5 mmol) were added. The cooling bath was removed and the solution was stirred for 2 h. This solution was washed with KHSO₄ solution (25mL x 2) and NaHCO₃ solution (25mL x 2), dried over magnesium sulfate and evaporated to provide crude title compound. The crude product was chromatographed on silica gel and eluted with 30 and 50% EtOAc in hexane to provide title sulfonamide as an oil (2.597 g, 55%).

### B. [S-(R*,R*)]-N-[2-[2-(Aminomethyl)-1-pyrrolidinyl]-2-oxo-1-[(phenylmethoxy)methyl]ethyl]-2-naphthalenesulfonamide

The Part A azide (2.5g, 5 mmol) was dissolved in absolute ethanol (100 mL) and treated with 10% palladium on carbon (300 mg). The flask was equipped with a hydrogen filled balloon via a three way stopcock. Air inside the flask was evacuated under reduced pressure and the flask was then filled with hydrogen from the balloon. This process was repeated three times. The mixture was stirred overnight in the hydrogen atmosphere. The catalyst was then removed by filtration through Celite and the pad was washed with ethanol. The solvent was removed from the filtrate in vacuo to give title amine as a foam (2.236 g, 96%).

### C. 1-[(Phenylmethoxy)carbonyl]-3-piperidineacetic acid

Platinum oxide (300 mg, Alfa) was added to a solution of 3-pyridyl acetic acid (3.3g, 24.08 mmol) in water (120 mL) and conc. HCl (2.5 mL). The solution was hydrogenated at up to 50 psi of H₂ pressure for 6.5 hrs. The catalyst was removed by filtration through a pad of Celite and the pad was washed with more water. The stirred aqueous solution of the 3-piperidine acetic acid was cooled (0-5°C) and treated with 5 N aqueous NaOH solution (20 mL). Benzyl chloroformate (4 mL) was added dropwise to the cooled, stirred solution. During the ten minute addition, the pH was maintained between 10 and 12 by addition of NaOH solution. After one hour the reaction mixture was extracted with ether (2 x 100 mL). The ether extracts were discarded and the aqueous layer was acidified with 2 N HCl solution. The acidified aqueous layer was then extracted with ethyl acetate (2 x 100 mL). The combined extracts were dried over magnesium sulfate and concentrated in vacuo to give title compound as an oil (7.0 g, contained EtOAc).

### D. N-[(S)-2-Oxo-2-[(S)-2-[[[[1-[(phenylmethoxy)carbonyl]-3-piperidinyl]acetyl]amino]methyl]-1-pyrrolidinyl]-1-[(phenylmethoxy)methyl]ethyl]-2-naphthalenesulfonamide

Part B amine (820mg, 1.75 mmol), and Part C acid (554mg, 2.0 mmol) were dissolved in DMF (20 mL) at RT. HOBT (270mg, 2 mmol), 4-methyl morpholine (1.5 mL) and WSC (400mg, 2 mmol) were added. The reaction was stirred 24 hours at room temperature. The mixture was then diluted with aqueous KHSO₄ solution and extracted with ethyl acetate (2 x 75 mL). The combined organic layers were washed with satd. NaHCO₃ solution (30 mL), dried over magnesium sulfate and concentrated in vacuo. The crude yellow foam was chromatographed on silica gel, eluting with 50% EtOAc in hexane, followed by 75% EtOAc in hexane, EtOAc and finally with 2% MeOH in EtOAc to provide title compound as a colorless oil (900mg, 71%).

### E. N-[(S)-1-(Hydroxymethyl)-2-oxo-2-[(S)-2-[[[[1-[(phenylmethoxy)carbonyl]-3-piperidinyl]acetyl]amino]methyl]-1-pyrrolidinyl]ethyl]-2-naphthalenesulfonamide, hydrochloride

Part D compound (900mg, 1.24 mmol) was dissolved in ethanol (100 mL) to which acetyl chloride (2.5 mL) had been added and the mixture was treated with 10% palladium on carbon (250 mg) and hydrogenated at 55 psi for 24 h. The catalyst was removed by filtration and the pad was washed with EtOH. The filtrate was concentrated in vacuo to obtain crude title compound which was used without purification.

### F. N-[(S)-2-[(S)-2-[[[[1-(Aminoiminomethyl)-3-piperidinyl]acetyl]amino]methyl]-1-pyrrolidinyl]-1-(hydroxymethyl)-2-oxoethyl]-2-naphthalenesulfonamide, trifluoroacetate (1:1) salt

The crude Part E amine was dissolved in dimethylformamide (3 mL). H-pyrazole-1-carboxamidine (226 mg, 1.54 mmol) and diisopropylethyl amine (536 µL, 3.1 mmol) were added. The mixture was stirred 3 days at room temperature. Ether (15 mL) was then added. Gummy material precipitated. The ether was decanted and the precipitate was washed with more ether. The gummy material was dissolved in methanol and taken to dryness in vacuo. The remaining material was purified by preparative HPLC (YMC S-10 ODS 50 x 500 mm column, eluting with 49% methanol in water, containing 0.1% TFA). Fractions containing clean title compound were combined and lyophilized to provide a white solid (283 mg, 33%), Purity ≧ 98%.
[α]_{D} = -39.2^{o} (c = 0.7, MeOH)

| Analysis: Calcd for 1.2 TFA + 0.4 H₂O: | | | | | |
|---|---|---|---|---|---|
| | C, 49.53; | H, 5.56; | N, 12.20; | F, 9.93; | S, 4.66 |
| Found: | C, 49.54; | H, 5.60; | N, 12.16; | F, 10.16; | S, 4.79 |

### Example 22

### [S-(R*,R*)]-N-[2-[2-[[[[1-(Aminoiminomethyl)-4-piperidinyl]acetyl]amino]methyl]-1-pyrrolidinyl]-1-(hydroxymethyl)-2-oxoethyl]-2-naphthalenesulfonamide, trifluoroacetate (1:1) salt

### A. 1-[(Phenylmethoxy)carbonyl]-4-piperidineacetic acid

Platinum oxide (300 mg, Alfa) was added to a solution of pyridyl-4-acetic acid, hydrochloride (3.3g, 19 mmol) in water (120 mL). The solution was hydrogenated at up to 50 psi of H₂ pressure for 17 hrs. The catalyst was removed by filtration through a pad of Celite and the pad was washed with more water. The stirred aqueous solution of the piperidine-4-acetic acid was cooled (0-5° C) and treated with 5 N aqueous NaOH solution (15 mL) to pH 12. Benzyl chloroformate (4 mL) was added dropwise to the vigorously stirred cooled solution. After one hour (maintaining the pH at 10 to 12), the reaction mixture was extracted with ether (2 x 100 mL). The ether extracts were discarded and the aqueous layer was acidified with 2 N HCl solution (50 mL). The acidified aqueous layer was then extracted with ethyl acetate (2 x 75 mL). The combined extracts were dried over magnesium sulfate and concentrated in vacuo to give title compound as a white solid (4.53 g, 86% yield).

### B. [S-(R*,R*)]-N-[2-Oxo-2-[2-[[[[1-[(phenylmethoxy)carbonyl]-4-piperidinyl]acetyl]amino]methyl]-1-pyrrolidinyl]-1-[(phenylmethoxy)methyl]ethyl]-2-naphthalenesulfonamide

Example 21 amine (820 mg, 1.75 mmol) and Part A acid (554mg, 2.0 mmol) were dissolved in DMF (20 mL) at RT. HOBT (270mg, 2 mmol), 4-methyl morpholine (1.5 mL) and WSC (400mg, 2 mmol) were added. The reaction was stirred 24 hours at room temperature. The mixture was then diluted with aqueous KHSO₄ solution (30 mL) and extracted with ethyl acetate (2 x 75 mL). The combined organic layers were washed with satd. NaHCO₃ solution (50 mL), dried over magnesium sulfate and concentrated in vacuo. The crude yellow foam was chromatographed on silica gel, eluting with 50% EtOAc in hexane, followed by 70% EtOAc in hexane, EtOAc and finally with 2% MeOH in EtOAc to provide title compound as a colorless oil (1.0 g, 79%).

### C. [S(R*,R*)]-N-[1-(Hydroxymethyl)-2-oxo-2-[2-[[[[1-[(phenylmethoxy)carbonyl]-4-piperidinyl]acetyl]amino]methyl]-1-pyrrolidinyl]ethyl]-2-naphthalenesulfonamide, hydrochloride

Part B compound (1.0 g, 1.377 mmol) was dissolved in ethanol (100 mL) to which acetyl chloride (2.5 mL) had been added and the mixture was treated with 10% palladium on carbon (250 mg) and hydrogenated at 55 psi for 48 h. The catalyst was removed by filtration and the pad was washed with EtOH. The filtrate was concentrated in vacuo to obtain crude title compound which was used without purification.

### D. [S-(R*,R*)]-N-[2-[2-[[[[1-(Aminoiminomethyl)-4-piperidinyl]acetyl]amino]methyl]-1-pyrrolidinyl]-1-(hydroxymethyl)-2-oxoethyl]-2-naphthalenesulfonamide, trifluoroacetate (1:1) salt

The crude Part C amine was dissolved in dimethylformamide (DMF) (3 mL). H-pyrazole-1-carboxamidine (226 mg, 1.54 mmol) and diisopropylethyl amine (536 µL, 3.1 mmol) were added. The mixture was stirred 4 days at room temperature, following the course of the reaction by TLC and HPLC during this time. Ether (15 mL) was then added. Gummy material precipitated. The ether was decanted and the precipitate was washed with more ether. The gummy material was dissolved in methanol and taken to dryness in vacuo. The remaining material was purified by preparative HPLC (YMC S-10 ODS 50 x 500 mm column, eluting with 47% methanol in water, containing 0.1% TFA). Fractions containing clean title compound were combined and lyophilized to provide a white solid. (344 mg, 40%), Purity ≧ 98%.
[α]_{D} = -41.5^{o} (c = 0.6, MeOH)

| Analysis: Calcd for 1.2 TFA +1.0 H₂O: | | | | | |
|---|---|---|---|---|---|
| | C, 48.76; | H, 5.65; | N, 12.01; | F, 9.78; | S, 4.58. |
| Found: | C, 48.76; | H, 5.58; | N, 12.06; | F, 9.63; | S, 4.73 |

### Example 23

### [S-(R*,R*)]-N-[2-[2-[[[5-[(Aminoiminomethyl)amino]-1-oxopentyl]amino]methyl]-1-pyrrolidinyl]-1-(hydroxymethyl)-2-oxoethyl]-2-naphthalenesulfonamide, trifluoroacetate (1:1) salt

Following the procedure of Examples 1 and 2 except substituting in Example 1, Part C N-CBZ-5-aminopentanoic acid for N-CBZ-4-aminobutyric acid, the title compound was obtained. The crude material was purified by preparative HPLC (YMC S-10 ODS 50 x 500 mm column, eluting with 51% methanol in water, containing 0.1% TFA). Fractions containing clean title compound were combined and lyophilized to provide a white solid (199 mg, 46%), Purity ≧ 98%.
[α]_{D} = -43.5^{o} (c = 0.6, MeOH)

| Analysis: Calcd for 1.0TFA + 0.70 H₂O: | | | | | |
|---|---|---|---|---|---|
| | C, 48.40; | H, 5.69; | N, 13.02; | F, 8.83; | S, 4.97 |
| Found: | C, 48.42; | H, 5.60; | N, 12.84; | F, 9.03; | S, 4.79. |

### Example 24

### [S-(R*,R*)]-N-[2-[2-[[[6-[(Aminoiminomethyl)amino]-1-oxohexyl]amino]methyl]-1-pyrrolidinyl]-1-(hydroxymethyl)-2-oxoethyl]-2-naphthalenesulfonamide, trifluoroacetate (1:1) salt

Following the procedure of Examples 1 and 2 except substituting N-CBZ-6-aminohexanoic acid for N-CBZ-4-aminobutyric acid, the title compund was prepared. The crude material was purified by preparative HPLC to provide a white solid (160 mg), Purity ≧ 98%. [α]_{D} = -44.3 ^{o} (c = 0.5, MeOH)

| Analysis: Calcd for 1.0TFA + 1.60 H₂O: | | | | | |
|---|---|---|---|---|---|
| | C, 48.01; | H, 6.00; | N, 12.44; | F, 8.44; | S, 4.75 |
| Found: | C, 48.33; | H, 5.70; | N, 11.92; | F, 8.18; | S, 4.71. |

### Example 25

### [1S[2R*(3R*)]]-N-[2-[2-[[[[1-(Aminoiminomethyl)-3-piperidinyl]carbonyl]amino]methyl]-1-pyrrolidinyl]-1-(hydroxymethyl)-2-oxoethyl]-2-naphthalenesulfonamide, trifluoroacetate (1:1) salt

### A. (S)-3-Piperidinecarboxylic acid, ethyl ester, D-tartrate salt

Ref: Johnston, G.A.R. et al, J Neurochemistry, 1976, Vol 26, pp.1029-1032.

Ethyl nipecotate (50g, 318mmol) and D-tartaric acid (47.74g, 318 mmol)were dissolved in hot abs. ethanol (250 mL). A very small amount of insoluble material was removed by filtration through a pad of Celite. The filtrate gave crystalline material on cooling. This was harvested and recrystallized eight times from absolute ethanol to give title compound as a white solid (∼27g). m.p. 155-156° C., [α]₃₆₅ = -200.7^{o} (c = 2.0, 0.2% aqueous ammonium molybdate).

### B. (S)-1-[(Phenylmethoxy)carbonyl]-3-piperidinecarboxylic acid

The Part A tartrate salt (1.0g) was dissolved in water (5 mL) and a solution of potassium carbonate was added to bring the pH to 9. The solution was cooled in an ice water bath and ether (5 mL) was added. Benzyl chloroformate (0.5 mL) was added dropwise to the well stirred solution. During the addition, the pH was maintained between 8 and 9 by addition of potassium carbonate solution. After addition was complete, the mixture was stirred cold for an additional 1.5 hours, maintaining the pH thoughout this period. The layers were separated and the aqueous was reextracted with ether. The combined ether layers were dried over magnesium sulfate and concentrated in vacuo. The ethyl ester obtained was purified on silica gel, eluting with 25% ethyl acetate in hexane. The purified material (930 mg) was dissolved in methanol (8 mL) and treated with 1 N NaOH solution (4 mL). After stirring at room temperature two hours, the methanol was removed in vacuo. The aqueous solution was acidified with 1 N HCl and the acid was extracted into ethyl acetate (2 x 10 mL). The combined extracts were dried over magnesium sulfate and concentrated in vacuo to give title compound as a crystalline solid. (773 mg). [α]_{D} = +49.9^{o} (c = 1.4, MeOH). The optical purity of this material was determined by coupling some of this material with (S)-(-)-α-methyl-benzylamine. The material obtained was submitted to the analytical HPLC group for determination of purity and was found to be 97.6 %, (e.e. = 95.2).

### C. [1S[2R*(3R*)]]-N-[2-[2-[[[[1-(Aminoiminomethyl)-3-piperidinyl]carbonyl]amino]methyl]-1-pyrrolidinyl]-1-(hydroxymethyl)-2-oxoethyl]-2-naphthalenesulfonamide, trifluoroacetate (1:1) salt

Following the procedure of Example 19, the title compound was prepared using the Example 19, Part B amine and the nonracemic Part B acid to give the crude product which was purified by preparative HPLC (360 mg, 46.4%), Purity ≧ 98%.
[α]_{D} = -15.1^{o} (c = 0.7, MeOH)

| Analysis: Calcd for 1.20 TFA + 0.4 H₂O: | | | | | |
|---|---|---|---|---|---|
| | C, 48.78; | H, 5.38; | N, 12.46; | F, 10.14; | S, 4.75. |
| Found: | C, 48.71; | H, 5.50;, | N, 12.43; | F, 10.12; | S, 4.78. |

### Example 26

### [1S[2R*(3S*)]]-N-[2-[2-[[[[1-(Aminoiminomethyl)-3-piperidinyl]carbonyl]amino]methyl]-1-pyrrolidinyl]-1-(hydroxymethyl)-2-oxoethyl]-2-naphthalenesulfonamide, trifluoroacetate (1:1) salt

### A. (R)-3-Piperidinecarboxylic acid, ethyl ester, L-tartrate salt

Ref: Johnston, G.A.R. et al, J Neurochemistry, 1976, Vol. 26, pp.1029-1032.

Ethyl nipecotate (50g, 318mmol) and L-tartaric acid (47.74g, 318 mmol)were dissolved in hot abs. ethanol (200 mL). Crystalline material was deposited on cooling. This was harvested and recrystallized 11 times from absolute ethanol to give title compound as a white solid (∼26g). m.p. 155-156° C., [α]₃₆₅ = +202.5^{o} (c = 2.0, 0.2% aqueous ammonium molybdate).

### B. (R)-1-[(Phenylmethoxy)carbonyl]-3-piperidinecarboxylic acid

The Part A tartrate salt (1.0g) was dissolved in water (5 mL) and a solution of potassium carbonate was added to bring the pH to 9. The solution was cooled in an ice water bath and ether (5 mL) was added. Benzyl chloroformate (0.5 mL) was added dropwise to the well stirred solution. During the addition, the pH was maintained between 8 and 9 by addition of potassium carbonate solution. After addition was complete, the mixture was stirred cold for an additional 1.5 hours, maintaining the pH throughout this period. The layers were separated and the aqueous was reextracted with ether. The combined ether layers were dried over magnesium sulfate and concentrated in vacuo. The ethyl ester obtained was purified on silica gel, eluting with 25% ethyl acetate in hexane. The purified material (971 mg) was dissolved in methanol (8 mL) and treated with 1 N NaOH solution (4 mL). After stirring at room temperature two hours, the methanol was removed in vacuo. The aqueous solution was acidified with 1 N HCl and the acid was extracted into ethyl acetate (2 x 10 mL). The combined extracts were dried over magnesium sulfate and concentrated in vacuo to give title compound as a crystalline solid. (881 mg). [α]_{D} = -49.6^{o} (c = 1.4, MeOH). The optical purity of this material was determined by coupling some of this material with (S)-(-)-α-methyl-benzylamine. The material obtained was submitted to the analytical HPLC group for determination of purity and was found to be 97.3 %, (e.e. = 94.6).

### C. [1S[2R*(3S*)]]-N-[2-[2-[[[[1-(Aminoiminomethyl)-3-piperidinyl]carbonyl]amino]methyl]-1-pyrrolidinyl]-1-(hydroxymethyl)-2-oxoethyl]-2-naphthalenesulfonamide, trifluoroacetate (1:1) salt

The title compound was prepared employing the procedure of Example 19 using Example 19, Part B amine and nonracemic Part B acid to give the crude product which was purified by preparative HPLC (133 mg, 32 %), Purity ≧ 98%.
[α]_{D} = -60.9^{o} (c = 0.6, MeOH)

| Analysis: Calcd for 1.05 TFA + 1.1 H₂O: | | | | | |
|---|---|---|---|---|---|
| | C, 48.57; | H, 5.60; | N, 12.54; | F, 8.93; | S, 4.78. |
| Found: | C, 48.52; | H, 5.44; | N, 12.31; | F, 8.82; | S, 4.82. |

### Example 27

### [S-(R*,R*)]-N-[2-[2-[[[7-[(Aminoiminomethyl)amino]-1-oxoheptyl]amino]methyl]-1-pyrrolidinyl]-1-(hydroxymethyl)-2-oxoethyl]-2-naphthalenesulfonamide, trifluoroacetate (1:1) salt

The title compound was prepared employing the procedure of Example 1 and 2 except that N-CBZ-7-aminoheptanoic acid was employed in place of N-CBZ-4-aminobutyric acid. The crude material was purified by preparative HPLC to give (276 mg).
Purity ≧ 98%.
[α]_{D} = -42.3 ^{o} (c = 0.6, MeOH)

| Analysis: Calcd for 1.05TFA + 0.60 H₂O: | | | | | |
|---|---|---|---|---|---|
| | C, 49.84; | H, 5.99; | N, 12.41; | F, 8.84; | S, 4.73. |
| Found: | C, 49.77; | H, 5.91; | N, 12.34; | F, 8.78; | S, 4.93. |

### Example 28

### [S-(R*,R*)]-4-(Aminomethyl)-N-[[1-[3-hydroxy-2-[(2-naphthalenylsulfonyl)amino]-1-oxopropyl]-2-pyrrolidinyl]methyl]benzamide, trifluoroacetate (1:) salt

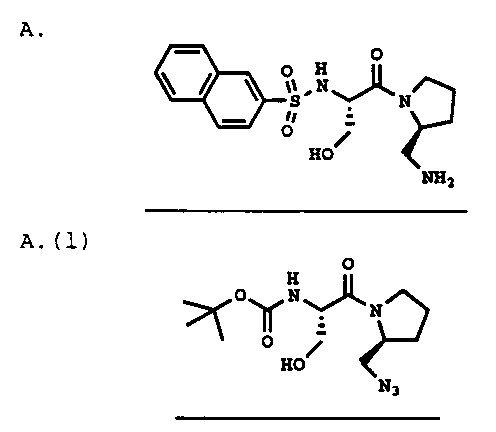

To a stirred solution of 1-BOC-2-(methyl-azide)pyrrolidine (12.54 g, 55.5 mmol) (prepared employing a procedure similar to that described in Example 1 Part A) in 10 mL of dry dichloromethane at 0°C was added a solution of 4N HCl in dioxane (25.0 mL, 100 mmol). The solution was stirred at room temperature for 2.5 h and concentrated in vacuo to give a crude amine as an oil. To a stirred solution of this amine, N-BOC-L-serine (11.4 g, 55.5 mmol) and 1-hydroxybenzotriazole monohydrate (9.37 g, 55.5 mmol) in 240 mL of DMF was added in order N-methylmorpholine (18.3 mL, 167 mmol) and ethyl 3-(3-dimethyl amino)propyl carbodiimide hydrochloride (10.6 g, 55.5 mmol). The reaction solution was stirred at room temperature for 19 h and concentrated under pump vacuum at 45°C. The residue was dissolved in 1 L of EtOAc and washed with 5%KHSO₄ solution (3x0.5 L), saturated NaHCO₃ solution (2x0.5 L) and brine (1x0.5 L). The EtOAc layer was dried (MgSO₄), filtered and concentrated in vacuo to give 14.4 g (83%) of title A(1) amide. 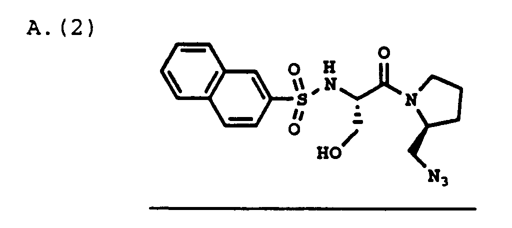
To a stirred solution of Part A(1) amide (14.3 g, 45.9 mmol) in 20 mL of dry dichloromethane at 0°C was added a solution of 4N HCl in dioxane (40.0 mL, 160 mmol). The solution was stirred at room temperature for 2 h and concentrated in vacuo to give a crude amine as an oil. To a stirred solution of this amine and triethylamine (15.4 mL, 110 mmol) in 100 mL of dry dichloromethane at 0°C was added dropwise a solution of 2-naphthalenesulfonyl chloride (10.9 g, 48.2 mmol) in 60 mL of dry dichloromethane over 40 min. The reaction was stirred at 0°C for 1 h and at room temperature for 2 h. The solution was diluted with 1 L of dichloromethane and washed with 1N HCl solution (3x0.5 L), saturated NaHCO₃ solution (2x0.5 L) and brine (1x0.5 L). The dichloromethane layer was dried (MgSO₄), filtered and concentrated in vacuo. This was triturated in EtOAc-hexane to give 11.5 g of title A(2) azide. The triturant was concentrated in vacuo and chromatographed on silica gel to give 12.9 g of title A(2) azide. 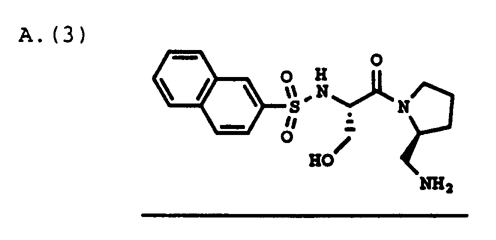
To a stirred solution of Part A(2) azide (11.2 g, 27.8 mmol) in 300 mL of EtOH and 600 mL of methanol was added 10%Pd/C (2.24 g). The atmosphere was replaced with hydrogen and the reaction mixture stirred at room temperature for 17 h. The catalyst was filtered off through a 4 µM polycarbonate film and rinsed with methanol (3x100 mL). The filtrate was concentrated in vacuo to give 9.9 g (94%) of crude title A amine. 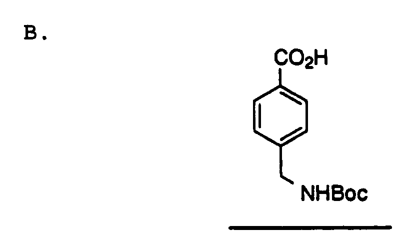
To a solution of 4-(aminomethyl)benzoic acid (10.33 g, 68.3 mmol) in ethanol (137 mL) and water (68 mL) were added aqueous NaOH (10 M, 7.6 mL, 76.0 mmol) and di-tert-butyl dicarbonate (16.48 g, 75.5 mmol). After 24 h, the reaction mixture was concentrated in vacuo. The residue was dissolved in EtOAc and 10% aqueous KHSO₄, the layers separated, and the aqueous layer was extracted with EtOAc. The organic layers were combined, dried over Na₂SO₄, filtered and concentrated in vacuo to give title compound (16.88 g, 98%) as a colorless solid. 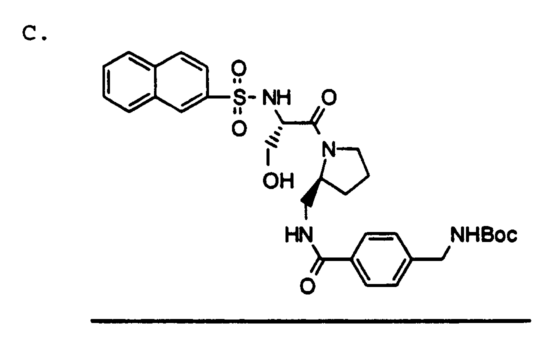
To a solution of Part B acid (0.23 g, 0.93 mmol) and 1-hydroxybenzotriazole hydrate (0.13 g, 0.94 mmol) in DMF (2.8 mL) at 0°C was added ethyl-3-(3-dimethylamino)propyl carbodiimide.HCl (0.17 g, 0.91 mmol). After 0.5 h, Part A amine (0.31 g, 0.83 mmol) in DMF (1.7 mL) was added, followed by 4-methylmorpholine (0.12 mL, 1.1 mmol). The reaction mixture was stirred for 15.5 h while allowing the reaction to warm to room temperature. The reaction mixture was poured into water:brine (1:1) and extracted with EtOAc, the organic layer washed with 0.25 M aqueous KHSO₄, water, saturated aqueous NaHCO₃, water and brine. The organic layer was dried over Na₂SO₄, filtered and concentrated in vacuo to give title compound (0.50 g, 98%) as a colorless foam.

### D. S-(R*,R*)]-4-(Aminomethyl)-N-[[1-[3-hydroxy-2-[(2-naphthalenylsulfonyl)amino]-1-oxopropyl]-2-pyrrolidinyl]methyl]benzamide, trifluoroacetate (1:1) salt

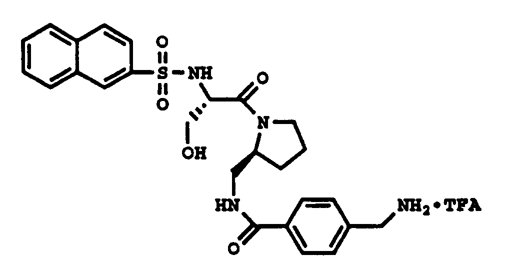

To a solution of Part C compound (0.44 g, 0.72 mmol) in CH₂Cl₂ at 0^{o} C was added trifluoroacetic acid (0.9 mL). After 1.5 h, the reaction mixture was concentrated in vacuo and purified by preparative HPLC. The appropriate fractions were combined, concentrated in vacuo, dissolved in H₂O and lyophilized to yield title compound (0.14 g, 32 %) as a colorless solid:
[α]_{D} = -38.6° (c 1.03, MeOH)
MS (M + H)⁺ = 511⁺

| Anal. Calc'd for C₂₆H₃₀N₄O₅S · 1.1TFA · 1.33 H₂0: | | | | | |
|---|---|---|---|---|---|
| | C, 51.32; | H, 5.16; | N, 8.49; | S, 4.86; | F, 9.50 |
| Found: | C, 51.32; | H, 4.92; | N, 8.42; | S, 5.03; | F, 9.82 |

### Example 29

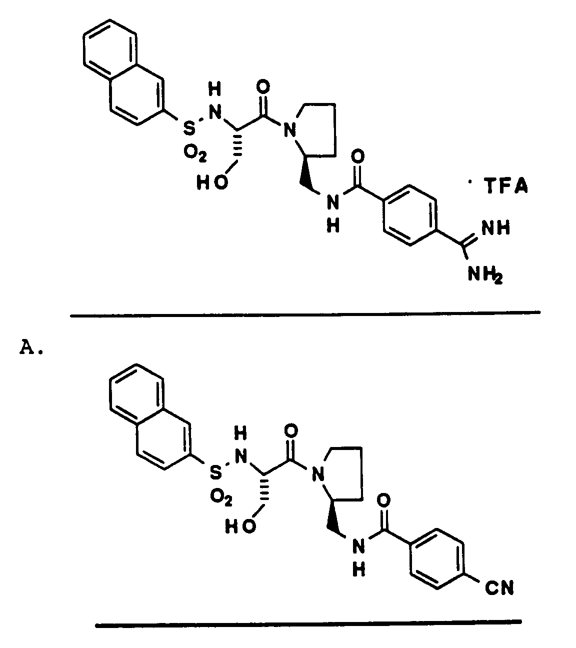

Example 28 Part A amine (1.93g, 5.15 mmol), 4-cyanobenzoic acid (757mg, 5.15 mmol) and 1-hydroxy-benzotriazole (869 mg, 6.44 mmol) were dissolved in DMF (30mL) at RT. 4-Methylmorpholine (1.1 mL, 10 mmol) was added dropwise followed by WSC (1.03g, 5.15 mmol). The reaction was stirred 20 hours at room temperature. The mixture was then diluted with ethyl acetate (75mL) and KHSO₄ solution (60 mL), the layers were separated and the aqueous layer was reextracted with ethyl acetate (75 ML). The combined organic layers was washed with saturated. NaHCO₃ solution (50mL) and 10% lithium chloride solution (2 x 25 mL), dried over magnesium sulfate and concentrated in vacuo. The crude oil was chromatographed on silica gel, eluting with 50% EtOAc in hexane followed by 75% EtOAc in hexane, EtOAc and finally 3 % MeOH in EtOAc to provide title compound as a foam (2.01g, 78%). 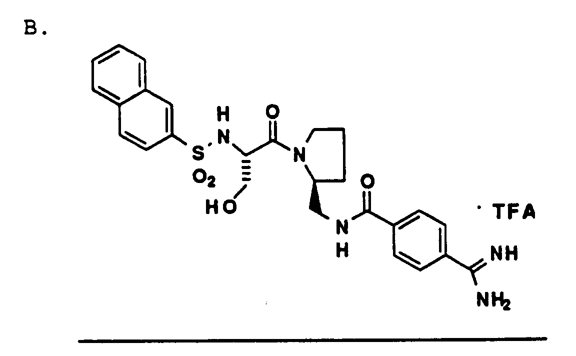
Sodium pellets (∼100 mg) were added to methanol (35 mL) and stirred until all the sodium had been consumed. Part A compound (759 mg, 1.5 mmol) was added and the mixture was stirred at room temperature. After three hours, additional sodium (∼50 mg) was added and the mixture was stirred an additional 2 hours. After this time an equilibrium mixture of product and starting material had been obtained. Ammonium chloride (4 g) was added and the mixture was left stirring overnight at room temperature. The reaction mixture was then acidified with 1 N HCl. Insoluble material was removed by filtration through Celite and the pad was washed with more methanol. The filtrate was freed of solvent in vacuo. The remaining material was purified by preparative HPLC (YMC S-15 ODS 50 x 500 mm column, eluting with 49% methanol in water, containing 0.1% TFA). Fractions containing clean title compound were combined and lyophilized to provide a white solid which was used to obtain NMR spectra, recovered, dissolved in water (40 mL) and relyophilized to give title compound (283 mg, 28%).
[α]_{D} = -37.6^{o} (c = 0.5, MeOH)

| Analysis: Calcd for 1.08 TFA + 2.1 H₂O: | | | | | |
|---|---|---|---|---|---|
| | C, 49.41; | H, 5.05; | N, 10.23; | F, 8.99; | S, 4.68 |
| Found: | C, 49.02; | H, 4.57; | N, 10.13; | F, 8.70; | S, 5.12. |

### Example 30

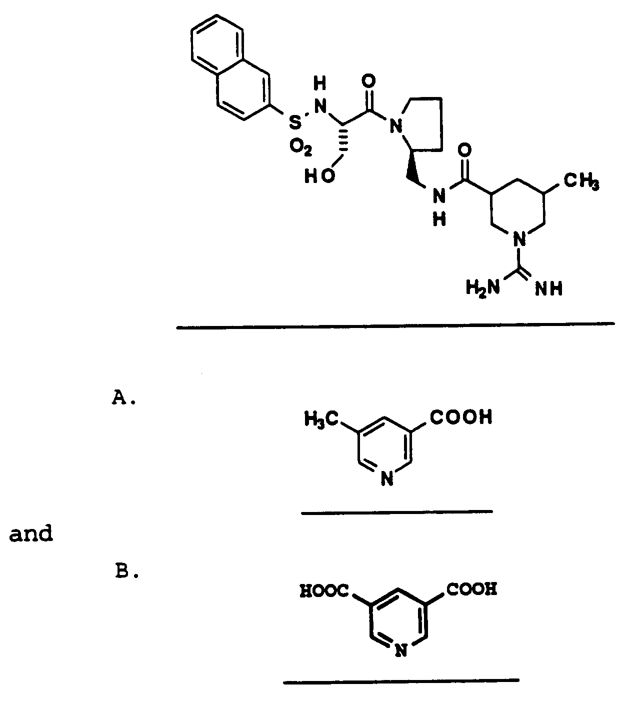

Ref: J Org. Chem, 53, 3513-3521, 1988.

Potassium permanganante (29.5g, 187mmol) was dissolved in water (450 mL) and 3,5-lutidine (10g, 93mmol) was added. After stirring for 1 hour the temperature was 40°C and the mixture was heated in an oil bath maintained at 40-50°C overnight while the stirring was continued. After cooling the mixture was filtered through Celite. The colorless filtrate was concentrated to ∼50 mL and then acidified with HCl. White solid precipitated and was harvested by filtration and washed with more water. Mass Spec indicated this was a mixture of the desired monoacid (A) and diacid (B) and was used in the next reaction. (5.2g). 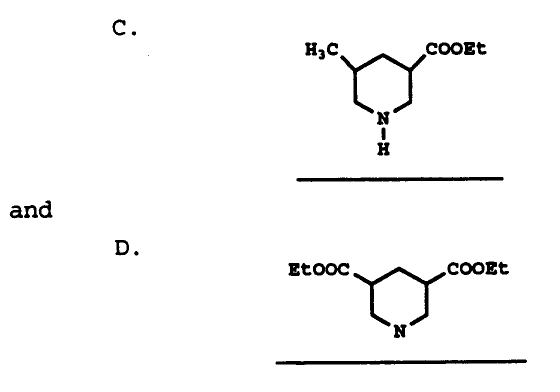
The mixture of Parts A and B acids was suspended in absolute ethanol (100 mL) in a Parr bottle. Acetyl chloride (5 mL) and platinum oxide (165 mg) were added and the mixture was hydrogenated at up to 55 psi for 20 hours. The catalyst was removed by filtration and the pad was washed with ethanol. The filtrate was taken to dryness in vacuo to give title compound. Mass spec indicated this was a mixture of the ethyl ester (D) and diester (D). 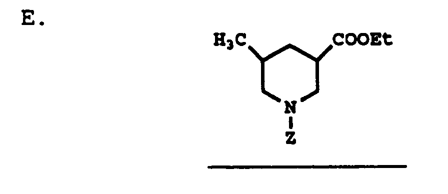
The mixture of Parts C and D ethyl esters was dissolved in water (50 mL) and a solution of potassium carbonate was added to bring the pH to 8.5. The solution was cooled in an ice water bath and ether (50 mL) was added. Benzyl chloroformate (8 mL) was added dropwise to the well stirred solution. During the addition, the pH was maintained between 8 and 9 by addition of potassium carbonate solution. After addition was complete, the mixture was stirred cold for an additional hour, maintaining the pH thoughout this period. The layers were separated and the aqueous was reextracted with ether. The combined ether layers were dried over magnesium sulfate and concentrated in vacuo. The material was chromatographed on silica gel, eluting with 10-20% ethyl acetate in hexane. The cis and trans isomers were separated. An NMR study of the acids indicated that the faster moving isomer (3.665 g) was the cis material. 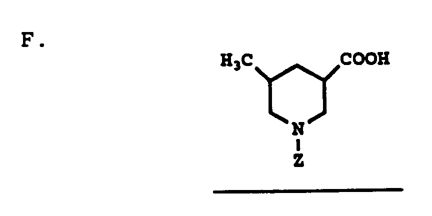
A portion of the purified Part E cis compound (1.525 g, 5 mmol)) was dissolved in methanol (16 mL) and treated with 1 N NaOH solution (10 mL). After stirring at room temperature two hours, the methanol was removed in vacuo. The aqueous solution was acidified with 1 N HCl and the acid was extracted into ethyl acetate (3 x 10 mL). The combined extracts were dried over magnesium sulfate and concentrated in vacuo to give title compound as a viscous oil (1.39 g, 100%). 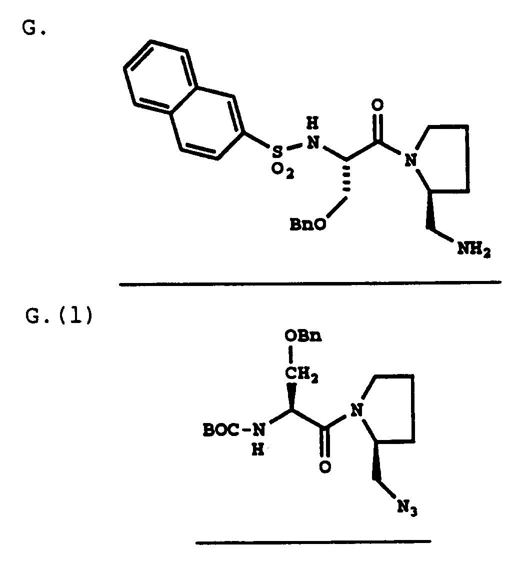
2-Azido-N-BOC pyrrolidine (2.13g, 9.4 mmol) was dissolved in dichloromethane (5 mL) and trifluoroacetic acid (7 mL) and stirred at RT 3 hours. The TFA and dichloromethane were removed by distillation under reduced pressure and by coevaporation with toluene to give the amine as a TFA salt. This and N-BOC-O-benzyl-L-serine (2.95g, 10 mmol) were dissolved in DMF (50 mL). HOBT (1.35g, 10 mmol), WSC (1.91g, 10 mmol) and NMM (3.3 mL, 30 mmol) were added. The reaction was stirred for 8 h at room temperature, diluted with ethyl acetate (50 mL) and saturated KHSO₄ solution (30 mL). The layers were separated and the aqueous layer was reextracted with ethyl acetate (50 mL). The combined organic layers were washed with saturated sodium bicarbonate solution (30 mL) and 10% lithium chloride solution (30 mL). The ethyl acetate layer was dried over magnesium sulfate and concentrated in vacuo to provide title G(1) compound (3.87g, 100%) as an oil which was used without further purification. 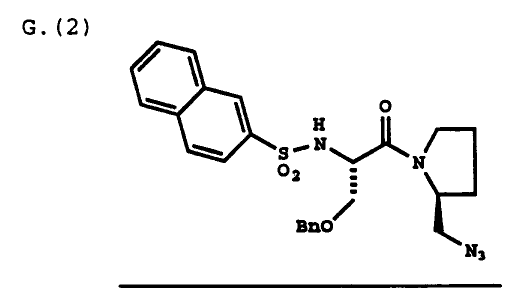
Part G(1) BOC-azide (3.85 mg, 9.55 mmol) was dissolved in TFA (20 mL) and stirred at RT 1.5 hours. The TFA was removed by distillation under reduced pressure and by coevaporation with toluene. The residue was dissolved in dichloromethane (100 mL), cooled in an ice bath, and triethylamine (4.0 mL, 28.7 mmol) and 2-naphthalene sulfonyl chloride (2.38g, 10.5 mmol) were added. The cooling bath was removed and the solution was stirred for 2 h. This solution was washed with KHSO₄ solution (25 mL x 2) and NaHCO₃ solution (25 mL x 2), dried over magnesium sulfate and evaporated to provide crude title sulfonamide. The crude title sulfonamide product was chromatographed on silica gel and eluted with 30% and 50% EtOAc in hexane to provide A(1) sulfonamide as an oil (2.597g, 55%). 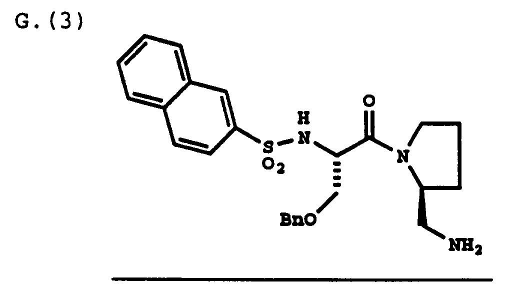
The Part G(2) azide (2.5g, 5 mmol) was dissolved in absolute ethanol (100 mL) and treated with 10% palladium on carbon (300 mg). The flask was equipped with a hydrogen filled balloon via a three way stopcock. Air inside the flask was evacuated under reduced pressure and the flask was then filled with hydrogen from the balloon. This process was repeated three times. The mixture was stirred overnight in the hydrogen atmosphere. The catalyst was then removed by filtration through Celite and the pad was washed with ethanol. The solvent was removed from the filtrate in vacuo to give title amine as a foam (2.236g, 96%). 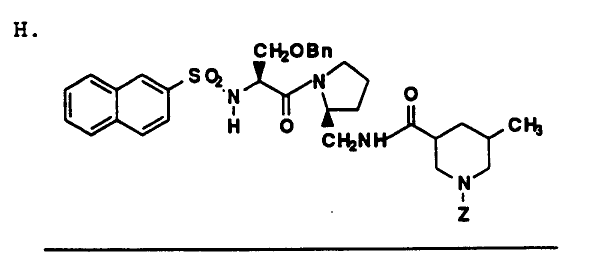
Part G amine (467mg, 1.0 mmol) and Part F acid (305mg, 1.1 mmol) were dissolved in DMF (2 mL) at RT. HOBT (148mg, 1.1 mmol), 4-methyl morpholine (242 µL, 2.2 mmol) and WSC (210mg, 1.1 mmol) were added. The reaction was stirred 20 hours at room temperature. The mixture was then diluted with ethyl acetate (50mL) and KHSO₄ solution (20 mL). The layers were separated. The organic layer was washed with saturated NaHCO₃ solution (20 mL) and 10% lithium chloride solution (2 x 20 mL), dried over magnesium sulfate and concentrated in vacuo. The crude oil was chromatographed on silica gel, eluting with 50% EtOAc in hexane followed by EtOAc to provide title compound (584 mg, 80%). 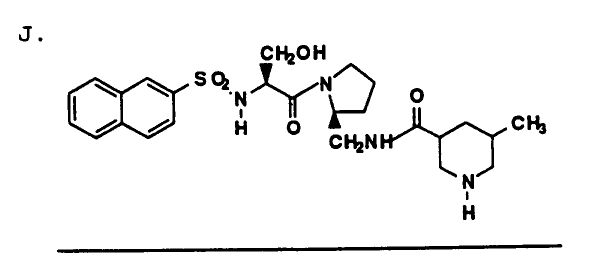
Part H compound (578 mg, 0.79 mmol) was dissolved in ethanol (100 mL) to which acetyl chloride (2.0 mL) had been added and the mixture was treated with 10% palladium on carbon (300 mg) and hydrogenated at 55 psi for 3 days. The catalyst was removed by filtration and the pad was washed with EtOH. The filtrate was concentrated in vacuo to obtain crude title compound as a foam (350 mg) which was used without purification. 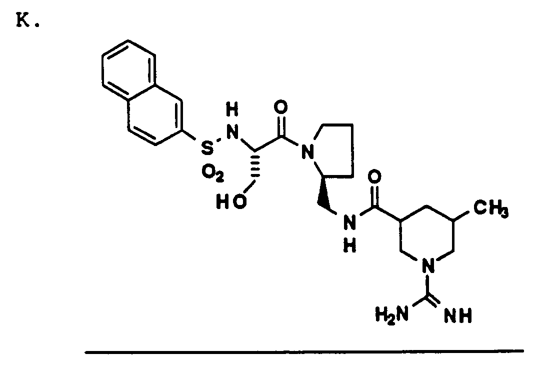
The crude Part J amine (∼0.79 mmol) was dissolved in dimethylformamide (1.6 mL). H-pyrazole-1-carboxamidine (131 mg, 0.89 mmol) and diisopropylethyl amine (310 µL) were added. The mixture was stirred 24 hours at room temperature. Ether (10 mL) was then added. Gummy material precipitated. The ether was decanted and the precipitate was washed with more ether. The gummy material was dissolved in methanol and taken to dryness in vacuo. The remaining material was purified by preparative HPLC (YMC S-15 ODS 50 x 500 mm column, eluting with 54 % methanol in water, containing 0.1% TFA). The two cis isomers were separated. The fractions that were clean by analytical HPLC were combined, concentrated to a small volume in vacuo and lyophilized. These samples were used to obtain NMR spectra, recovered, dissolved in water and relyophilized to give title compound, Isomer A (from Part H compound) and Example 48 compound, Isomer B (124 mg, 22% from Part H compound). Also obtained was a small amount of mixed fractions (54 mg, ∼10%). Title compound, Isomer A: [α]_{D} = -49.0° (c = 0.4, MeOH)

| Analysis: Calcd for 1.15 TFA + 1.3 H₂O: | | | | | |
|---|---|---|---|---|---|
| | C, 48.61; | H, 5.73; | N, 12.02; | F, 9.37; | S, 4.59. |
| Found: | C, 48.59; | H, 5.62; | N, 11.92; | F, 9.35; | S, 4.74. |

### Example 31

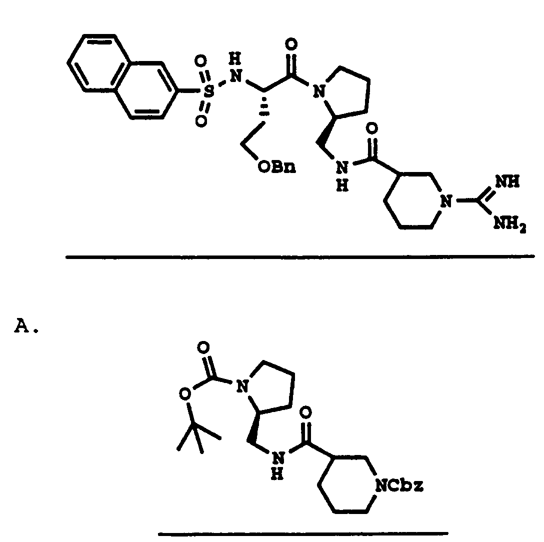

To a stirred solution of 1-BOC-2-(methyl-azide)pyrrolidine (3.00 g, 13.3 mmol) in 80 mL of methanol under argon was added 20%Pd(OH)₂/C (0.60 g, 20% based on the weight of the azide compound). The atmosphere was replaced with hydrogen by several vacuum-fill cycles. The reaction mixture was stirred at room temperature for 19 h. The catalyst was filtered off through a 4 µM polycarbonate film and rinsed with methanol (4x30 mL). The filtrate was concentrated in vacuo to give 2.65 g of an intermediate amine in a quantitative yield. To a stirred solution of this amine, 1-hydroxybenzotriazole monohydrate (2.62 g, 15.5 mmol) and N-carbobenzyloxy-3-piperidine carboxylic acid (4.08 g, 15.5 mmol) in 85 mL of DMF was added in order N-methylmorpholine (8.51 mL, 77.5 mmol) and ethyl 3-(3-dimethylamino)propyl carbodiimide hydrochloride (5.94 g, 31.0 mmol). The reaction solution was stirred at room temperature for 18 h and concentrated under pump vacuum at 50°C. The residue was dissolved in 500 mL of EtOAc and washed with 1N HCl solution (3x200 mL), saturated NaHCO₃ solution (2x200 mL) and brine (1x200 mL). The EtOAc layer was dried (MgSO₄), filtered and concentrated in vacuo. Purification was effected by flash chromatography on silica gel to give 3.33 g (56%) of title carbamate. 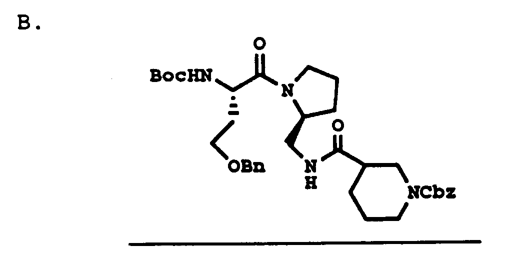
To a stirred solution of Part A carbamate (3.20 g, 7.19 mmol) in 10 mL of dry dichloromethane at 0°C was added a 4N HCl solution in dioxane (15.0 mL, 60.0 mmol). The solution was stirred at room temperature for 3 h and concentrated in vacuo. The residue was dissolved in 100 mL of dichloromethane and 100 mL of methanol. The solution was concentrated in vacuo to give an intermediate amine. To a stirred solution of this amine (1.10 g, 2.88 mmol), 1-hydroxybenzotriazole monohydrate (0.48 g, 2.88 mmol) and N-Boc-O-benzylhomoserine (0.89 g, 2.88 mmol) in 20 mL of DMF was added in order N-methylmorpholine (1.04 mL, 9.51 mmol) and ethyl 3-(3-dimethylamino)propyl carbodiimide hydrochloride (0.55 g, 2.88 mmol). The reaction solution was stirred at room temperature for 18 h and concentrated under pump vacuum at 45°C. The residue was dissolved in 300 mL of EtOAc and washed with 1N HCl solution (3x100 mL), saturated NaHCO₃ solution (2x100 mL) and brine (1x100 mL). The organic layer was dried (MgSO₄), filtered and concentrated in vacuo and chromatographed on silica gel to give 1.28 g (70%) of title amide. 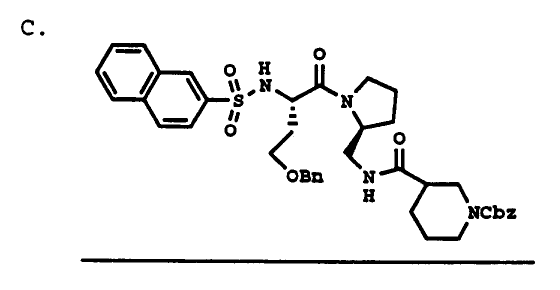
To a stirred solution of Part B amide (804 mg, 1.27 mmol) in 5.0 mL of dry dichloromethane at 0°C was added a 4N HCl solution in dioxane (10.0 mL, 40.0 mmol). The reaction solution was stirred at room temperature for 3 h and concentrated in vacuo to give a crude amine. To a stirred solution of this amine and triethyl amine (0.39 mL, 2.78 mmol) in 20 mL of dry dichloromethane under argon was added 2-naphthalenesulfonyl chloride (301 mg, 1.33 mmol). The reaction solution was stirred at room temperature for 3.5 h and diluted with 200 mL of EtOAc. The solution was washed with 1N HCl solution (3x60 mL) saturated NaHCO₃ solution (2x60 mL) and brine (1x60 mL). The organic layer was dried (MgSO₄), filtered, concentrated in vacuo and was chromatographed on silica gel to give 0.79 g (86%) of title carbamate. 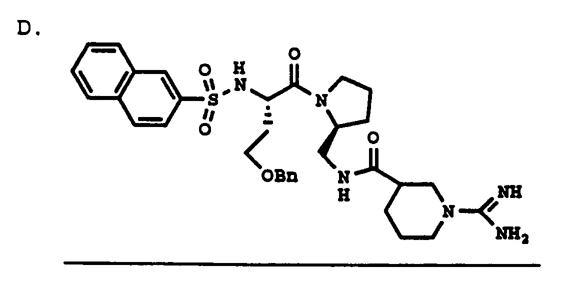
To a stirred solution of Part C carbamate (0.71 g, 0.98 mmol) in 10 mL of methanol under argon was added 20%Pd(OH)₂/C (142 mg, 20% based on the weight of Part C compound). The atmosphere was replaced with hydrogen by several vacuum-fill cycles. The reaction mixture was stirred at room temperature for 20 h. The catalyst was filtered off through a 4 µM polycarbonate film and rinsed with methanol (5x30 mL). The filtrate was concentrated in vacuo to give 0.58 g of the intermediate amine. To a stirred solution of this amine (124 mg, 0.21 mmol) and DIEA (73.0 mL, 0.42 mmol) in 0.25 mL of DMF was added guanopyrazole hydrochloride (37.0 mg, 0.25 mmol). The reaction solution was stirred at room temperature for 24 h at which time another batch of guanopyrazole hydrochloride (6.20 mg, 0.04 mmol) was added. The solution was stirred at room temperature for 22 h. The reaction solution was then diluted with 20 mL of ether and treated with 0.15 mL of a solution of 4N HCl in dioxane. The solvent was decanted and the precipitate was purified by preparative HPLC. The fractions were concentrated in vacuo and lyophilized to give 118 mg (73%) of title guanidine.

| Analysis: calc'd for 1.00 TFA + 1.00 H₂O: | | | | | |
|---|---|---|---|---|---|
| | C, 54.82; | H, 5.91; | N, 10.96; | F, 7.43; | S, 4.18 |
| Found: | C, 54.87; | H, 5.83; | N, 10.58; | F, 7.82; | S, 4.25. |

### Example 32

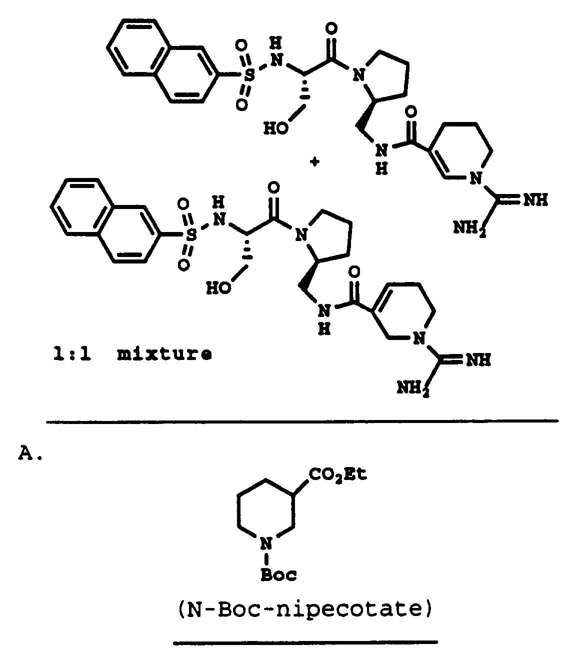

To a stirred solution of (±) ethyl nipecotate (5.00 g, 31.8 mmol) and triethyl amine (6.64 mL, 47.7 mmol) in 100 mL of dichloromethane at 0°C was added portionwise a solution of di-t-butyl dicarbonate (7.64 g, 35.0 mmol) in 100 mL of dichloromethane over 5 min. The reaction solution was stirred at room temperature for 25 h and then diluted with 600 mL of EtOAc. The solution was washed with 1N HCl solution (3x200 mL), saturated NaHCO₃ solution (2x200 mL) and brine (1x200 mL). The organic layer was dried (MgSO₄), filtered and concentrated in vacuo. This was chromatographed on silica gel to give 6.44 g (79%) of title N-Boc nipecotate. 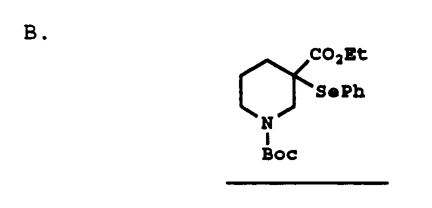
To a stirred solution of Part A nipecotate (1.00 g, 3.91 mmol) in 40 mL of dry THF under argon at -78°C was added a 1M solution of sodium hexamethyldisilazide (4.30 mL, 4.30 mmol) over 5 min. The solution was stirred at -78°C for 40 min at which time a solution of diphenyl diselenide (1.28 g, 4.10 mmol) in 6.0 mL of THF was added dropwise over 10 min. This reaction solution was stirred at -78°C for 1 h and quenched dropwise with 15 mL of saturated NaHCO₃ solution. The resulting mixture was stirred vigorously without cooling for 5 min. The mixture was concentrated in vacuo. The residue was diluted with 300 mL of EtOAc and washed with 5% KHSO₄ solution (3x150 mL), saturated NaHCO₃ solution (2x150 mL) and brine (1x150 mL). The organic layer was dried (MgSO₄), filtered, concentrated in vacuo; and purified on silica gel to give 1.29 g (80%) of Part B ester. 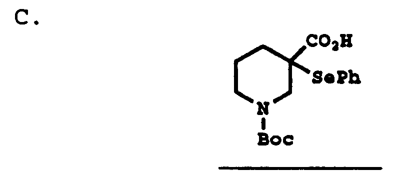
To a stirred solution of Part B ester (1.06 g, 2.57 mmol) in 65 mL of methanol and 20 mL of water was added 1N NaOH solution (7.69 mL, 7.69 mmol). The reaction solution was stirred at room temperature for 10 days. The solution was concentrated in vacuo. The remaining aqueous solution was acidified to pH 2 by the addition of 1N HCl solution. The solution was extracted with EtOAc (4x60 mL). The combined EtOAc extracts were dried (MgSO₄), filtered, concentrated in vacuo, and chromatographed on silica gel to give 350 mg (36%) of title acid and some recovered starting Part B ester. 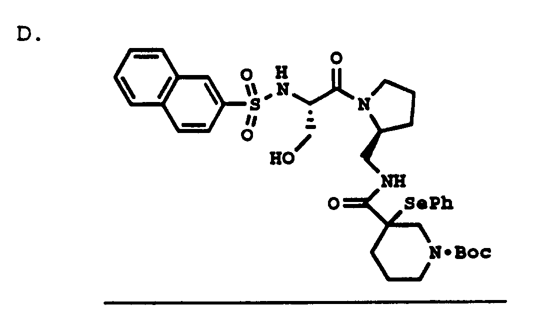
To a stirred solution of Part C acid (263 mg, 0.68 mmol), Example 30 Part G amine (283 mg, 0.68 mmol) and 1-hydroxybenzotriazole monohydrate (115 mg, 0.68 mmol) in 4.4 mL of DMF was added in order N-methylmorpholine (0.23 mL, 2.05 mmol) and ethyl 3-(3-dimethylamino)propyl carbodiimine hydrochloride (131 mg, 0.68 mmol). The reaction solution was stirred at room temperature for 16 h and concentrated under pump vacuum at 45°C. The residue was dissolved in 200 mL of EtOAc and washed with 1N HCl solution (2x60 mL), saturated NaHCO₃ solution (2x60 mL) and brine (1x60 mL). The EtOAc layer was dried (MgSO₄), filtered and concentrated in vacuo, and was chromatographed on silica gel to give 417 mg (82%) of title carbamate. 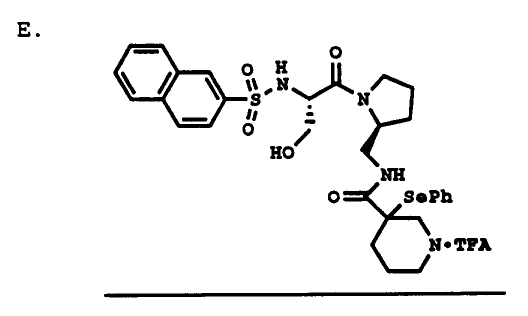
To a stirred solution of Part D carbamate (415 mg, 0.56 mmol) in 3.0 mL of dry dichloromethane was added TFA (9.00 mL, 117 mmol). The reaction solution was stirred at room temperature for 1 h and concentrated in vacuo to give title amine · TFA (384 mg, 91%). 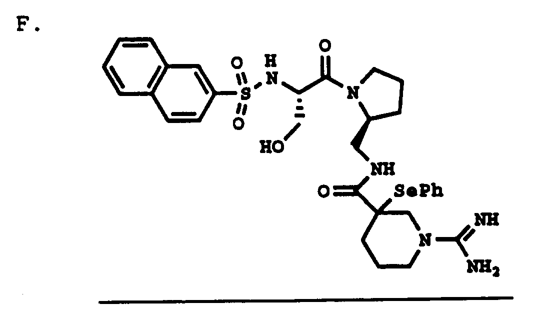
To a stirred solution of Part E amine · TFA (381 mg, 0.50 mmol) and DIEA (0.26 mL, 1.51 mmol) in 0.5 mL of DMF was added guanopyrazole hydrochloride (88.0 mg, 0.60 mmol). The reaction solution was stirred at room temperature for 24 h at which time another of batch of guanopyrazole hydrochloride (14.7 mg, 0.10 mmol) was added. The reaction solution was stirred at room temperature for 24 h and stood at room temperature for 21 h. The solution was diluted with 20 mL of ether. To this mixture was added 0.3 mL of a solution of 4N HCl in dioxane. The solution was decanted and the precipitated residue was purified by preparative HPLC. The fractions were concentrated in vacuo and lyophilized to give 236 mg (59%) of title selenide. 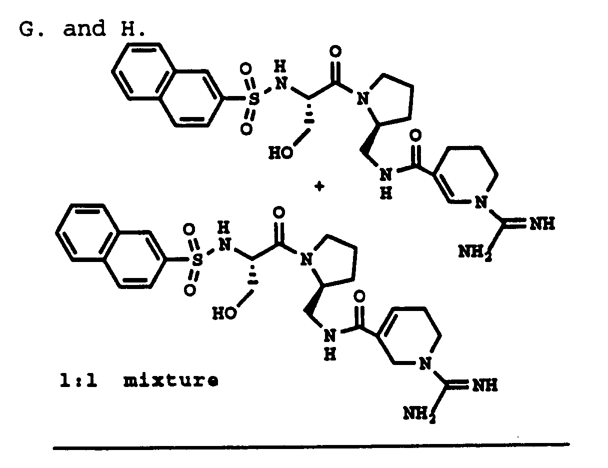
To a stirred solution of Part F selenide (190 mg, 0.24 mmol) in 9 mL of THF was added 1.6 mL of 30% H₂O₂ solution. The reaction solution was stirred at room temperature for 30 min, concentrated in vacuo and purified by preparative HPLC to give 95 mg (62%) of a 1:1 mixture of title G and H compounds.
MS: (M+H)⁺ = 529.

### Example 33

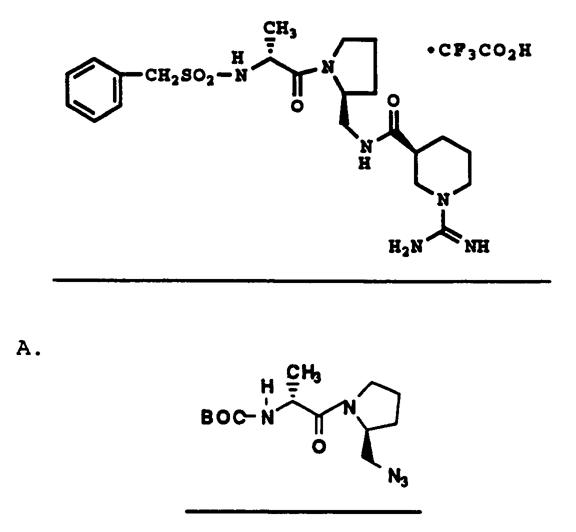

A solution of 1-BOC-2-(methylazido)pyrrolidine (1.36 g, 6.0 mmol) in dichloromethane (5 mL) and trifluoroacetic acid (4 mL) was stirred at room temperature for two hours. The reaction mixture was concentrated under reduced pressure and toluene was added and removed at reduced pressure. This process was repeated two more times. The crude amine, N-BOC-D-alanine (1.14 g, 6 mmol) and 1-hydroxybenzotriazole (1.01 g, 7.5 mmol) were dissolved in DMF (30 mL) and treated with 4-methyl morpholine (1.32 mL, 12 mmol) followed by WSC (1.2 g, 6.0 mmol). The reaction mixture was stirred overnight at room temperature. The mixture was diluted with ethyl acetate (70 mL) and washed with saturated KHSO₄ solution (20 mL), NaHCO₃ solution (25 mL) and 10% lithium chloride solution (2 x 15 mL), dried over magnesium sulfate and concentrated in vacuo to give title compound as an oil (1.74 g, 98%) which was used without purification. 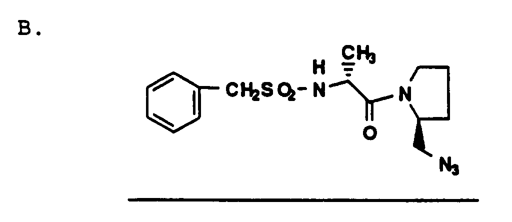
A solution of Part A azide (1.74 g, 5.85 mmol) in dichloromethane (5 mL) and trifluoroacetic acid (4 mL) was stirred at room temperature for two hours. The reaction mixture was concentrated under reduced pressure and toluene was added and removed at reduced pressure. This process was repeated two more times. A solution of the crude des-BOC-amine and α-toluenesulfonyl chloride in dichloromethane (30 mL) was cooled to 0-5°C and triethylamine (4.2 mL, 30 mmol) was added dropwise. The mixture was stirred cold for one hour and then diluted with 1 N HCl solution (50 mL). The dichloromethane layer was separated, dried over magnesium sulfate and concentrated in vacuo. The crude oil was chromatographed on silica gel, eluting with 50% and 75% ethyl acetate in hexanes to give the title benzylsulfonamide as a white foam (1.55g, 75%). 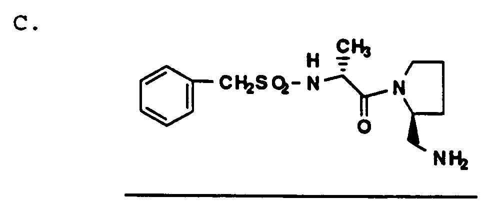
The Part B azide (1.55 g, 4.42 mmol) was dissolved in absolute ethanol (60 mL) and treated with 10% palladium on carbon (310 mg). The reaction flask was equipped with a hydrogen filled balloon via a three-way stopcock. Air inside the flask was evacuated under reduced pressure and replaced with hydrogen from the balloon. This operation was repeated (3x). Hydrogenolysis was continued for twenty hours. The balloon was removed and the catalyst was removed by filtration. The pad was washed with more ethanol. The filtrate was concentrated in vacuo to obtain title amine (1.33 mg, 93%) as a foam which was used without purification. 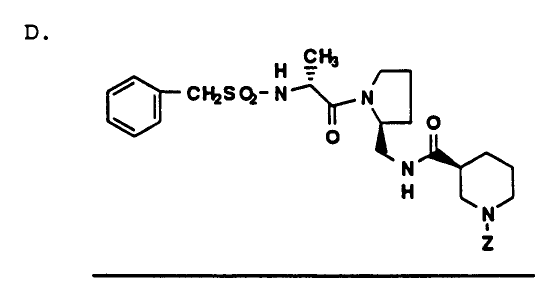
Part C amine (488 mg, 1.5 mmol) and N-carbobenzyloxy-3(S)-piperidine carboxylic acid (395 mg, 1.5 mmol) were dissolved in DMF (15 mL) at RT. HOBT (270mg, 2.0 mmol), 4-methyl morpholine (550 µL, 5 mmol) and WSC (300mg, 1.5 mmol) were added. The reaction was stirred 20 hours at room temperature. The mixture was then diluted with ethyl acetate (50 mL) and washed with KHSO₄ solution (30 mL), saturated NaHCO₃ solution (25 mL) and 10% lithium chloride solution (3 x 15 mL), dried over magnesium sulfate and concentrated in vacuo to give title compound (730 mg, 85 %) as a white foam. 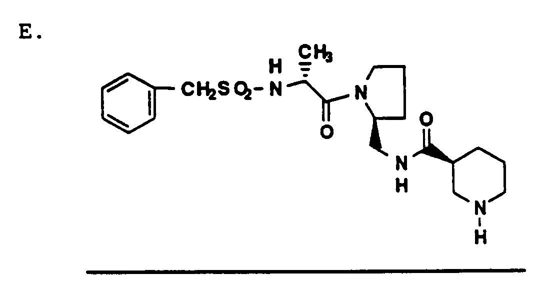
Part D compound (730 mg, 1.28 mmol) was dissolved in ethanol (100 mL) and treated with Pearlman's catalyst (300mg). The reaction flask was connected to a hydrogen filled balloon via a three-way stopcock. Air inside the flask was evacuated under reduced pressure and replaced with hydrogen from the balloon. This operation was repeated (3x). Hydrogenation was continued 18 hours. TLC indicated that a large amount of starting material remained. The catalyst was removed by filtration, the pad was washed with more ethanol and the filtrate was taken to dryness at reduced pressure. The residue was dissolved in ethanol (100 mL), acetyl chloride (1.5 mL) and 10% palladium on carbon (300 mg) were added and the mixture was hydrogenated on the Parr apparatus at 55 psi for twenty hours. The catalyst was removed by filtration. The pad was washed with more ethanol. The filtrate was concentrated in vacuo to obtain title amine which was used without purification. 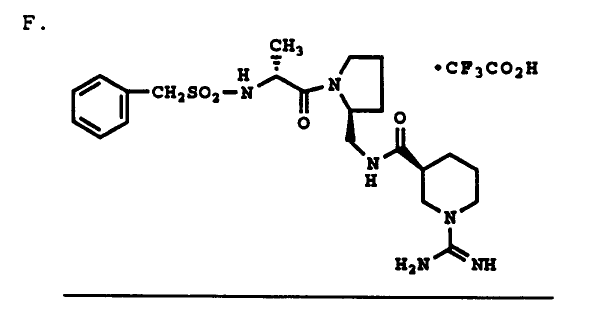
The crude Part E amine was dissolved in dimethylformamide (2.5 mL). H-pyrazole-1-carboxamidine (263 mg, 1.8 mmol) and diisopropylethyl amine (700 µL) were added. The mixture was stirred 24 hours at room temperature. Ether (15 mL) was then added. Gummy material precipitated. The ether was decanted and the precipitate was washed with more ether. The gummy material was dissolved in methanol and taken to dryness in vacuo. The remaining material was purified by preparative HPLC (YMC S-15 ODS 50 x 500 mm column, eluting with 40% methanol in water, containing 0.1% TFA). Fractions containing clean title compound were combined and lyophilized to provide a white solid which was used to obtain NMR spectra, recovered, dissolved in water (30 mL) and relyophilized to give title compound ( 271 mg, 34%).
[α]_{D} = +39.7° (C = 0.6, MeOH)

| Analysis: Calcd for 1.20 TFA + 0.3 H₂O: | | | | | |
|---|---|---|---|---|---|
| | C, 47.20; | H, 5.81; | N, 13.54; | F, 11.02; | S, 5.16. |
| Found: | C, 47.26; | H, 5.86; | N, 13.48; | F, 10.93; | S, 5.24. |

### Example 34

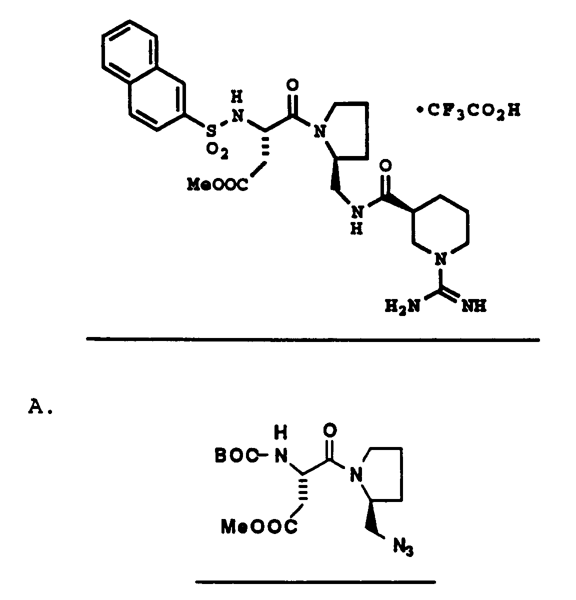

A solution of 1-BOC-2-(methylazido)pyrrolidine (1.36 g, 6.0 mmol) in dichloromethane (5 mL) and trifluoroacetic acid (4 mL) was stirred at room temperature for two hours. The reaction mixture was concentrated under reduced pressure and toluene was added and removed at reduced pressure. This process was repeated two more times. The crude amine, N-BOC-L-aspartic acid, methyl ester (1.48 g, 6 mmol) and 1-hydroxybenzotriazole (1.01g, 7.5 mmol) were dissolved in DMF (30 mL) and treated with 4-methyl morpholine (1.32 mL, 12 mmol) followed by WSC (1.2 g, 6.0 mmol). The reaction mixture was stirred overnight at room temperature. The mixture was diluted with ethyl acetate (70 mL) and washed with satd. KHSO₄ solution (20 mL), NaHCO₃ solution (25 mL) and 10% lithium chloride solution (2 x 15 mL), dried over magnesium sulfate and concentrated in vacuo to give title compound as a foam (1.68 g, 84%) which was used without purification. 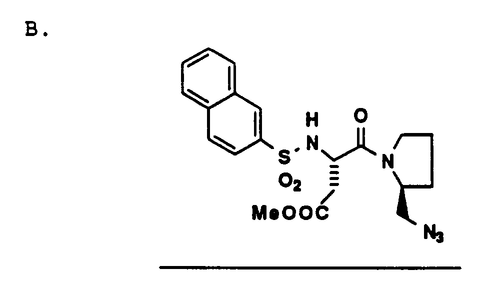
A solution of Part A compound (1.68 g, 5.03 mmol) in dichloromethane (5 mL) and trifluoroacetic acid (4 mL) was stirred at room temperature for two hours. The reaction mixture was concentrated under reduced pressure and toluene was added and removed at reduced pressure. This process was repeated two more times. A solution of the crude des-BOC-amine and napthalee-2-sulfonyl chloride (1.25 g, 5.5 mmol) in dichloromethane (25 mL) was cooled to 0-5°C and triethylamine (2.1 mL, 15 mmol) was added dropwise. The mixture was stirred cold for two hours and then diluted with 1 N HCl solution (30 mL). The dichloromethane layer was separated, dried over magnesium sulfate and concentrated in vacuo. The crude oil was chromatographed on silica gel, eluting with 50% and 75% ethyl acetate in hexanes to give the title sulfonamido adduct as a white foam (1.53g, 68%). 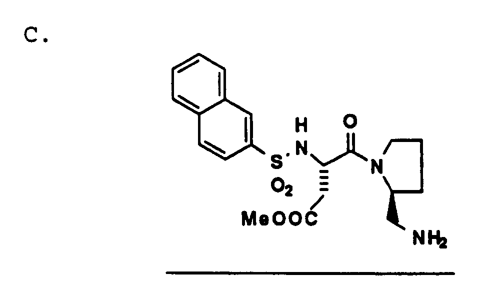
The Part B azide (1.5 g, 3.37 mmol) was dissolved in absolute ethanol (80 mL) and treated with 10% palladium on carbon (300 mg). The reaction flask was equipped with a hydrogen filled balloon via a three-way stopcock. Air inside the flask was evacuated under reduced pressure and replaced with hydrogen from the balloon. This operation was repeated (3x). Hydrogenolysis was continued for twenty hours. The balloon was removed and the catalyst was removed by filtration. The pad was washed with more ethanol. The filtrate was concentrated in vacuo to obtain title amine (Quant.) as a foam which was used without purification. 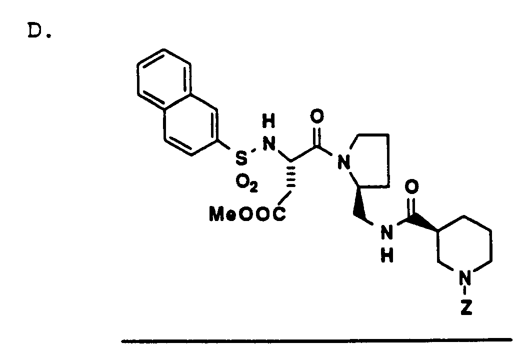
Part C amine (3.37 mmol) and N-carbobenzyloxy-3(S)-piperidine carboxylic acid (790 mg, 3.7 mmol) were dissolved in DMF (20 mL) at RT. HOBT (500mg, 3.7 mmol), 4-methyl morpholine (1.5 mL) and WSC (708mg, 3.7 mmol) were added. The reaction was stirred 20 hours at room temperature. The mixture was then diluted with ethyl acetate (60 mL) and washed with KHSO₄ solution (20 mL), saturated NaHCO₃ solution (15 mL) and 10% lithium chloride solution (2 x 15 mL), dried over magnesium sulfate and concentrated in vacuo. The material obtained was chromatographed on silica gel, eluting with ethyl acetate followed by 5% methanol in ethyl acetate to give title compound (1.56 g, 72%) as a white foam. 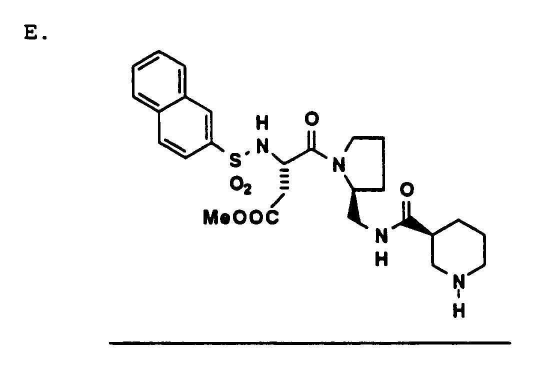
Part D compound (625 mg, 0.97 mmol) was dissolved in methanol (60 mL) and treated with Pearlman's catalyst (200mg). The reaction flask was connected to a hydrogen filled balloon via a three-way stopcock. Air inside the flask was evacuated under reduced pressure and replaced with hydrogen from the balloon. This operation was repeated (3x). Hydrogenation was continued 18 hours. TLC indicated that the reaction was complete. The catalyst was removed by filtration, the pad was washed with more methanol and the filtrate was taken to dryness at reduced pressure to give title amine (470 mg, 96%) which was used without purification. 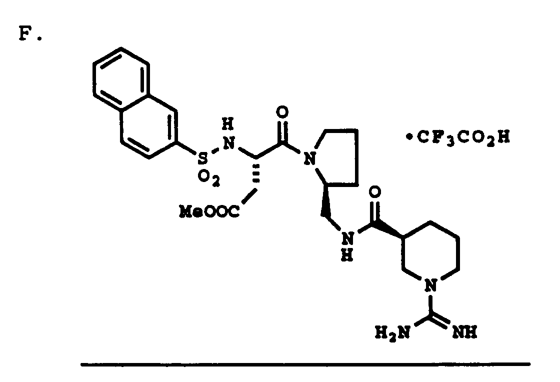
The crude Part E amine (470 mg, 0.928 mmol) was dissolved in dimethylformamide (2.0 mL). H-pyrazole-1-carboxamidine (190 mg, 1.3 mmol) and diisopropylethylamine (360 µL, 2 mmol) were added. The mixture was stirred over a weekend at room temperature. Ether (25 mL) was then added. Gummy material precipitated. The ether was decanted and the precipitate was washed with more ether. The gummy material was dissolved in methanol and taken to dryness in vacuo. The remaining material was purified by preparative HPLC (YMC S-15 ODS 50 x 500 mm column, eluting with 53% methanol in water, containing 0.1% TFA). Fractions containing clean title compound were combined and lyophilized to provide a white solid which was used to obtain NMR spectra, recovered, dissolved in water (30 mL) and relyophilized to give title compound (324 mg, 49%).
[α]_{D}= -20.3° (C = 0.6, MeOH)

| Analysis: Calcd for 1.10 TFA + 0.9 H₂O: | | | | | |
|---|---|---|---|---|---|
| | C, 49.10; | H, 5.49; | N, 11.77; | F, 8.78; | S, 4.49. |
| Found: | C, 49.11; | H, 5.23; | N, 11.58; | F, 6.96; | S, 4.42. |

### Example 35

### [1S(2R*,3R*)]-N-[2-[[[[1-(Aminoiminomethyl)-3-piperidinyl]carbonyl]amino]methyl]-1-pyrrolidinyl]-1-(hydroxymethyl)-2-oxoethyl]-α-toluenesulfonamide

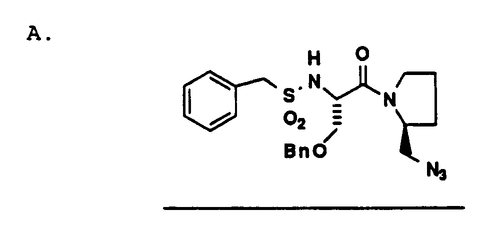

Example 30 Part G(1) BOC-azide ( 3.85 mg, 9.55 mmol) was dissolved in TFA (20 mL) and stirred at RT 1.5 hours. The TFA was removed by distillation under reduced pressure and by coevaporation with toluene. The residue is dissolved in dichloromethane (100 mL), cooled in an ice bath, and triethylamine (4.0 mL, 28.7 mmol) and α-toluene sulfonyl chloride (2.00 g, 10.5 mmol) are added. The cooling bath is removed and the solution is stirred for 2 h. This solution is washed with KHSO₄ solution (25 mL x 2) and NaHCO₃ solution(25 mL x 2), dried over magnesium sulfate and evaporated to provide crude title compound. The crude product is chromatographed on silica gel and eluted with 30% and 50% EtOAc in hexane to provide title sulfonamide (2.597 g, 55%).

### B. [1S(2R*,3R*)]-N-[2-[[[[1-(Aminoiminomethyl)-3-piperidinyl]carbonyl]amino]methyl]-1-pyrrolidinyl]-1-(hydroxymethyl)-2-oxoethyl]-α-toluenesulfonamide

The title compound is prepared from Part A azide following the procedure described in Example 26.

### Example 36

### [1S(2R*,3R*)]-N-[2-[[[[1-(Aminoiminomethyl)-3-piperidinyl]carbonyl]amino]methyl]-1-pyrrolidinyl]-1-(carbomethoxymethyl)-2-oxoethyl]-α-toluenesulfanamide

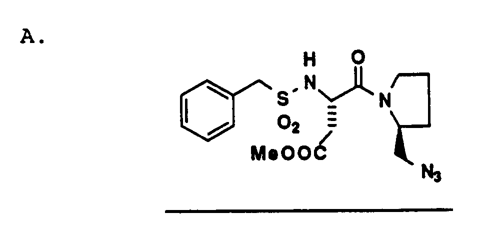

A solution of Example 34 Part A compound (1.68 g, 5.03 mmol) in dichloromethane (5 mL) and trifluoroacetic acid (4 mL) was stirred at room temperature for two hours. The reaction mixture was concentrated under reduced pressure and toluene was added and removed at reduced pressure. This process was repeated two more times. A solution of the crude des-BOC-amine and α-toluenesulfonyl chloride (1.14 g, 6 mmol) in dichloromethane (25 mL) is cooled to 0-5°C and triethylamine (2.1 mL, 15 mmol) is added dropwise. The mixture is stirred cold for two hours and then diluted with 1 N HCl solution (30 mL). The dichloromethane layer is separated, dried over magnesium sulfate and concentrated in vacuo. The crude oil is chromatographed on silica gel, eluting with 50% and 75% ethyl acetate in hexanes to give the title sulfonamido adduct (1.53g, 74%).

### B. [1S(2R*,3R*)]-N-[2-[[[[1-(Aminoiminomethyl)-3-piperidinyl]carbonyl]amino]methyl]-1-pyrrolidinyl]-1-(carbomethoxymethyl)-2-oxoethyl]-α-toluenesulfonamide

The title compound is prepared from Part A compound, following the procedure described in Example 34.

### Example 37

### [1S(2R*,3R*)]-N-[2-[[[[1-(Aminoiminomethyl)-3-piperidinyl]carbonyl]amino]methyl]-1-pyrrolidinyl]-1-(carboxamidomethyl)-2-oxoethyl]-α-toluenesulfonamide

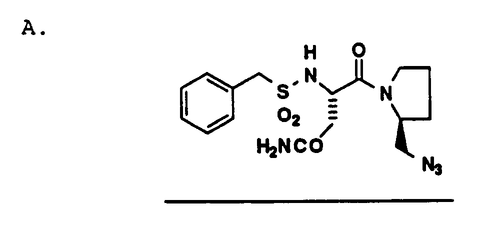

A solution of Example 36 Part A compound (816 mg, 2 mmol) in a saturated methanolic ammonia (5 mL) is stirred at room temperature in a sealed tube for 48 h. The mixture is concentrated under reduced pressure and in vacuo to form the title product.

### B. [1S(2R*,3R*)]-N-[2-[[[[1-(Aminoiminomethyl)-3-piperidinyl]carbonyl]amino]methyl]-1-pyrrolidinyl]-1-(carboxamidomethyl)-2-oxoethyl]-α-toluenesulfonamide

The title compound is prepared from Part A compound, following the procedure described in Example 34.

### Examples 38 to 54

The following compounds were prepared carrying out procedures described in the specification and working examples.
38. [S-(R*,R*)]-3-(Aminomethyl)-N-[[1-[3-hydroxy-2-[(2-napthalenylsulfonyl)amino]-1-oxopropyl]-2-pyrrolidinyl]methyl]benzamide, trifluoroacetate (1:1) salt
[α]_{D} = 39.4^{o} (c 0.50, MeOH)

| Anal. Calc'd for C₂₆H₃₀N₄O₅S · 1.26 TFA · 0.97 H₂0: | | | | | |
|---|---|---|---|---|---|
| | C, 50.99; | H, 4.98; | N, 8.34; | S, 4.77; | F, 10.69 |
| Found: | C, 50.99; | H, 4.83; | N, 8.35; | S, 4.57; | F, 10.72 |

39. [S-(R*,R*)]-4-(Aminomethyl)-N-[[1-[3-hydroxy-2-[(2-napthalenylsulfonyl)amino]-1-oxopropyl]-2-pyrrolidinyl]methyl]benzeneacetamide, trifluoroacetate (1:1) salt
[α]_{D} = 43.0^{o} (c 0.50, MeOH)

| Anal. Calc'd for C₂₇H₃₂N₄O₅S · 1.42 TFA · 0.74 H₂0: | | | | | |
|---|---|---|---|---|---|
| | C, 51.21; | H, 5.03; | N, 8.01; | S, 4.58; | F, 11.56 |
| Found: | C, 51.21; | H, 4.95; | N, 8.08; | S, 4.31; | F, 11.56 |

40. [S-(R*,R*)]-3-(Aminomethyl)-N-[[1-[3-hydroxy-2-[(2-napthalenylsulfonyl)amino]-1-oxopropyl]-2-pyrrolidinyl]methyl]benzeneacetamide, trifluoroacetate (1:1) salt
[α]_{D} = 44.1^{o} (c 1.00, MeOH)

| Anal. Calc'd for C₂₇H₃₃N₄O₅S · 1.35 TFA · 1.48 H₂0: | | | | | |
|---|---|---|---|---|---|
| | C, 50.58; | H, 5.19; | N, 7.94; | S, 4.55; | F, 10.91 |
| Found: | C, 50.58; | H, 4.99; | N, 7.84; | S, 4.47; | F, 11.09 |

41. [3S-[3R*,3(R*,R*)]]-1-(Aminoiminomethyl)-N-[[1-[2-[(2-napthalenylsulfonyl)amino]-1-oxopropyl]-2-pyrrolidinyl]methyl]-3-piperidinecarboxamide, trifluoroacetate (2:3) salt
[α]_{D} = -18.3^{o} (c 0.52, MeOH)

| Anal. Calc'd for C₂₅H₃₄N₆O₄S · 1.67 TFA · 0.23 H₂0: | | | | | |
|---|---|---|---|---|---|
| | C, 48.00; | H, 5.13; | N, 11.85; | S, 4.52; | F, 13.42 |
| Found: | C, 48.00; | H, 5.37; | N, 11.94; | S, 4.45; | F, 13.43 |

42. [3S-[3R*,3(R*,R*)]]-1-(Aminoiminomethyl)-N-[[1-[3-hydroxy-2-[[(4-methylphenyl)sulfonyl]amino]-1-oxopropyl]-2-pyrrolidinyl]methyl]-3-piperidinecarboxamide, trifluoroacetate (1:1) salt
[α]_{D} = +16.0^{o} (c 0.50, MeOH)

| Anal. Calc'd for C₂₂H₃₄N₆O₅S · 1.1 TFA · 1.32 H₂0: | | | | | |
|---|---|---|---|---|---|
| | C, 45.15; | H, 5.91; | N, 13.05; | S, 4.98; | F, 9.74 |
| Found: | C, 45.15; | H, 5.52; | N, 12.88; | S, 4.95; | F, 9.53 |

43. [3S-[3R*,3(R*,R*)]]-1-(Aminoiminomethyl)-N-[[1-[3-hydroxy-2-[[carbobenzyloxy]amino]-1-oxopropyl]-2-pyrrolidinyl]methyl]-3-piperidinecarboxamide, trifluoroacetate (1:1) salt

| Anal. Calc'd for C₂₃H₃₄N₆O₅S· 1.05 TFA · 1.60 H₂0: | | | | |
|---|---|---|---|---|
| | C, 48.38; | H, 6.19; | N, 13.49; | F, 9.60 |
| Found: | C, 48.39; | H, 5.85; | N, 13.16; | F, 9.45 |

44. [1S[2(R*,S*))]-N-[2-[[[[1-(aminoiminomethyl)-4-piperidinyl]carbonyl]amino]methyl]-1-piperidinyl]-1-(hydroxymethyl)-2-oxoethyl]-2-naphthalenesulfonamide, trifluoroacetate (1:1) salt
[α]_{D} = +0.55^{·} (c = 0.5, MeOH),

| Analysis: Calcd for 0.95 TFA + 1.7 H₂O: | | | | | |
|---|---|---|---|---|---|
| | C, 49.02; | H, 5.95; | N, 12.29; | F, 7.92; | S, 4.69. |
| Found: | C, 49.15; | H, 5.61; | N, 11.95; | F, 7.68; | S, 4.89. |

45. [S-(R*,R*)]-3-[(aminoiminomethyl)amino]-N-[[1-[3-hydroxy-2-[[2-naphthalenesulfonyl]-amino]-1-oxopropyl]-2-pyrrolidinyl]methyl]propionamide, trifluoroacetate (1:1) salt
[α]_{D} = -36.8^{·} (c = 0.6, MeOH),

| Analysis: Calcd for 1.20TFA + 0.50 H₂O: | | | | | |
|---|---|---|---|---|---|
| | C, 46.05; | H, 5.10; | N, 13.21; | F, 10.75; | S, 5.04. |
| Found: | C, 46.05; | H, 4.98;, | N, 13.02; | F, 10.82; | S, 4.97. |

46. [1S[2(R*,S*),(3R*)]]-N-[2-[[[[1-(aminoiminomethyl)-3-piperidinyl]carbonyl]amino]methyl]-1-piperidinyl]-1-(hydroxymethyl)-2-oxoethyl]-2-naphthalenesulfonamide, trifluoroacetate (1:1) salt
[α]_{D} = +24.1^{·} (c = 0.9, MeOH),

| Analysis: Calcd for 1.20 TFA + 0.7 H₂O: | | | | | |
|---|---|---|---|---|---|
| | C, 49.14; | H, 5.61; | N, 12.11; | F, 9.85; | S, 4.62. |
| Found: | C, 49.17; | H, 5.36; | N, 12.04; | F, 9.70; | S, 4.70. |

47. [1S[2R*]]-N-[2-[[[[1-(aminoiminomethyl)-3-phenyl]carbonyl]amino]methyl]-1-pyrrolidinyl]-1-(hydroxymethyl)-2-oxoethyl]-2-naphthalenesulfonamide, trifluoroacetate (1:1) salt
[α]_{D} = -41.0° (c = 0.4, MeOH),

| Analysis: Calcd for 1.20 TFA + 1.1 H₂O: | | | | | |
|---|---|---|---|---|---|
| | C, 50.14 | H, 4.80; | N, 10.30; | F, 10.05; | S, 4.71. |
| Found: | C, 50.13; | H, 4.52;, | N, 10.08; | F, 9.78; | S, 4.42. |

48. 1S[2R*]]-N-[2-[[[[1-(aminoiminomethyl)-cis-5-methyl-3-piperidinyl]carbonyl]amino]methyl]-1-pyrrolidinyl]-1-(hydroxymethyl)-2-oxoethyl]-2-naphthalenesulfonamide, trifluoroacetate(1:1) salt
[α]_{D} = -25.5° (c = 0.4, MeOH)

| Analysis: Calcd for 1.25 TFA + 1.2 H₂O: | | | | | |
|---|---|---|---|---|---|
| | C, 48.29; | H, 5.64; | N, 11.86; | F, 10.05; | S, 4.52 |
| Found: | C, 48.34; | H, 5.54; | N, 11.91; | F, 9.99; | S, 4.55. |

49. [1S[2R*]]-N-[2-[[[[1-(aminoiminomethyl)-trans-5-methyl-3-piperidinyl]carbonyl]amino]methyl]-1-pyrrolidinyl]-1-(hydroxymethyl)-2-oxoethyl]-2-naphthalenesulfonamide,trifluoroacetate(1:1) salt
[α]_{D} = -28.8° (c = 0.6, MeOH),

| Analysis: Calcd for 1.10 TFA + 1.0 H₂O: | | | | | |
|---|---|---|---|---|---|
| | C, 49.22; | H, 5.73; | N, 12.21; | F, 9.11; | S, 4.66. |
| Found: | C, 48.23; | H, 5.66; | N, 12.08; | F, 9.12; | S, 4.87. |

50. [1S(2R*,3R*,4R*)]-N-[2-[[[[1-(aminoiminomethyl)-3-piperidinyl]carbonyl]amino]methyl]-1-pyrrolidinyl]-1-(1-hydroxyethyl)-2-oxoethyl]-2-naphthalenesulfonamide, trifluoroacetate (1:1) salt
[α]_{D} = -10.0^{·} (c = 0.7, MeOH),

| Analysis: Calcd for 1.10 TFA + 1.7 H₂O: | | | | | |
|---|---|---|---|---|---|
| | C, 48.34; | H, 5.83; | N, 11.99; | F, 8.95; | S, 4.58. |
| Found: | C, 48.32; | H, 5.57; | N, 11.84; | F, 8.83; | S, 4.71. |

51. [1S(2R*,3S*,4R*)]-N-[2-[[[[1-(aminoiminomethyl)-3-piperidinyl]carbonyl]amino]methyl]-1-pyrrolidinyl]-1-(1-hydroxyethyl)-2-oxoethyl]-2-naphthalenesulfonamide, trifluoroacetate (1:1) salt
[α]_{D} = -19.5^{·} (c = 0.6, MeOH),

| Analysis: Calcd for 1.10 TFA + 1.0 H₂O: | | | | | |
|---|---|---|---|---|---|
| | C, 49.22; | H, 5.73; | N, 12.21; | F, 9.11; | S, 4.66. |
| Found: | C, 49.12; | H, 5.71; | N, 12.16; | F, 9.38; | S, 4.99. |

52. [R-(S*,R*)]-N-[2-[2[[[[1-(aminoiminomethyl)-4-piperidinyl]carbonyl]amino]methyl]-1-pyrrolidinyl]-1-(phenylmethyl)-2-oxoethyl]methanesulfonamide, trifluoroacetate (1:1) salt
[α]_{D} = -34.7° (c = 0.6, MeOH),

| Analysis: Calcd for 1.25 TFA + 0.2 H₂O: | | | | | |
|---|---|---|---|---|---|
| | C, 47.10; | H, 5.75; | N, 13.45; | F, 11.40; | S, 5.13. |
| Found: | C, 47.09; | H, 5.84;, | N, 13.15; | F, 11.44; | S, 5.32. |

53. [S(R*,R*)]-N-[2-[[[[1-(aminoiminomethyl)-3-piperidinyl]carbonyl]amino]methyl]-1-pyrrolidinyl]-2-oxoethyl]-2-naphthalenesulfonamide, trifluoroacetate (1:1) salt
[α]_{D} = +9.1^{·} (c = 0.3, MeOH),

| Analysis: Calcd for 1.20 TFA + 0.8 H₂O: | | | | | |
|---|---|---|---|---|---|
| | C, 48.64; | H, 5.38; | N, 12.89; | F, 10.49; | S, 4.92. |
| Found: | C, 48.95; | H, 5.23; | N, 12.39; | F, 10.32; | S, 4.93. |

54. [R(S*,R*)]N-[2-[2-[[[[1-(aminoiminomethyl)-4-piperidinyl]carbonyl]amino]methyl]-1-pyrrolidinyl]-1-(methyl)-2-oxoethyl]benzylsulfonamide, trifluoroacetate (1:1) salt
[α]_{D} = +13.5^{·} (C = 0.7 , MeOH),

| Analysis: Calcd for 1.30 TFA + 0.2 H₂O: | | | | | |
|---|---|---|---|---|---|
| | C, 46.87; | H, 5.71; | N, 13.33; | F, 11.75; | S, 5.09. |
| Found: | C, 47.23; | H, 5.78; | N, 12.86; | F, 11.36; | S, 5.29. |

## Claims

1. A compound having the structure 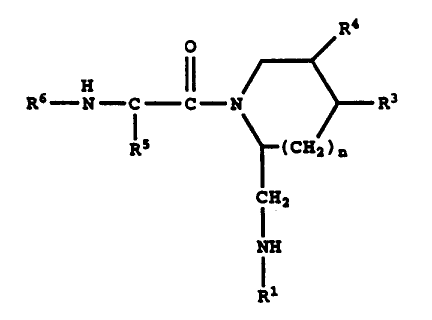 including all stereoisomers, wherein n is 0, 1 or 2;
R¹ is -A-R², -CO-A-R² or -SO₂-A-R^{2;}
wherein R² is guanidine, amidine or amino, and A is an alkyl, alkenyl or alkynyl chain of 2 to 6 carbons; or
R¹ is -(CH₂)ₚ-A'-R^{2'} or -(CH₂)ₚ-CO-A'R^{2'} where p is 0, 1 or 2, R^{2'} is amidine and A' is an azacycloalkyl, azaheteroalkyl or azaheteroalkenyl ring of 4 to 8 atoms, optionally substituted by alkyl, CO or halo as given by the strucutre: 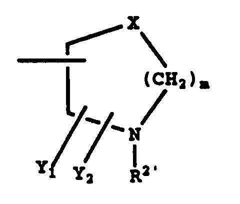 where X is CH₂, O, S, NH or CH=CH; p is 0, 1 or 2;
m = 0, 1, 2, 3 or 4 if X = CH₂ or CH=CH;
m = 2,3 or 4 if X = O, S, NH; and
Y₁, Y₂ are independently H, alkyl, halo or keto; or
R¹ is -(CH₂)ₚ-A''-R^{2''}, -(CH₂)ₚ-CO-A''-R^{2''}, or -(CH₂)ₚ-SO₂-A''-R^{2''},
wherein R^{2''}is guanidine, amidine or aminomethyl, and A'' is aryl or cycloalkyl;
R³ and R⁴ are independently hydrogen, lower alkyl, cycloalkyl, aryl, hydroxy, alkoxy, keto, thioketal, thioalkyl, thioaryl, amino or alkylamino; or R³ and R⁴ together with the carbons to which they are attached form a cycloalkyl, aryl or heteroaryl ring;
R⁵ is hydrogen, hydroxyalkyl, aminoalkyl, alkyl, cycloalkyl, aryl, arylalkyl, alkenyl, alkynyl, amidoalkyl, arylalkoxyalkyl or an amino acid side chain, either protected or unprotected; and
R⁶ is hydrogen, 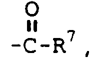 -SO₂R⁷ or -CO₂R⁷ (wherein R⁷ is lower alkyl, aryl or cycloheteroalkyl);
including pharmaceutically acceptable salts thereof.

2. The compound as defined in Claim 1 wherein R¹ is -A-R₂.

3. The compound as defined in Claim 1 wherein R¹ is

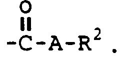

4. The compound as defined in Claim 1 wherein R¹ is -SO₂-A-R².

5. The compound as defined in Claim 1 wherein R¹ is -(CH₂)ₚ-A'-R^{2'}.

6. The compound as defined in Claim 1 wherein R¹ is

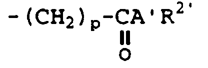

7. The compound as defined in Claim 1 wherein R¹ is -(CH₂)ₚ-SO₂A'R².

8. The compound as defined in Claim 1 wherein R¹ is -(CH₂)ₚ-A''-R^{2''}.

9. The compound as defined in Claim 1 wherein R¹ is -(CH₂)ₚ-CO-A''-R^{2''}.

10. The compound as defined in Claim 1 wherein R¹ is -(CH₂)ₚ-SO₂-A''-R^{2''}.

11. The compound as defined in Claim 1 wherein n is O.

12. The compound as defined in Claim 1 wherein R⁶ is 2-naphthylsulfonyl, H, benzyloxycarbonyl, t-butoxycarbonyl or methylsulfonyl.

13. The compound as defined in Claim 1 wherein R² is guanidine or amino.

14. The compound as defined in Claim 1 wherein n is O, R⁶ is 2-naphthylsulfonyl, H, methylsulfonyl, benzyloxycarbonyl or t-butoxycarbonyl, R⁵ is arylalkyl, hydroxyalkyl or benzyloxyalkyl, R³ is H, R⁴ is H and R¹ is

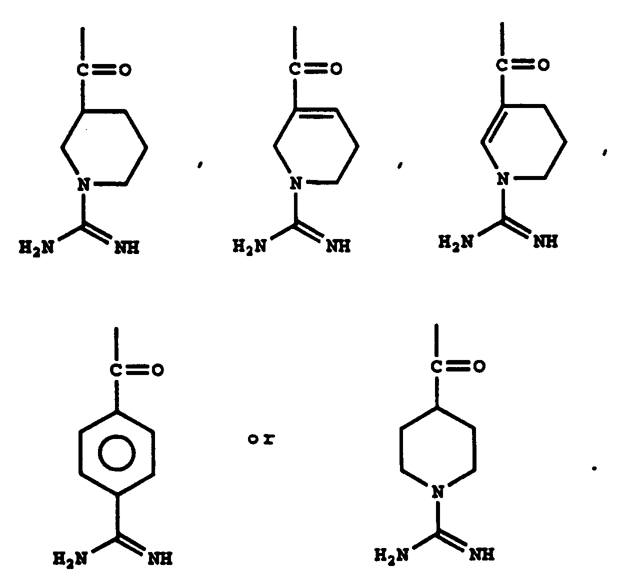

15. The compound as defined in Claim 1 having the structure

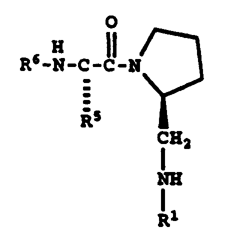

16. The compound as defined in Claim 1 which is [S-(R*,R*)]-4-[(aminoiminomethyl)amino]-N-[[1-[3-hydroxy-2-[(2-naphthalenylsulfonyl)amino]-1-oxopropyl]-2-pyrrolidinyl]methyl]butanamide, or pharmaceutically acceptable salts thereof including its trifluoroacetate (1:1) salt;
[R-(R*,S*)]-[2-[2-[[[4-[(aminoiminomethyl)amino]butyl]amino]methyl]-1-pyrrolidinyl]-2-oxo-1-(phenylmethyl)ethyl]carbamic acid, phenylmethyl ester, or pharmaceutically acceptable salts thereof including its trifluoroacetate (1:2) salt;
(2S)-N-[4-[[[1-(D-phenylalanyl)-2-pyrrolidinyl]methyl]amino]butyl]guanidine, or pharmaceutically acceptable salts thereof including its trifluoroacetate (1:2) salt;
[R-(R*,S*)]-4-[(aminoiminomethyl)amino]-N-[[1-(2-amino-1-oxo-3-phenylpropyl)-2-pyrrolidinyl]methyl]butanesulfonamide, or pharmaceutically acceptable salts thereof including its trifluoroacetate (1:2) salt;
[R-(R*,S*)]-[2-[2-[[[[3-[(aminoiminomethyl)amino]propyl]sulfonyl]amino]methyl]-1-pyrrolidinyl-2-oxo-1-(phenylmethyl)ethyl]carbamic acid, 1,1-dimethylethyl ester or pharmaceutically acceptable salts thereof including its trifluoro-acetate (1:1) salt;
[R-(R*,S*)]-3-[(aminoiminomethyl)amino]-N-[[1-(2-amino-1-oxo-3-phenylpropyl)-2-pyrrolidinyl]methyl]propanesulfonamide, or pharmaceutically acceptable salts thereof including its trifluoroacetate (1:2) salt;
[R-(R*,S*)]-2-amino-1-[2-[[[4-(aminomethyl)phenyl]amino]methyl]-1-pyrrolidinyl]-3-phenyl-1-propanone, or pharmaceutically acceptable salts thereof including its trifluoroacetate (1:3) salt;
[R-(R*,S*)]-N-[2-[2-[[[4-(aminomethyl)phenyl]amino]methyl]-1-pyrrolidinyl]-2-oxo-1-(phenylmethyl)ethyl]-2-naphthalenesulfonamide, or pharmaceutically acceptable salts thereof including its trifluoroacetate (2:3) salt;
[R-(R*,S)]-2-amino-1-[2-[3-[(aminoiminomethyl)amino]propyl]-1-pyrrolidinyl]-3-phenyl-1-propanone, or pharmaceutically acceptable salts thereof including its trifluoroacetate (1:3) salt;
[S-(R*,S*)]-4-[(aminoiminomethyl)amino]-N-[[1-[2-[(methylsulfonyl)amino]-1-oxo-3-phenylpropyl]-2-pyrrolidinyl]methyl]butanamide, or pharmaceutically acceptable salts thereof including its trifluoroacetate (1:1) salt;
[R-(R*,S*)]-2-amino-1-[2-[[[4-(aminomethyl)phenyl]amino]methyl]-1-pyrrolidinyl]-3-phenyl-1-propanone, or pharmaceutically acceptable salts thereof including its trifluoroacetate (1:3) salt;
[R-(R*,S*)]-N-[2-[2-[[[4-(aminomethyl)phenyl]amino]methyl]-1-pyrrolidinyl]-2-oxo-1-(phenylmethyl)ethyl]-2-naphthalenesulfonamide, or pharmaceutically acceptable salts thereof including its trifluoroacetate (2:3) salt;
[S-(R*,R*)]-4-[(aminoiminomethyl)amino]-N-[[1-[3-hydroxy-2-[[(7-methoxy-2-naphthalenyl)sulfonyl]amino]-1-oxopropyl]-2-pyrrolidinyl]methyl]butanamide, or pharmaceutically acceptable salts thereof including its trifluoroacetamide (1:1) salt;
[R-(R*,S*)]-4-[(aminoiminomethyl)amino]-N-[[(1-(2-amino-1-oxo-3-phenylpropyl)-2-pyrrolidinyl]methyl]butanamide, or pharmaceutically acceptable salts thereof including its trifluoroacetate (1:2) salt;
[R-(R*,S*)]-N-[2-[2-[[[4-(aminoiminomethyl)phenyl]amino]methyl]pyrrolidinyl]-2-oxo-1-(phenylmethyl)ethyl]methanesulfonamide, or pharmaceutically acceptable salts thereof including its trifluoroacetate (1:1) salt;
[S-(R*,S*)]-4-[[[1-(2-amino-1-oxo-3-phenylpropyl)-2-pyrrolidinyl]methyl]amino]benzenecarboximidamide, or pharmaceutically acceptable salts thereof including its trifluoroacetate (1:2) salt;
[S-(R*,R*)]-4-[(aminoiminomethyl)amino]-N-[[1-[3-hydroxy-2-[[(7-methoxy-2-naphthalenyl)sulfonyl]amino]-1-oxopropyl]-2-pyrrolidinyl]methyl]butanamide, or pharmaceutically acceptable salts thereof including its trifluoroacetamide (1:1) salt
[R-(R*,S*)]-N-[2-[2-[[[4-(aminoiminomethyl)phenyl]amino]methyl]pyrrolidinyl]-2-oxo-1-(phenylmethyl)ethyl]methanesulfonamide, or pharmaceutically acceptable salts thereof including its trifluoroacetate (1:1) salt;
[1(S),2S]-1-(aminoiminomethyl)-N-[[1-[3-hydroxy-2-[(2-naphthalenylsulfonyl)amino]-1-oxopropyl]-2-pyrrolidinyl]methyl]-3-piperidinecarboxamide, or pharmaceutically acceptable salts thereof including its trifluoroacetate (1:1) salt;
[S-(R*,R*)]-1-(aminoiminomethyl)-N-[[1-[3-hydroxy-2-[(2-naphthalenylsulfonyl)amino]-1-oxopropyl]-2-pyrrolidinyl]methyl]-4-piperidinecarboxamide, or pharmaceutically acceptable salts thereof including its trifluoroacetate (1:1) salt;
N-[(S)-2-[(S)-2-[[[[1-(aminoiminomethyl)-3-piperidinyl]acetyl]amino]methyl]-1-pyrrolidinyl]-1-(hydroxymethyl)-2-oxoethyl]-2-naphthalenesulfonamide, or pharmaceutically acceptable salts thereof including its trifluoroacetate (1:1) salt;
[S-(R*,R*)]-N-[2-[2-[[[[1-(aminoiminomethyl)-4-piperidinyl]acetyl]amino]methyl]-1-pyrrolidinyl]-1-(hydroxymethyl)-2-oxoethyl]-2-naphthalenesulfonamide, or pharmaceutically acceptable salts thereof including its trifluoroacetate (1:1) salt;
[S-(R*,R*)]-N-[2-[2-[[[5-[(aminoiminomethyl)amino]-1-oxopentyl]amino]methyl]-1-pyrrolidinyl]-1-(hydroxymethyl)-2-oxoethyl]-2-naphthalenesulfonamide, or pharmaceutically acceptable salts thereof including its trifluoroacetate (1:1) salt;
[S-(R*,R*)]-N-[2-[2-[[[6-[(aminoiminomethyl)amino]-1-oxohexyl]amino]methyl]-1-pyrrolidinyl]-1-(hydroxymethyl)-2-oxoethyl]-2-naphthalenesulfonamide, or pharmaceutically acceptable salts thereof including its trifluoroacetate (1:1) salt;
[1S[2R*(3S*)]]-N-[2-[2-[[[[1-(aminoiminomethyl)-3-piperidinyl]carbonyl]amino]methyl]-1-pyrrolidinyl]-1-(hydroxymethyl)-2-oxoethyl]-2-naphthalenesulfonamide, or pharmaceutically acceptable salts thereof including its trifluoroacetate (1:1) salt;
[S-(R*,R*)]-N-[2-[2-[[[7-[(aminoiminomethyl)amino]-1-oxoheptyl]amino]methyl]-1-pyrrolidinyl]-1-(hydroxymethyl)-2-oxoethyl]-2-naphthalenesulfonamide, or pharmaceutically acceptable salts thereof including its trifluoroacetate (1:1) salt;
[1S[2R*(3R*)]]-N-[2-[2-[[[[1-aminoiminomethyl)-3-piperidinyl]carbonyl]amino]methyl]-1-pyrrolidinyl]-1-(hydroxymethyl)-2-oxoethyl]-2-naphthalenesulfonamide, or pharmaceutically acceptable salts thereof including its trifluoroacetate (1:1) salt.

17. The compound as defined in Claim 1 which is
[S-(R*,R*)]-4-(aminomethyl)-N-[[1-[3-hydroxy-2-[(2-naphthalenylsulfonyl)amino]-1-oxopropyl]-2-pyrrolidinyl]methyl]benzamide, or salts thereof including the trifluoroacetate (1:1) salt;
[1S(2R*,3R*)]-N-[2-[[[[1-(aminoiminomethyl)-3-piperidinyl]carbonyl]amino]methyl]-1-pyrrolidinyl]-1-(hydroxymethyl)-2-oxoethyl]-α-toluenesulfonamide or salts thereof;
[1S(2R*,3R*)]-N-[2-[[[[1-(aminoiminomethyl)-3-piperidinyl]carbonyl]amino]methyl]-1-pyrrolidinyl]-1-(carbomethoxymethyl)-2-oxoethyl-α-toluenesulfonamide or salts thereof;
[1S(2R*,3R*)]-N-[2-[[[[1-(aminoiminomethyl)-3-piperidinyl]carbonyl]amino]methyl]-1-pyrrolidinyl]-1-(carboxamidomethyl)-2-oxoethyl]-α-toluenesulfonamide or salts thereof;
S-(R*,R*)]-3-(aminomethyl)-N-[[1-[3-hydroxy-2-[(2-napthalenylsulfonyl)amino]-1-oxopropyl]-2-pyrrolidinyl]methyl]benzamide, or salts thereof including its trifluoroacetate (1:1) salt;
S-(R*,R*)]-4-(aminomethyl)-N-[[1-[3-hydroxy-2-[(2-napthalenylsulfonyl)amino]-1-oxopropyl]-2-pyrrolidinyl]methyl]benzeneacetamide, or salts thereof including its trifluoroacetate (1:1) salt;
[S-(R*,R*)]-3-(aminomethyl)-N-[[1-[3-hydroxy-2-[(2-napthalenylsulfonyl)amino]-1-oxopropyl]-2-pyrrolidinyl]methyl]benzeneacetamide, or salts thereof including its trifluoroacetate (1:1) salt
[3S-[3R*,3(R*,R*)]]-1-(minoiminomethyl)-N-[[1-[2-[(2-napthalenylsulfonyl)amino]-1-oxopropyl]-2-pyrrolidinyl]methyl]-3-piperidinecarboxamide, or salts thereof including its trifluoroacetate (2:3) salt;
[3S-[3R*,3(R*,R*)]]-1-(aminoiminomethyl)-N-[[1-[3-hydroxy-2-[[(4-methylphenyl)sulfonyl]amino]-1-oxopropyl]-2-pyrrolidinyl]methyl]-3-piperidine-carboxamide, or salts thereof including its trifluoroacetate (1:1) salt;
[3S-[3R*,3(R*,R*)]}-1-(aminoiminomethyl)-N-[[1-[3-hydroxy-2-[[carbobenzyloxy]amino]-1-oxopropyl]-2-pyrrolidinyl]methyl]-3-piperidine-carboxamide,or salts thereof including its trifluoroacetate (1:1) salt;
[1S[2(R*,S*)]]-N-[2-[[[[1-(aminoiminomethyl)-4-piperidinyl]carbonyl]amino]methyl]-1-piperidinyl]-1-(hydroxymethyl)-2-oxoethyl]-2-naphthalenesulfonamide, or salts thereof including its trifluoroacetate salt;
[S-(R*,R*)]-3-[(aminoiminomethyl)amino]-N-[[1-[3-hydroxy-2-[[2-naphthalenesulfonyl]amino]-1-oxopropyl]-2-pyrrolidinyl]methyl]propionamide, or salts thereof including its trifluoroacetate salt;
[1S[2(R*,S*),(3R*)]]-N-[2-[[[[1-(aminoiminomethyl)-3-piperidinyl]carbonyl]amino]methyl]-1-piperidinyl]-1-(hydroxymethyl)-2-oxoethyl]-2-naphthalenesulfonamide, or salts thereof including its trifluoroacetate salt;
[1S[2R*]]-N-[2-[[[[1-(aminoiminomethyl)-3-phenyl]carbonyl]amino]methyl]-1-pyrrolidinyl]-1-(hydroxymethyl)-2-oxoethyl]-2-naphthalenesulfonamide, or salts thereof including its trifluoroacete salt;
[1S[2R*]]-N-[2-[[[[1-(aminoiminomethyl)-cis-5-methyl-3-piperidinyl]carbonyl]amino]methyl]-1-pyrrolidinyl]-1-(hydroxymethyl)-2-oxoethyl]-2-naphthalenesulfonamide, or salts thereof including its trifluoroacetate salt;
[1S[2R*]]-N-[2-[[[[1-(aminoiminomethyl)-trans-5-methyl-3-piperidinyl]carbonyl]amino]methyl]-1-pyrrolidinyl]-1-(hydroxymethyl)-2-oxoethyl]-2-naphthalenesulfonamide, or salts thereof including its trifluoroacetate salt;
[1S(2R*,3R*,4R*)]-N-[2-[[[[1-(aminoiminomethyl)-3-piperidinyl]carbonyl]amino]methyl]-1-pyrrolidinyl]-1-(1-hydroxyethyl)-2-oxoethyl]-2-naphthalenesulfonamide, or salts thereof including its trifluoroacetate salt;
[1S(2R*,3S*,4R*)]-N-[2-[[[1-(aminoiminomethyl)-3-piperidinyl]carbonyl]amino]methyl]-1-pyrrolidinyl]-1-(1-hydroxyethyl)-2-oxoethyl]-2-naphthalenesulfonamide, or salts thereof including its trifluoroacetate salt;
[R-(S*,R*)]-N-[2-[2-[[[[1-(aminoiminomethyl)-4-piperidinyl]carbonyl]amino]methyl]-1-pyrrolidinyl]-1-(phenylmethyl)-2-oxoethyl]methanesulfonamide, or salts thereof including its trifluoroacetate salt;
[S-(R*,R*)]-N-[2-[[[[1-(aminoiminomethyl)-3-piperidinyl]carbonyl]amino]methyl-1-pyrrolidinyl]-2-oxoethyl]-2-naphthalenesulfonamide, or salts thereof including its trifluoroacetate salt;
[R-(S*,R*)]-N-[2-[2-[[[[1-(aminoiminomethyl)-4-piperidinyl]carbonyl]amino]methyl]-1-pyrrolidinyl]- 1-(methyl)-2-oxoethyl]benzylsulfonamide, or salts thereof including its trifluoroacetate salt.

18. The compound as defined in Claim 1 having the formula 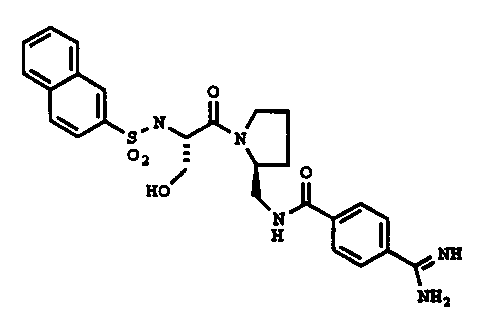 or salts thereof including its trifluoroacetate salt; 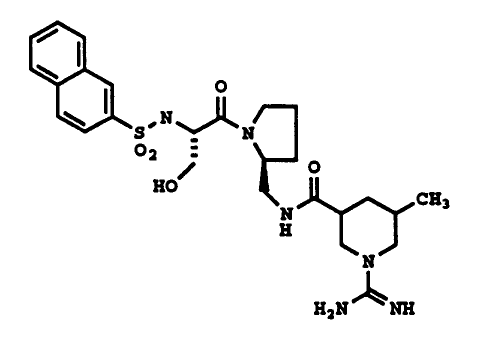 or salts thereof; 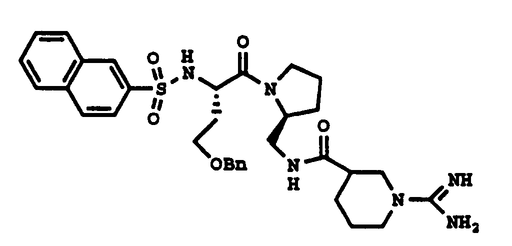 or salts thereof; 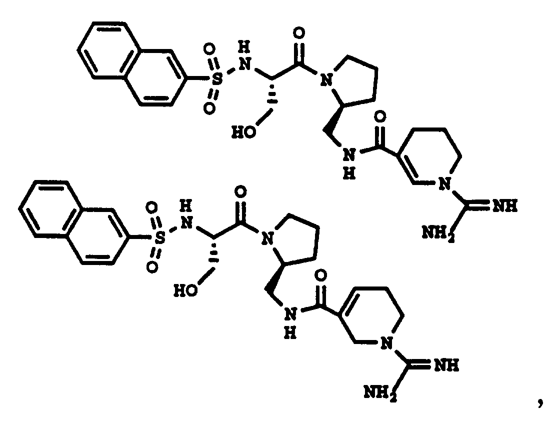 or salts thereof; 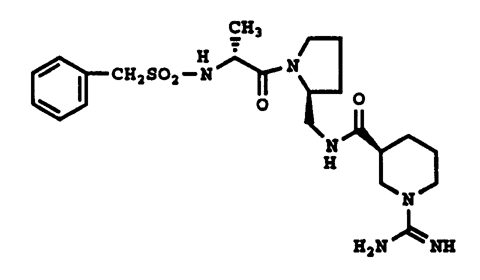 or salts thereof inlcluding its trifluoroacetate salt; 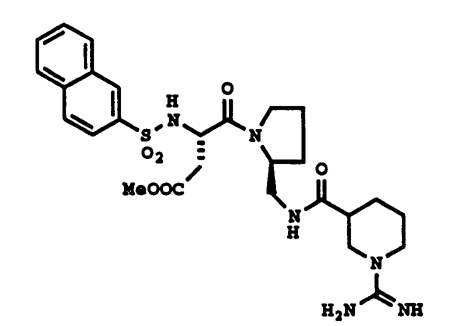 or salts thereof inlcluding its trifluoroacetate salt.

19. A pharmaceutical composition comprising a compound as defined in any of Claims 1 to 18 and a pharmaceutically acceptable carrier therefor.

20. Use of a compound as defined in any of Claims 1 to 18 for the preparation of a pharmaceutical composition for inhibiting or preventing formation of blood clots.
